# EUROPEAN PATENT SPECIFICATION

(11) **EP 0 409 070 B1**
(45) Date of publication and mention of the grant of the patent: **03.03.1999**
(21) Application number: 90113297.7
(22) Date of filing: 12.07.1990
(51) Int. Cl.: C07D 207/44, C08G 73/12, C08L 63/00, C09K 19/38, C07D 405/10, C07D 405/12, C07D 498/04, C08F 22/40

(54) **Mesogenic cyanite functional maleimides and thermosets thereof**
Mesogene Cyanatgruppen enthaltende Maleimide
Maléimides mésogènes à fonction cyanate

(30) Priority: 17.07.1989 US 380936
(43) Date of publication of application: 23.01.1991
(73) Proprietor: THE DOW CHEMICAL COMPANY, Midland, Michigan 48674 (US)
(72) Inventor: Hefner, Robert E., Jr., Lake Jackson, Texas 77566 (US); Earls, Jimmy D., Lake Jackson, Texas 77566 (US)
(74) Representative: Kremer, Robert A.M.

(56) References cited:
- EP-A- 291 552
- US-A- 4 680 378
- US-A- 4 749 760

## Description

The present invention concerns cyanate functional maleimides containing one or more mesogenic or rodlike moieties.

Copolymerization products of compounds containing two or more cyanate groups with compounds containing two or more maleimide groups are known, for example, from U.S. Patent Nos. 4,110,364; 4,287,014; 4,369,304; 4,370,467; 4,371,689; 4,373,086; 4,383,903; 4,393,195; 4,396,745; 4,404,330 and 4,469,859. Representative of said copolymerization products is the bismaleimide-triazine resin prepared by copolymerization of bisphenol A dicyanate and N,N'-(methylene-di-phenylene)bismaleimide. Preparation of said copolymerization products always requires premixing or contacting together two separate components: the polycyanate compound and the polymaleimide compound.

Hefner, Jr. in U.S. Patent Nos. 4,680,378; 4,683,276; 4,731,426 and 4,769,440 provides novel compositions which simultaneously contain both a cyanate group and a maleimide group. Thus, said compositions avoid the premixing or contacting together of separate polycyanate and maleimide components to provide products containing cyanate group and polymaleimide group copolymerization structures.

The novel compositions of the present invention also simultaneously contain both a cyanate group and a maleimide group but additionally contain one or more mesogenic or rodlike moieties. The presence of one or more mesogenic or rodlike moieties serves to improve one or more physical or mechanical properties of the cured compositions relative to the cured compositions prepared using the cyanate functional maleimides of the prior art.

The present invention pertains to cyanate functional maleimide compositions containing one or more mesogenic or rodlike moieties, particularly those represented by the following Formulas I, II, III or IV: 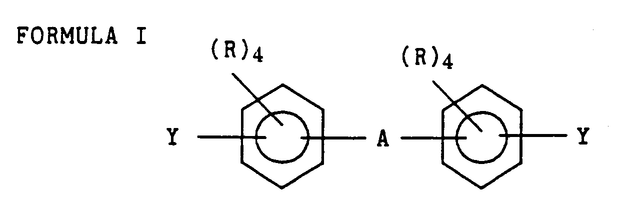 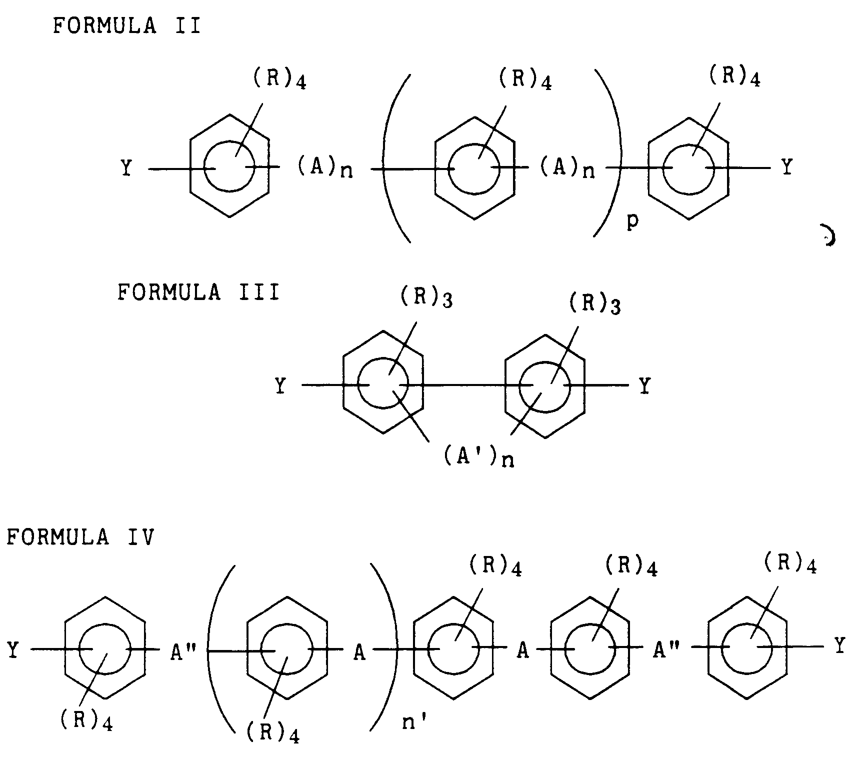 wherein at least about 80 percent of the -A- linkages in Formulas I, II and IV and the direct bond in Formula III and the Y groups are in the para position with respect to each other; one Y group is a cyanate, -O-C≡N, group and the other Y group is a maleimide group represented by the formula 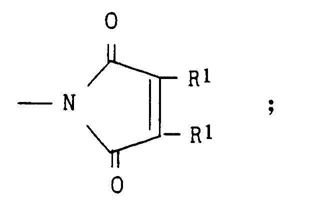 each A is independently -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}--N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 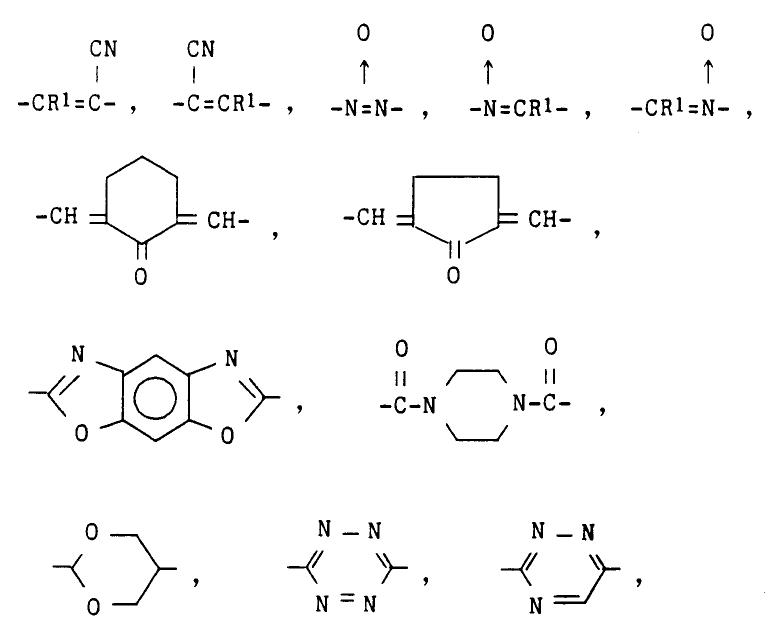 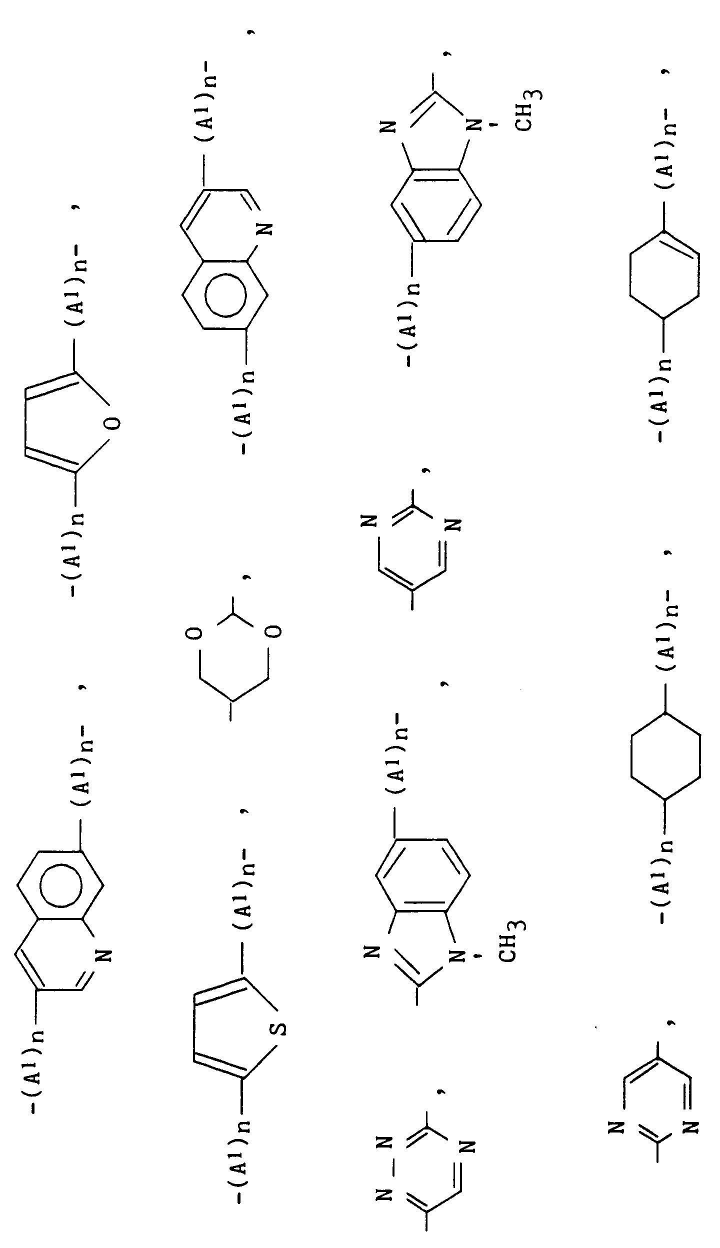 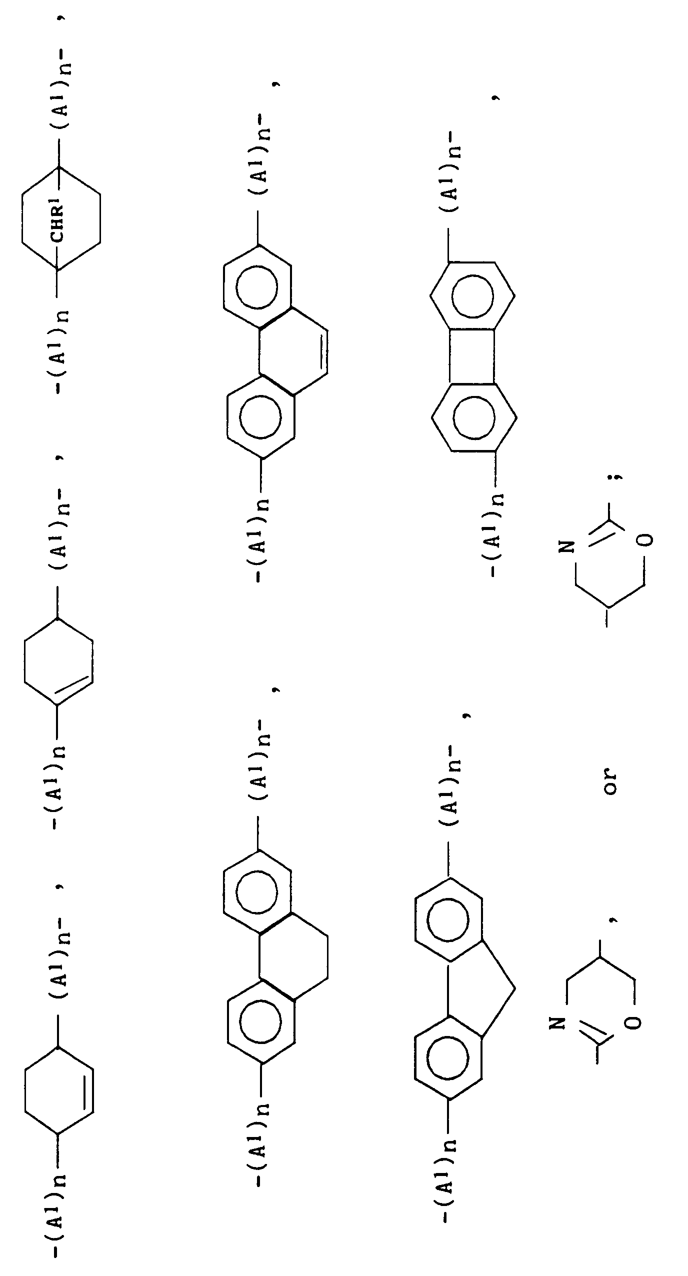 each A' is independently a divalent hydrocarbyl group having from 1 to 10, preferably from 1 to 4 carbon atoms; each A'' is independently an alkylene group having from 1 to 10 carbon atoms, preferably from 1 to 4 carbon atoms, a direct bond, -O-, -CO-, -S-, -S-S-, -SO-, -SO₂- or -O-CO-O-; each A¹ is independently a -CO-, -O-CO-, -CO-O-, -CO-NR¹-, or -NR¹-CO- group; each R is independently hydrogen or a hydrocarbyl or hydrocarbyloxy group having from 1 to 10, preferably from 1 to 4 carbon atoms, a halogen atom, preferably chlorine or bromine, a nitro group, a nitrile group, a phenyl group or a -CO-R¹ group; each R¹ is independently hydrogen or a hydrocarbyl group having 1 to 3 carbon atoms; n has a value of zero or one; n' has an average value from zero to 6, preferably zero to 3; and p has an average value from 1 to 30, preferably from 1 to 3. The aromatic rings can also contain one or more heteroatoms selected from, for example, N, O or S.

Another aspect of the present invention is directed to a process for preparing the aforementioned cyanate functional maleimide compositions containing one or more mesogenic or rodlike moieties.

Another aspect of the present invention pertains to compositions resulting from curing (thermosetting) one or more of the cyanate functional maleimides containing one or more mesogenic or rodlike moieties, optionally in the presence of one or more curing agents or curing catalysts.

Another aspect of the present invention is directed to polymerizable compositions comprising a mixture containing
(A) at least one thermosettable cyanate functional maleimide containing one or more mesogenic or rodlike moieties; and
(B) at least one of
   (1) at least one polycyanate or polycyanamide which does not contain mesogenic or rodlike structures;
   (2) at least one epoxy resin;
   (3) at least one polymaleimide;
   (4) at least one polyamine;
   (5) at least one polyphenol;
   (6) at least one compound containing one or more polymerizable ethylenically unsaturated group(s);
   (7) at least one compound which contains in the same molecule both a cyanate or cyanamide group and a polymerizable ethylenically unsaturated group;
   (8) at least one compound which contains in the same molecule both a 1,2-epoxide group and a polymerizable ethylenically unsaturated group;
   (9) at least one compound which contains in the same molecule both a maleimide group and a cyanate group and does not contain mesogenic or rodlike structures;
   (10) at least one compound which contains one or more mesogenic or rodlike moieties and only one cyanate or cyanamide group per molecule;
   (11) at least one prepolymer of any of the aforesaid components (1) through (10) or any combination of any two or more of said components; or
   (12) a mixture of any two or more of components (1) through (11) in any proportion and any combination.

Another aspect of the present invention is directed to a process for preparing the aforementioned polymerizable compositions.

Another aspect of the present invention pertains to compositions resulting from polymerizing the aforementioned polymerizable compositions.

A further aspect of the present invention pertains to products resulting from orienting any of the aforementioned polymerizable compositions.

The term prepolymers as employed herein means that the compound has been homooligomerized or cooligomerized or interoligomerized or homopolymerized or copolymerized or interpolymerized so as to cause an increase in molecular weight, but not to such an extent that the product has become cured, i.e., insoluble and infusible, but rather, the product is capable of being subsequently cured to an insoluble, infusible state.

### PREPARATION OF THE CYANATE FUNCTIONAL MALEIMIDES CONTAINING ONE OR MORE MESOGENIC OR RODLIKE MOIETIES

The cyanate functional maleimides of the present invention are prepared by reacting one or more aminophenols containing one or more mesogenic or rodlike moieties with a stoichiometric quantity of a maleic anhydride per amine group of said aminophenol in the presence of a suitable solvent and then cyanating the resulting phenolic functional maleimide.

Suitable aminophenols which can be employed herein to prepare the cyanate functional maleimides containing one or more mesogenic or rodlike moieties include, for example, any compound which has an average of one aromatic hydroxyl group and aromatic primary amino group per molecule and include, for example, those represented by the following Formulas V, VI, VII or VIII: 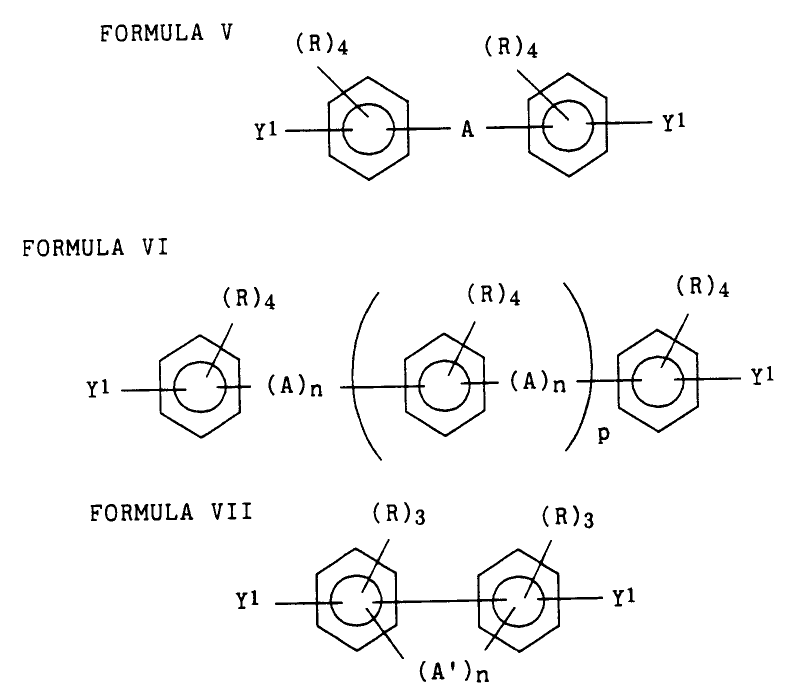 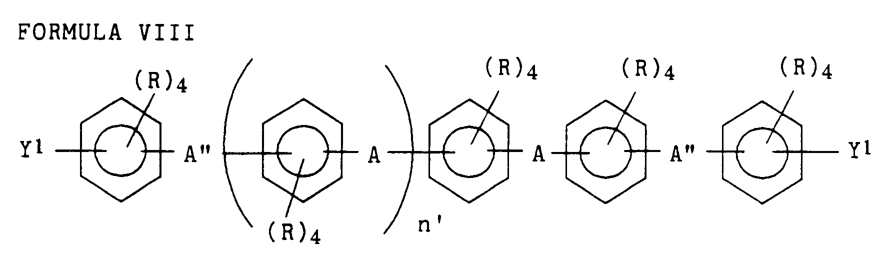 wherein at least about 80 percent of the -A- linkages in Formulas V, VI and VIII and the direct bond between the two aromatic rings in Formula VII and the Y¹ groups are in the para position with respect to each other; one Y¹ group is -OH and the other is -NH₂; each A, A', A'', A¹, R, R¹, n, n' and p are as hereinbefore defined. The aromatic rings can also contain one or more hereoatoms selected from, for example, N, O or S.

The term "hydrocarbyl", as employed herein, means any aliphatic, cycloaliphatic, aromatic, aryl substituted aliphatic or cycloaliphatic, or aliphatic or cycloaliphatic substituted aromatic group. The aliphatic or cycloaliphatic groups can be saturated or unsaturated. When applied to the A' group of Formulas III and VII, the hydrocarbyl group can also contain one or more heteroatoms selected from, for example, N, O or S. Likewise, the term "hydrocarbyloxy" means a hydrocarbyl group having an oxygen linkage between it and the carbon atom to which it is attached.

Particularly suitable aminophenols are, for example, 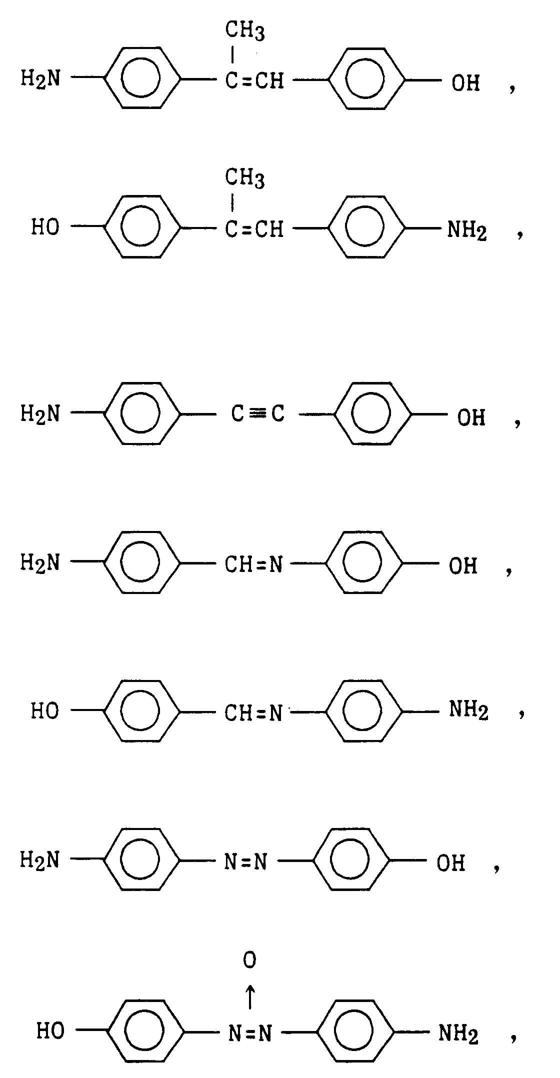 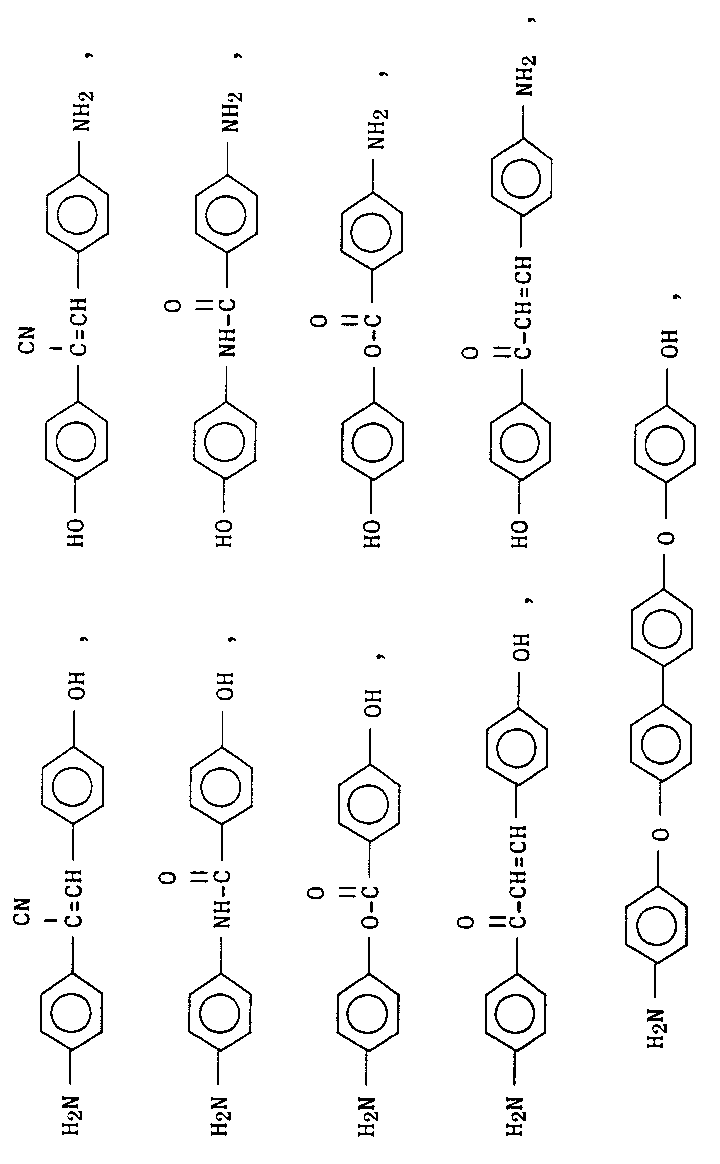 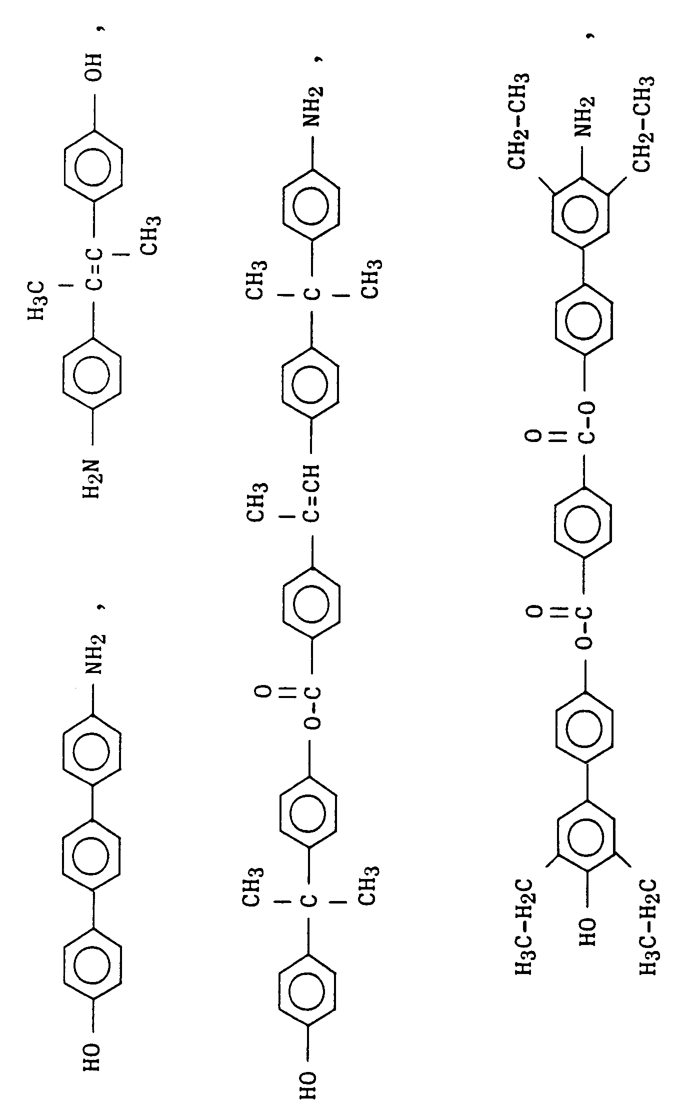 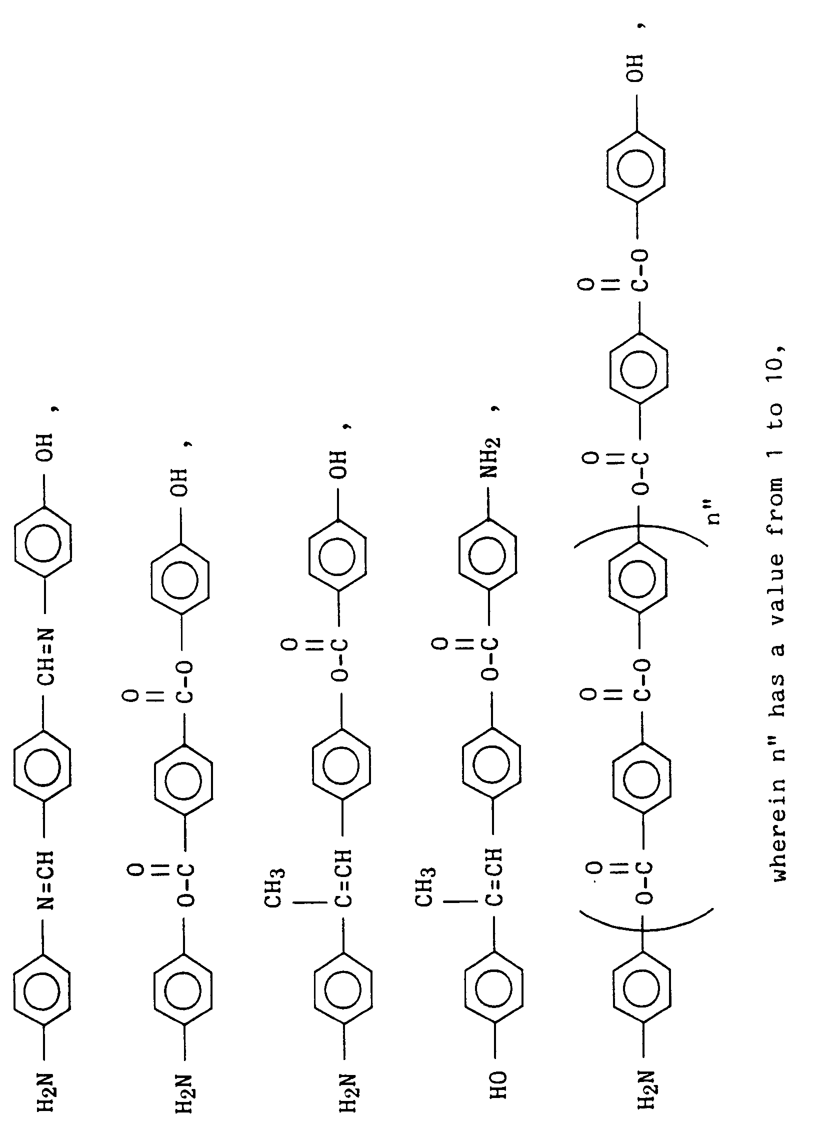 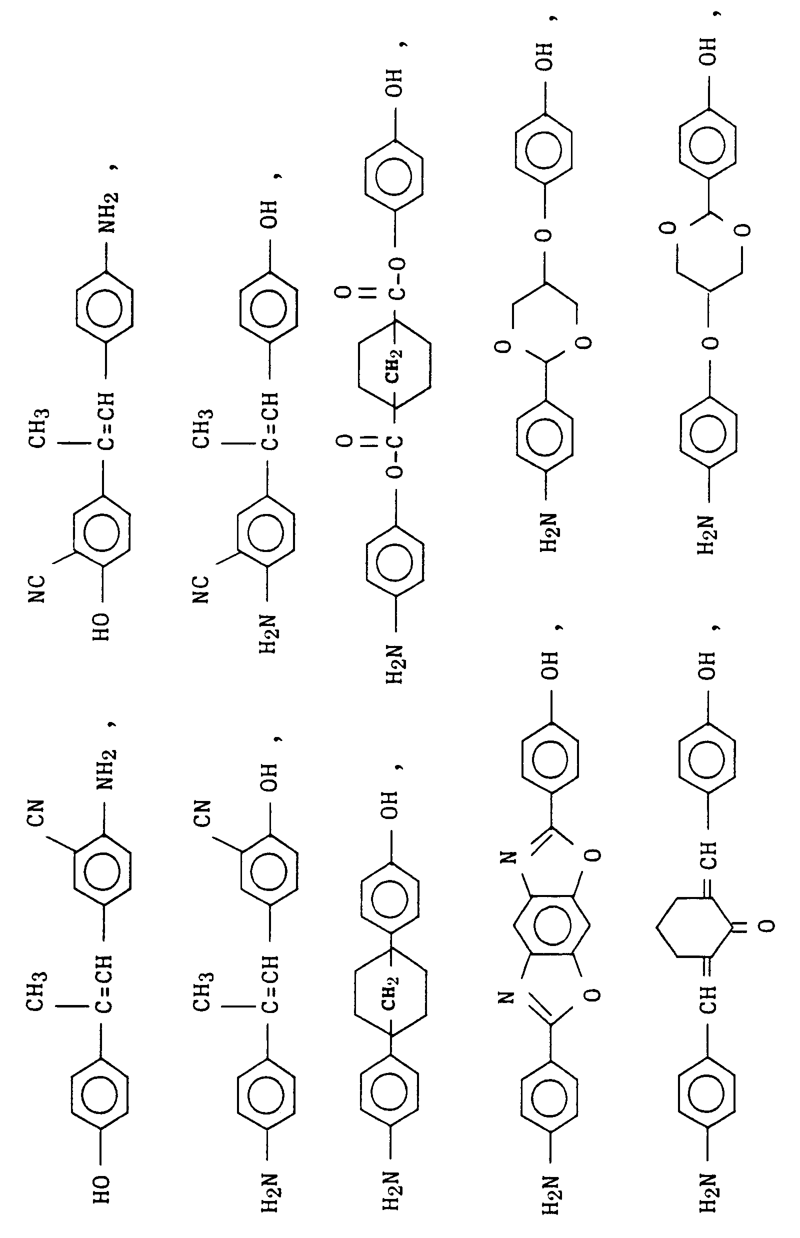 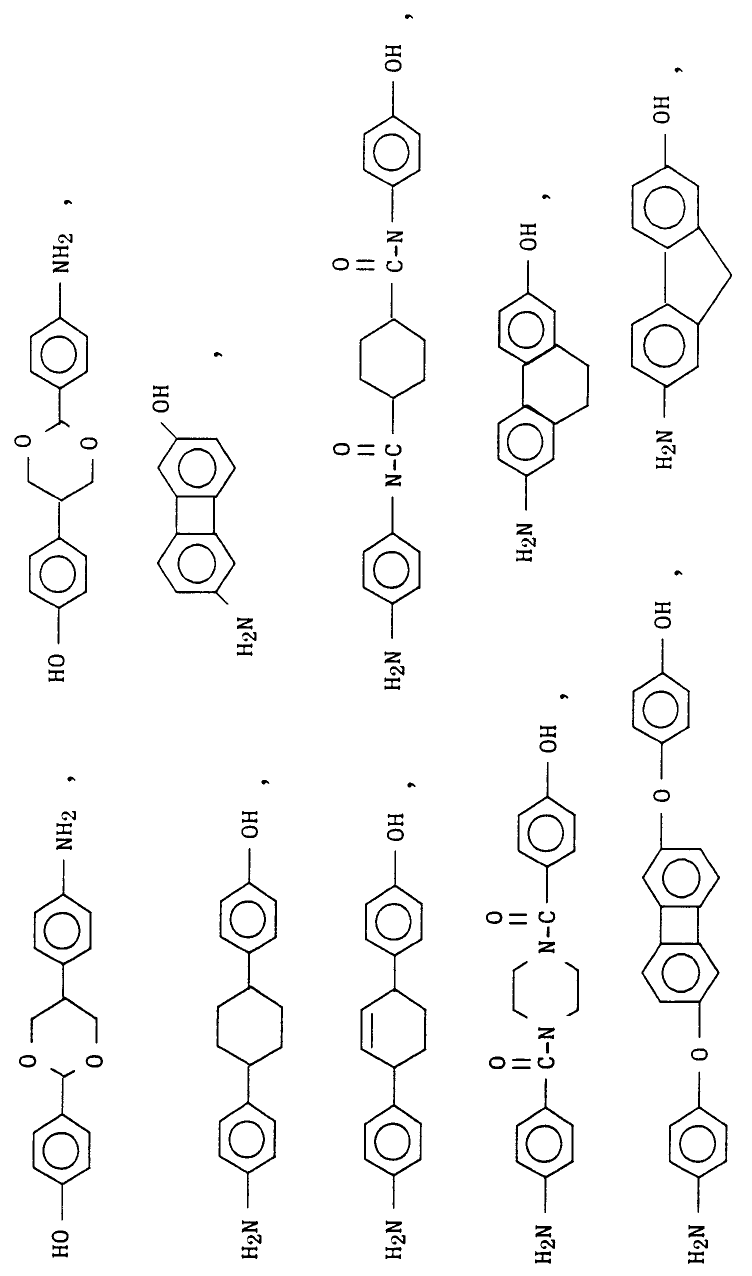 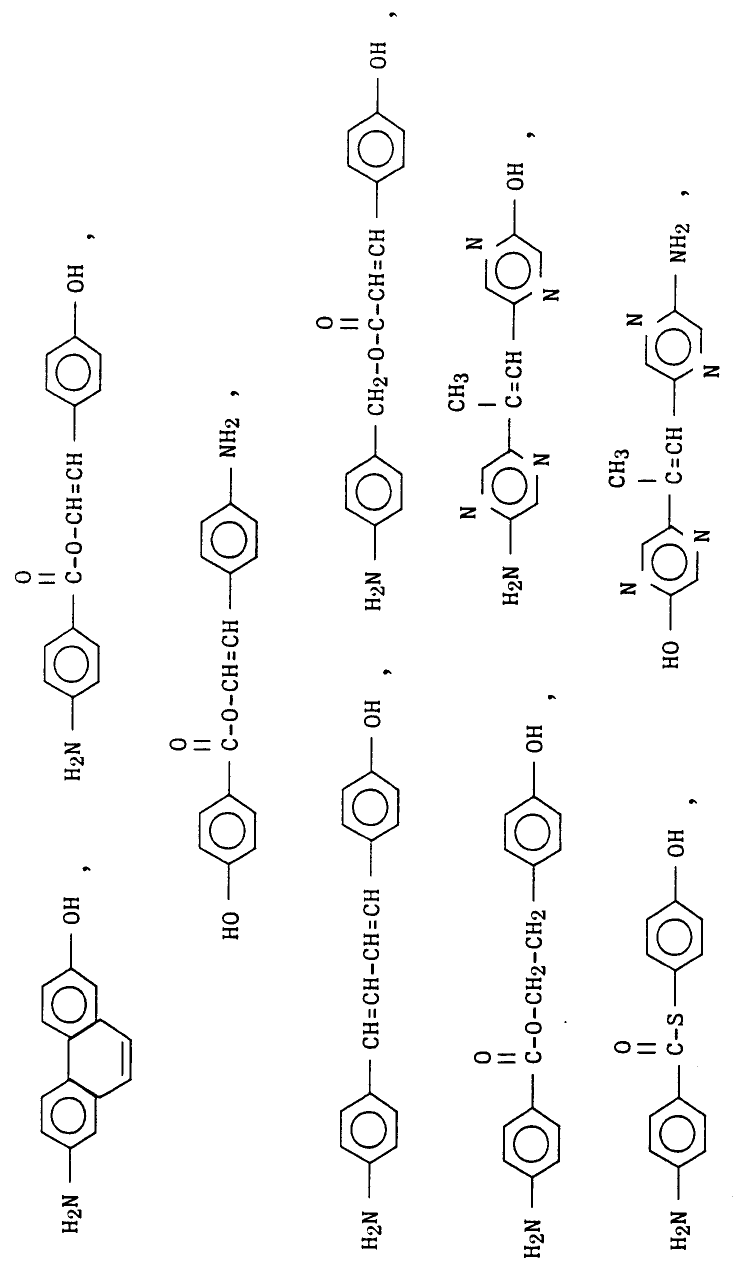 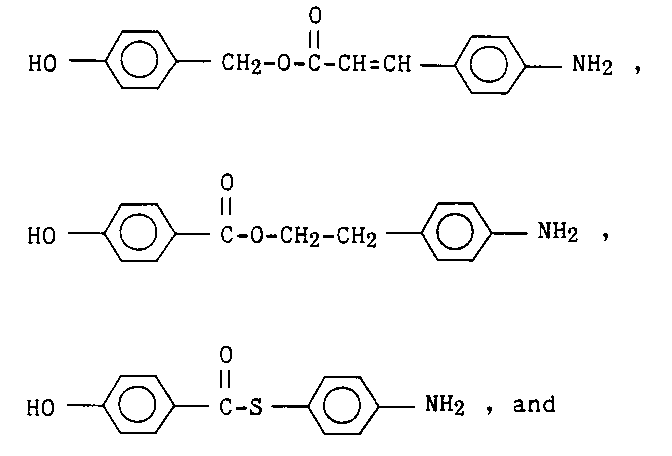 and
mixtures thereof.

Suitable maleic anhydrides include, for example, those represented by the following Formula IX: 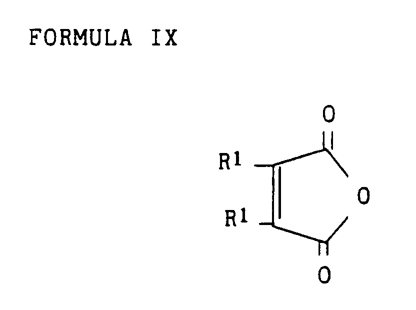 wherein R¹ is as hereinbefore defined. Suitable maleic anhydrides include, for example, maleic anhydride, methyl maleic anhydride and mixtures thereof. Most preferred as the maleic anhydride is maleic anhydride, per se.

Suitable solvents include, for example, aliphatic monocarboxylic acids such as acetic acid, propionic acid and mixtures thereof. Most preferred as the solvent is acetic acid. The maleamic acid resulting from reaction of a maleic anhydride and an aminophenol compound, typically in an inert solvent such as chloroform, toluene or dioxane, may be isolated then dehydrated in an aliphatic monocarboxylic acid to the corresponding phenolic functional maleimide. Alternately, the reaction may be performed in a single continuous step in the aliphatic monocarboxylic acid solvent. The product resulting from this reaction is a phenolic functional maleimide represented by the following Formulas X, XI, XII and XIII: 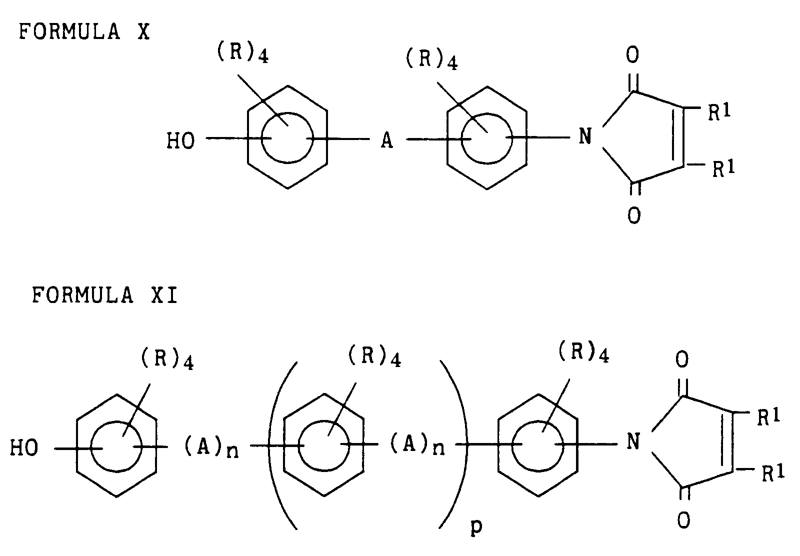 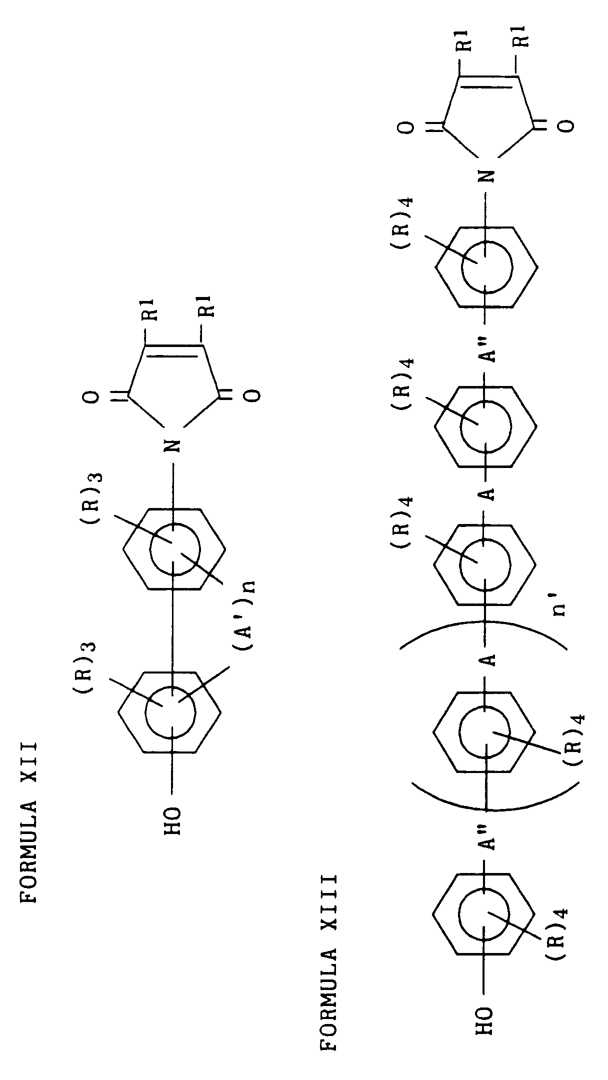 wherein R, R¹, A, A', A'', A¹, n, n' and p are as hereinbefore defined.

Cyanate functional maleimide compositions containing one or more mesogenic or rodlike moieties are conveniently prepared by reacting a stoichiometric quantity up to a slight stoichiometric excess (up to about 20 percent excess) of a cyanogen halide with a phenolic functional maleimide containing one or more mesogenic or rodlike moieties, such as those represented by Formulas X, XI, XII and XIII, in the presence of a stoichiometric quantity of a base material.

Suitable cyanogen halides include cyanogen chloride and cyanogen bromide. Alternately, the method of Martin and Bauer described in Organic Synthesis, volume 61, pages 35-68 (1983) published by John Wiley and Sons, can be used to generate the required cyanogen halide in situ from sodium cyanide and a halogen such as chlorine or bromine.

Suitable base compounds include both inorganic bases and tertiary amines such as sodium hydroxide, potassium hydroxide, trimethylamine, triethylamine and mixtures thereof. Triethylamine is most preferred as the base.

Suitable solvents for the cyanation reaction include, for example, water, aliphatic ketones, chlorinated hydrocarbons, aliphatic and cycloaliphatic ethers and diethers, aromatic hydrocarbons and mixtures thereof. Acetone, methylethylketone, methylene chloride or chloroform are particularly suitable as the solvent. Reaction temperatures of from -40°C to 60°C are operable, with reaction temperatures of -15°C to 10°C being preferred. Reaction times can vary substantially, for example, as a function of the reactants being employed, the reaction temperature, solvent(s) used and the scale of the reaction, but are generally between 15 minutes and 4 hours, with reaction times of 30 minutes to 90 minutes being preferred.

### CURING OF THE CYANATE FUNCTIONAL MALEIMIDES CONTAINING ONE OR MORE MESOGENIC OR RODLIKE MOIETIES

The cyanate functional maleimides containing one or more mesogenic or rodlike structure(s) are cured (thermoset) by heating from 50°C to 400°C, preferably by heating from 100°C to 250°C, optionally in the presence of a suitable catalyst. Suitable catalysts include, for example, acids, bases, salts, free radical forming materials, nitrogen and phosphorus compounds, such as for example, Lewis acids such as AlCl₃, BF₃, FeCl₃, TiCl₄, ZnCl₂, SnCl₄; protonic acids such as HCl, H₃PO₄; aromatic hydroxy compounds such as phenol, p-nitrophenol, pyrocatechol, dihydroxynaphthalene; organic peroxides and hydroperoxides such as t-butylperoxybenzoate, benzoyl peroxide, t-butylhydroperoxide; azo and diazo compounds such as azobisisobutyronitrile; sodium hydroxide, sodium methylate, sodium phenolate, trimethylamine, triethylamine, tributylamine, diazabicyclo-(2.2.2)-octane, quinoline, isoquinoline, tetrahydroisoquinoline, tetraethylammonium chloride, pyridine-N-oxide, tributyl phosphine, zinc octoate, tin octoate, zinc naphthenate, cobalt naphthenate, cobalt octoate and cobalt acetylacetonate. Also suitable as catalysts are the metal chelates such as the chelates of transition metals and bidentate or tridentate ligands, particularly the chelates of iron, cobalt, zinc, copper, manganese, zirconium, titanium, vanadium, aluminum and magnesium. These and other operable catalysts are disclosed in U.S. Patent Nos. 3,694,410 and 4,094,852. Cobalt naphthenate, cobalt octoate and cobalt acetylacetonate are most preferred as the catalysts. The quantity of catalyst used, if any, depends on, for example, the structure of the particular catalyst, the structure of the cyanate functional maleimide being cured, the cure temperature and the cure time. Generally, catalyst concentrations of from 0.001 to 2 percent by weight are preferred.

B-staging or prepolymerization of the compositions of the cyanate functional maleimides of the present invention can be accomplished by using lower temperatures and/or shorter curing times. Curing of the thus formed B-staged (prepolymerized) resin can then be accomplished at a later time or immediately following B-staging (prepolymerization) by increasing the temperature and/or curing time.

The cured (thermoset) products prepared from the cyanate functional maleimides containing mesogenic or rodlike structure(s) possess a complex variety of curing structures including the cyanate group homopolymerization structure 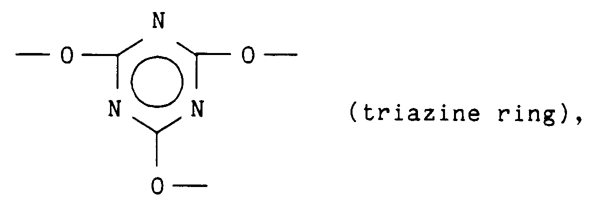 the maleimide group homopolymerization structure 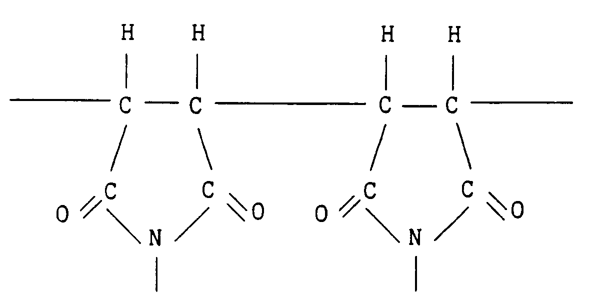 and cyanate group and maleimide group copolymerization structures such as 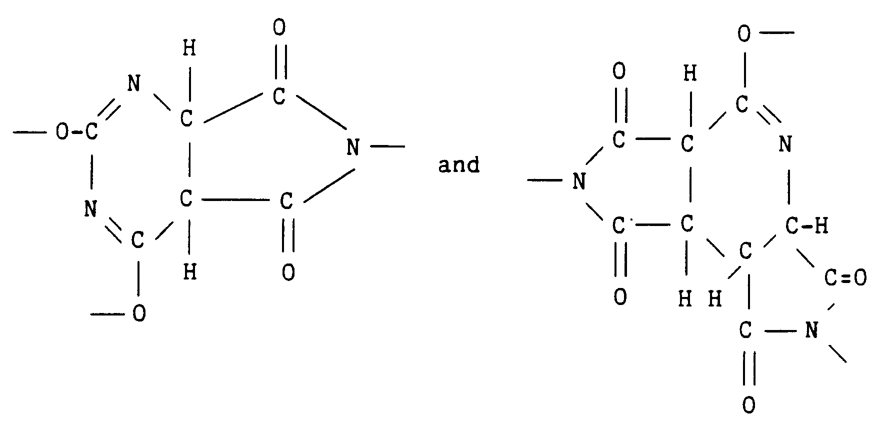 unless other functionalities are present in the polycyanate that participate in the curing process.

### POLYCYANATES OR POLYCYANAMIDES WHICH DO NOT CONTAIN MESOGENIC OR RODLIKE MOIETIES AND WHICH CAN BE EMPLOYED IN THE CURABLE AND CURED COMPOSITIONS

Suitable polycyanates or polycyanamides which do not contain mesogenic or rodlike structures and which can be employed to prepare the polymerizable mixtures of the present invention include, for example, those represented by the following Formulas XIV, XV, XVI and XVII: 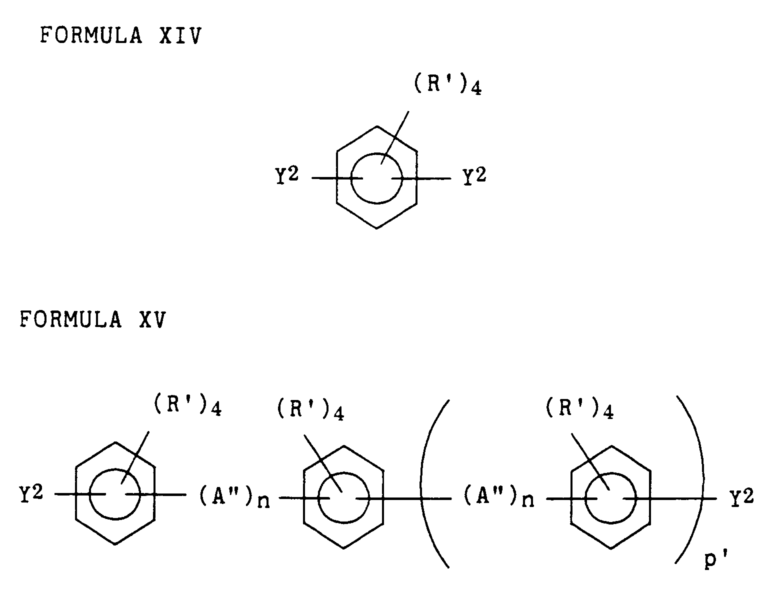 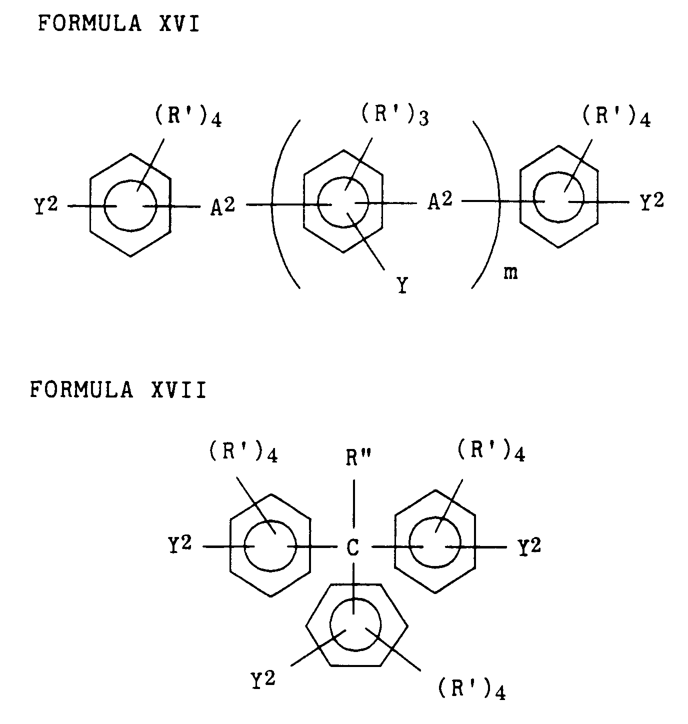 wherein A'' and n are as hereinbefore defined; each Y² is a -O-C≡N or a -N-R¹-C≡N group; each A² is independently an alkylene group having from 1 to 10, preferably from 1 to 4 carbon atoms or a 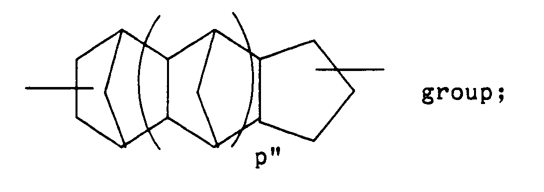 group;
each R' is independently hydrogen, a hydrocarbyl or hydrocarbyloxy group having from 1 to 10, preferably 1 to 4 carbon atoms, a halogen, preferably chlorine or bromine, a phenyl group, a -O-C≡N group, or a -N-R¹-C≡N group; wherein R¹ is as hereinbefore defined; each R'' is independently hydrogen, a hydrocarbyl or hydrocarbyloxy group having from 1 to 10, preferably 1 to 4 carbon atoms, a halogen, preferably chlorine or bromine, or a phenyl group; p' has a value from zero to 100, preferably from zero to 30; p'' has a value of from zero to 10, preferably from zero to 3 and m has a value of from 0.001 to 6, preferably from 0.01 to 3. The aromatic rings can also contain one or more heteroatoms selected from, for example, N, O or S.

Suitable polycyanates or polycyanamides which do not contain mesogenic or rodlike structures represented by Formulas XIV, XV, XVI and XVII include, for example, bisphenol A dicyanate, the dicyanates of 4,4'-dihydroxydiphenyl, 4,4'-dihydroxydiphenyl oxide, resorcinol, hydroquinone, 4,4'-thiodiphenol, 4,4'-sulfonyldiphenol, 3,3',5,5'-tetrabromobisphenol A, 2,2',6,6'-tetrabromobisphenol A, 2,2'-dihydroxydiphenyl, 3,3'-dimethoxybisphenol A, 4,4'-dihydroxydiphenylcarbonate, dicyclopentadiene diphenol, 4,4'-dihydroxybenzophenone, 4,4'-dihydroxydiphenyl methane, tricyclopentadiene diphenol, the tricyanate of tris(hydroxyphenyl)methane, the tetracyanate of 2,2',4,4'-tetrahydroxydiphenyl methane, the polycyanate of a phenolformaldehyde condensation product (novolac), the polycyanate of a dicyclopentadiene and phenol condensation product, the dicyanamide of 4,4'-diaminodiphenyl methane and the cyanate cyanamide of p-aminophenol.

The polycyanates or polycyanamides which do not contain mesogenic or rodlike structures are prepared using the corresponding polyphenol, polyamine or aminophenol precursor and the previously described cyanation (cyanamidation) chemistry.

### EPOXY RESINS WHICH CAN BE EMPLOYED IN THE CURABLE AND CURED COMPOSITIONS

Suitable epoxy resins which can be employed to prepare the polymerizable mixtures of the present invention include materials having an average of more than one vicinal epoxide group per molecule, for example, the epoxy resins represented by the following Formulas XVIII, XIX, XX, XXI, XXII, XXIII, XXIV, XXV, XXVI, XXVII: 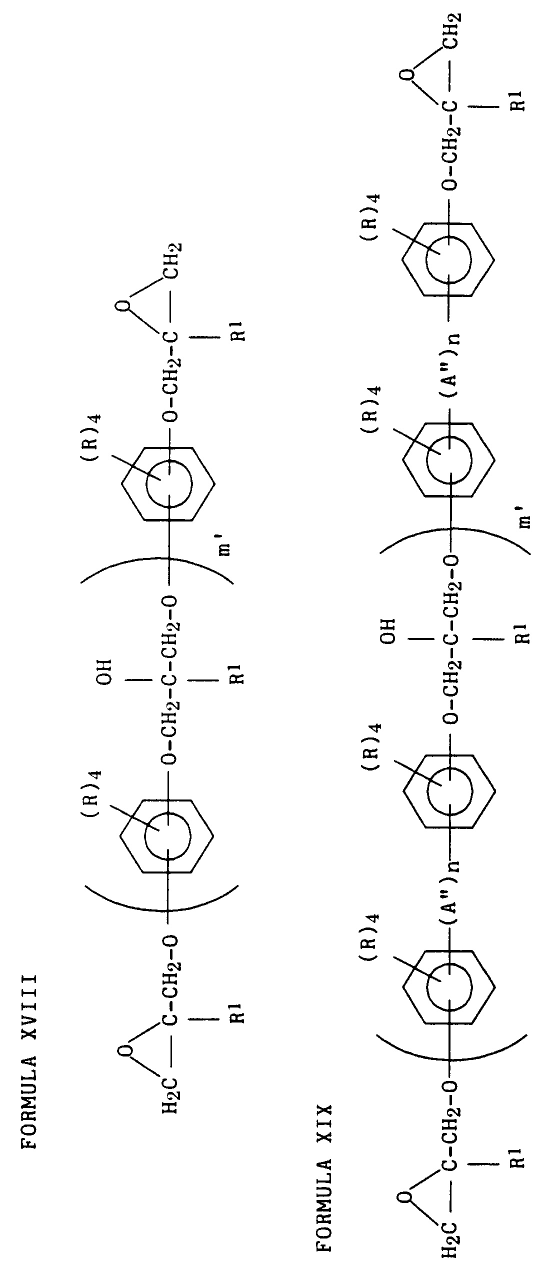 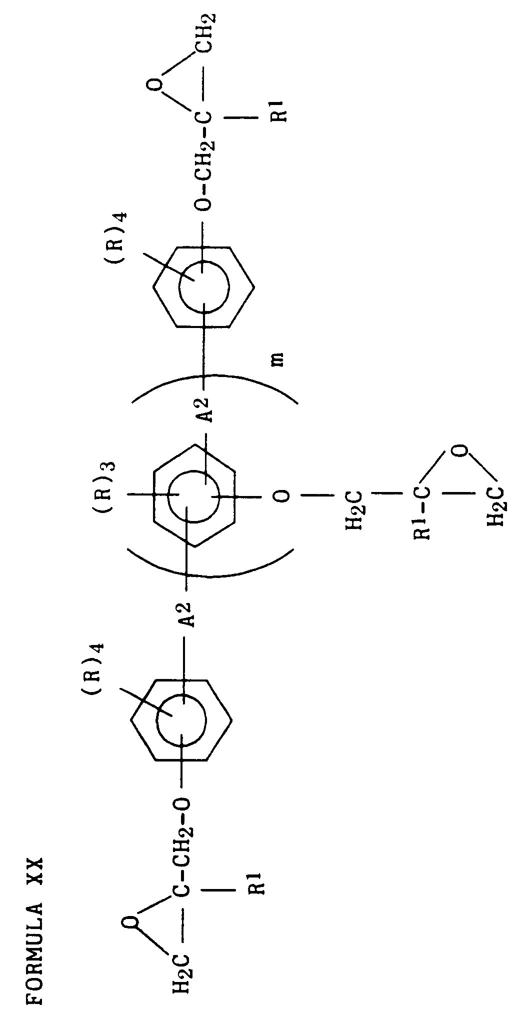 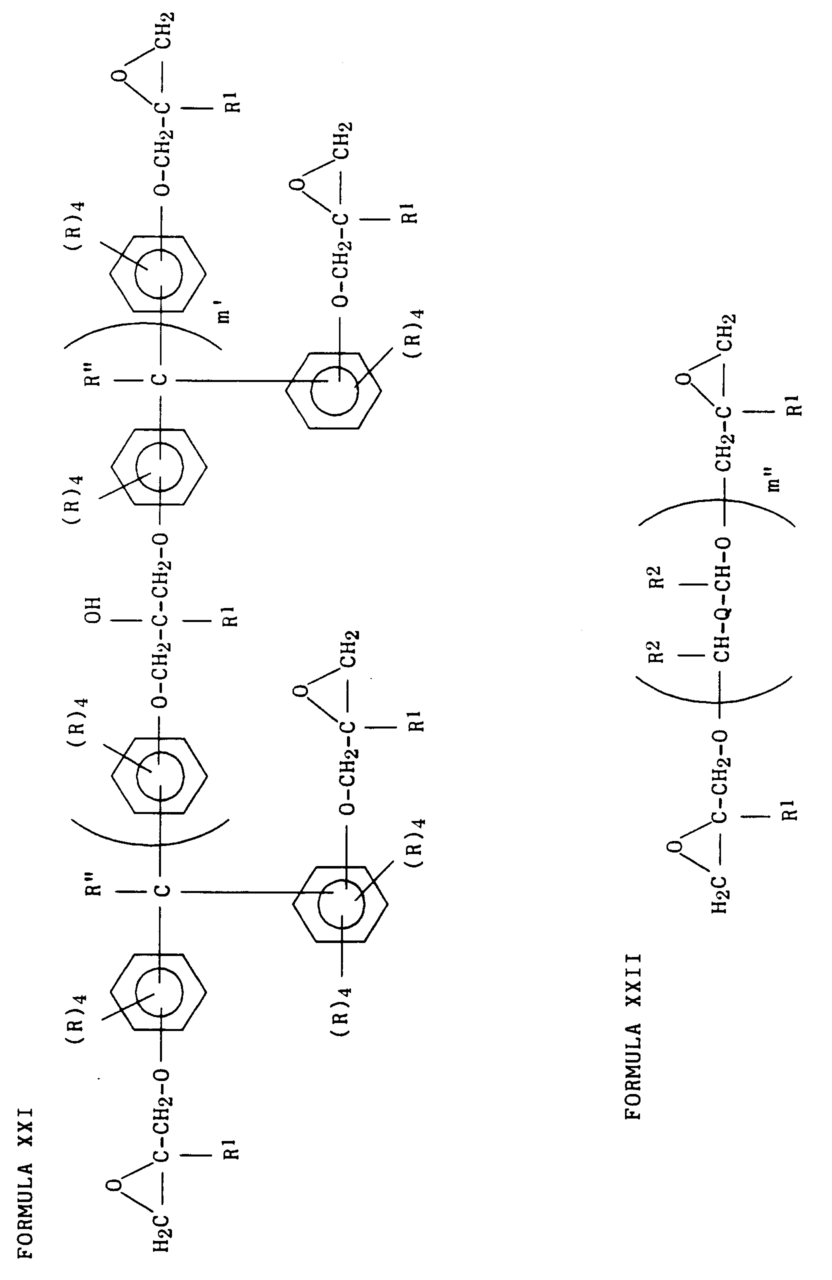 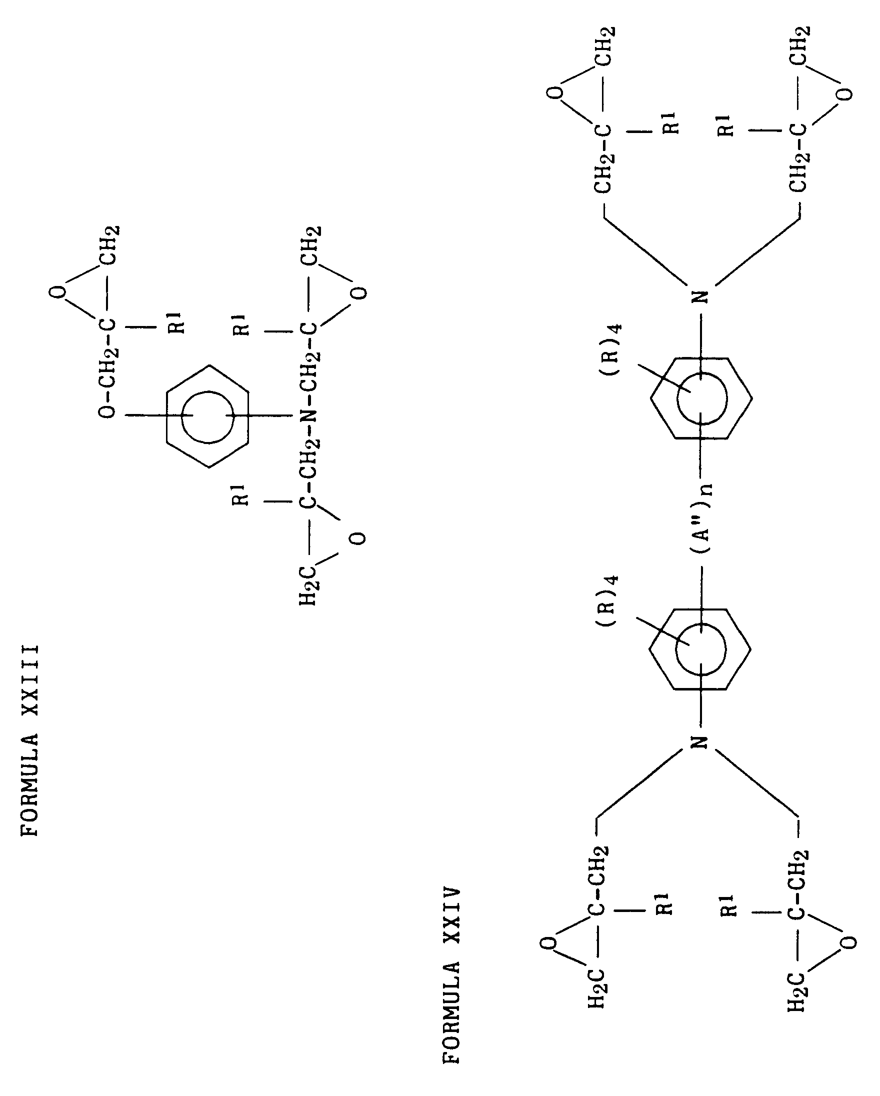 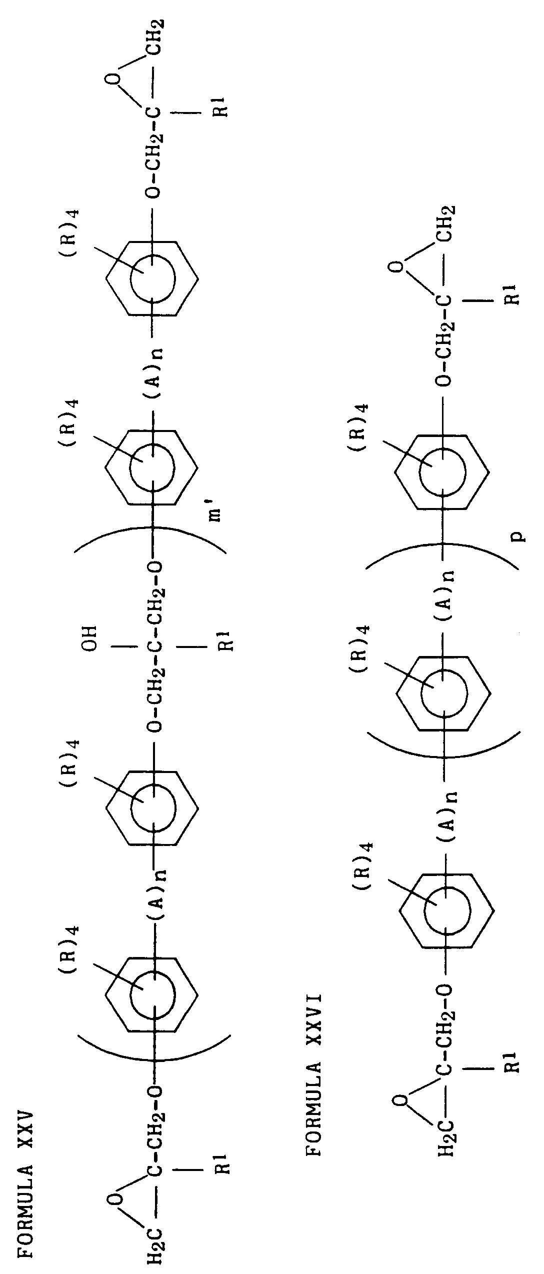 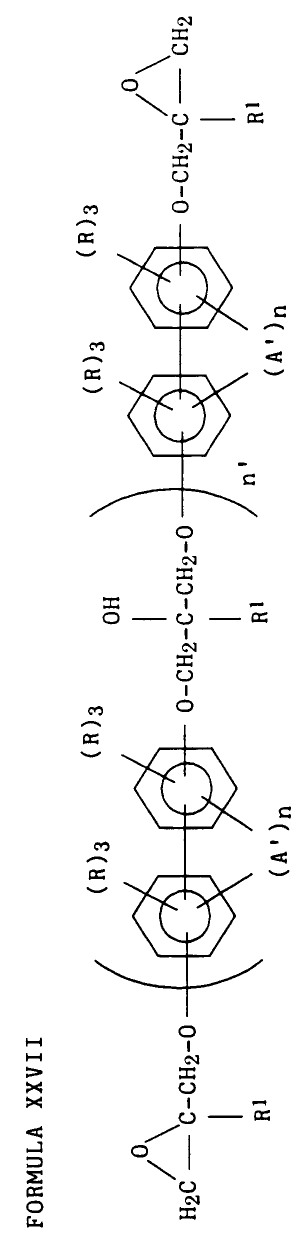 wherein A, A², A', A'', R, R¹, R'', m, n, n' and p and are as hereinbefore defined; each R² is independently hydrogen, or a hydrocarbyl or halohydrocarbyl group having from 1 to 6, preferably 1 to 2 carbon atoms; Q is a direct bond, -CH₂-S-CH₂-, -(CH₂)_{n''}-, or 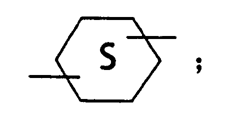 m' has a value of from zero to 30, preferably from zero to 5; m'' has a value from 1 to 10, preferably from 1 to 4 and n'' has an average value from 1 to 10. The aromatic rings can also contain one or more heteroatoms selected from, for example N, O or S.

Particularly suitable epoxy resins represented by formulas XXVIII, XIX, XX, XXI, XXII, XXIII, XXIV, XXV, XXVI and XXVII are, for example, the diglycidyl ethers of resorcinol, hydroquinone, dihydroxydiphenyl methane, bisphenol A, 3,3',5,5'-tetrabromobisphenol A, 4,4'-sulfonyldiphenol, 4,4'-thiodiphenol, 4,4'-dihydroxydiphenyl oxide, 4,4'-dihydroxybenzophenone, 2,2'-dihydroxydiphenyl, dicyclopentadiene diphenol, tricyclopentadiene diphenol, 4,4'-dihydroxydiphenyl, 4,4'-dihydroxystilbene, 4,4'-dihydroxy-alpha-methylstilbene, 4,4'-dihydroxy-alpha-cyanostilbene, 4,4'-dihydroxychalcone, 4,4'-dihydroxydiphenylacetylene, 4,4'-dihydroxydiphenylazomethine, 4,4'-dihydroxyazobenzene, 4,4'-bis(4-hydroxyphenoxy)diphenyl, 4,4'-dihydroxybenzanilide, ethylene glycol, thiodiglycol, diethylene glycol, dipropylene glycol, polypropylene glycol, polyethylene glycol, 1,4-cyclohexanediol, dibutylene glycol, the advancement reaction product of the diglycidyl ether of bisphenol A and bisphenol A, the advancement reaction product of the diglycidyl ether of 4,4'-dihydroxy-alpha-methylstilbene and 4,4'-dihydroxy-alpha-methylstilbene, the triepoxide of p-aminophenol, the tetraepoxide of 4,4'-diaminodiphenyl methane, the triglycidyl ether of tris(hydroxyphenyl)methane, the tetraglycidyl ether of 2,2',4,4'-tetrahydroxydiphenyl methane, the polyglycidyl ether of a phenolformaldehyde condensation product (novolac), the polyglycidyl ether of a condensation product of dicyclopentadiene or oligomer thereof and phenol or halogen or alkyl substituted phenol.

The aforementioned epoxy resins can be prepared by reaction of a polyphenol (polyamine, aminophenol, polyalkylene glycol) with an epihalohydrin and a basic acting material. Said reaction generally involves two distinct steps: coupling reaction of the epihalohydrin and polyphenol to provide a halohydrin intermediate and dehydrohalogenation reaction of the halohydrin intermediate to provide the glycidyl ether product. Suitable catalysts and reaction conditions for preparing epoxy resins are described in the Handbook of Epoxy Resins by Lee and Neville, McGraw-Hill (1967).

### POLYMALEIMIDES FOR USE IN THE CURABLE AND CURED COMPOSITIONS

Suitable polymaleimides which can be employed to prepare the polymerizable mixtures of the present invention include, for example, those represented by the following formulas XXVIII, XXIX, XXX, XXXI, XXXII, XXXIII, XXXIV and XXXV: 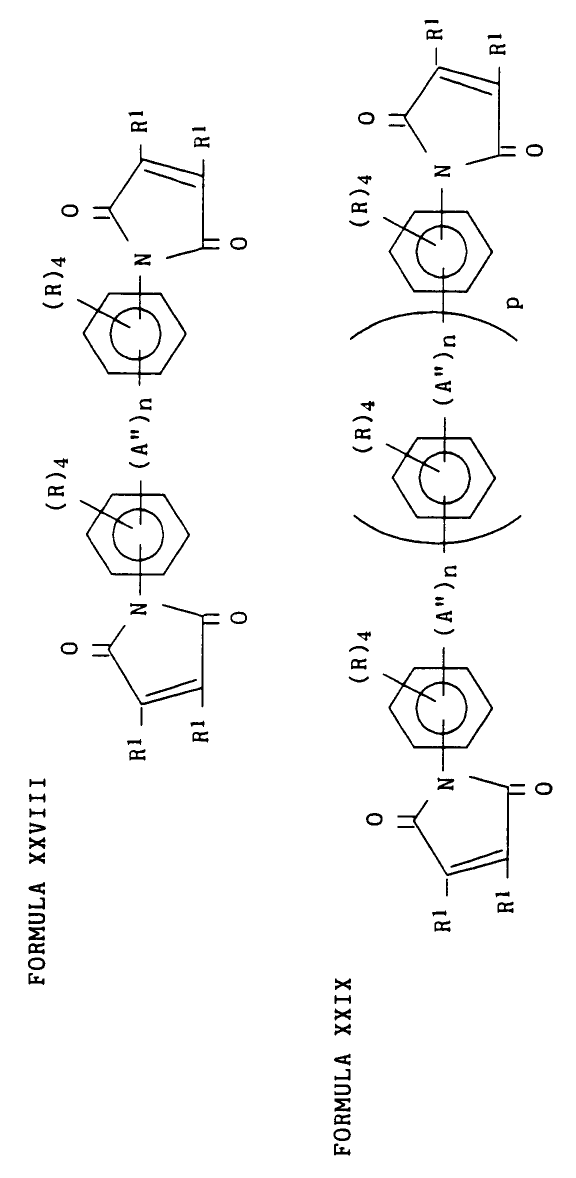 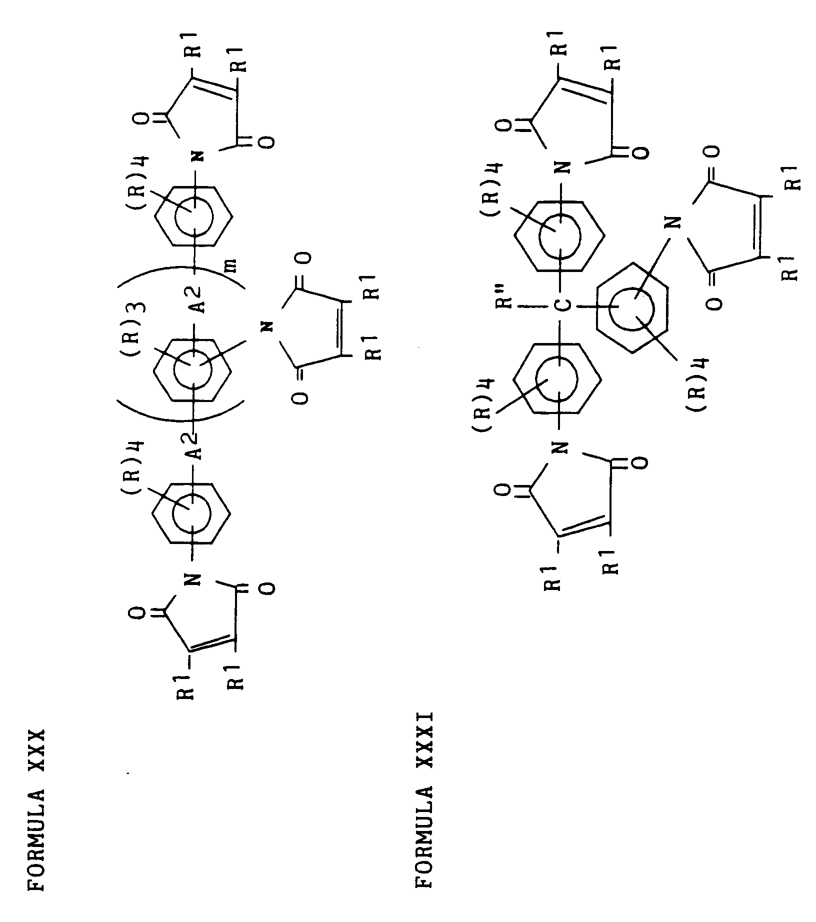 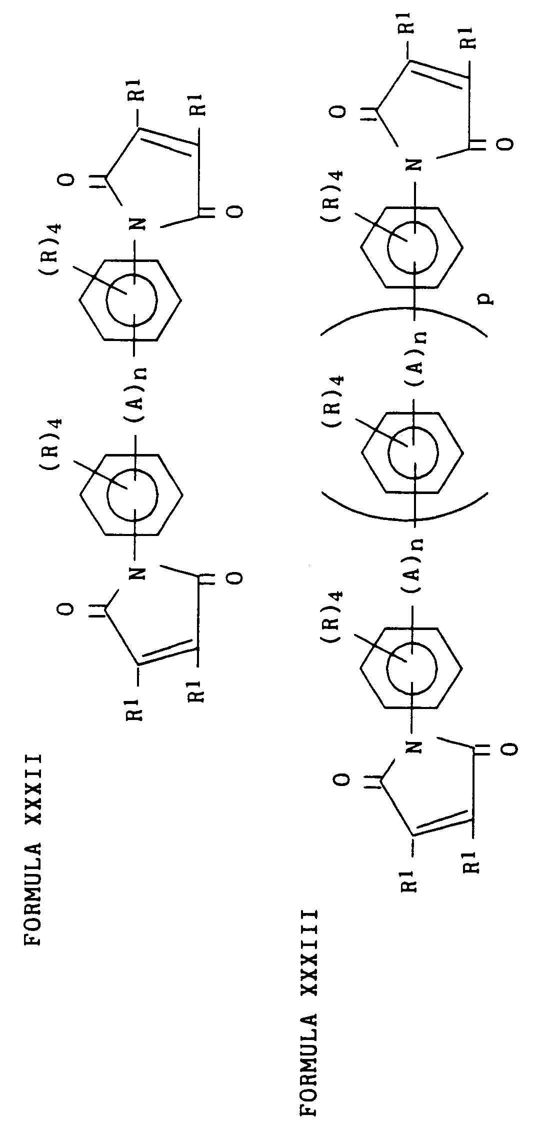 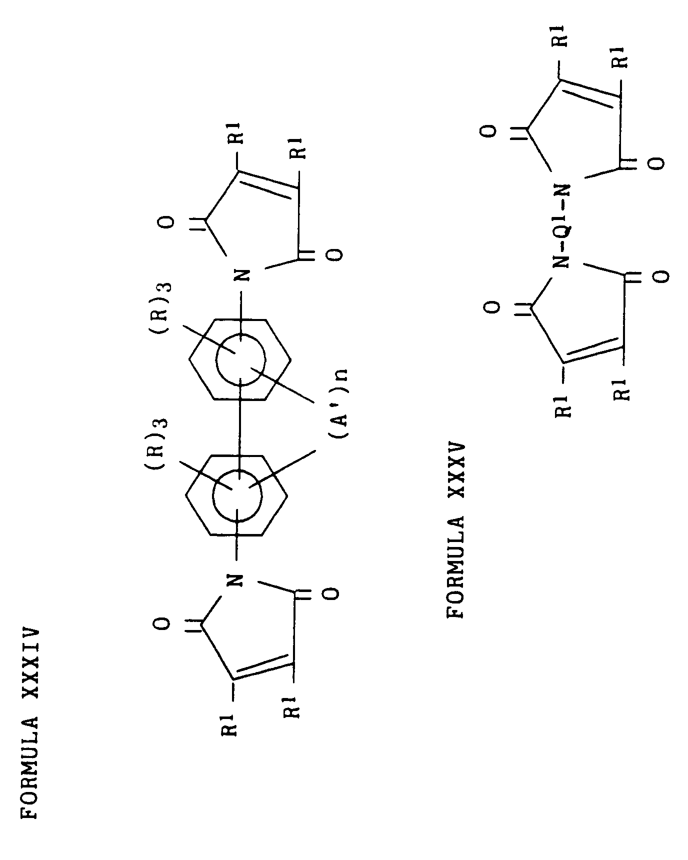 wherein A, A², A', A'', R, R¹, R'', m, n and p are as hereinbefore defined and Q¹ is a divalent hydrocarbyl group having from 2 to 12 carbon atoms and may be linear or branched aliphatic, cycloaliphatic or polycycloaliphatic. The aromatic rings can also contain one or more heteroatoms selected from, for example, N, O or S.

Particularly suitable polymaleimides represented by Formulas XXVIII, XXIX, XXX, XXXI, XXXII, XXXIII, XXXIV and XXXV are, for example, N,N'-ethylenebismaleimide, N,N'-ethylenebis(2-methylmaleimide), N,N'-hexamethylenebismaleimide, N,N'-(oxydi-p-phenylene)bismaleimide, N,N'-(methylenedi-p-phenylene)bismaleimide, N,N'-(methylene-di-p-phenylene)bis(2-methylmaleimide), N,N'-(thio-di-p-phenylene)bismaleimide, N,N'-(sulfonyldi-m-phenylene)bismaleimide, N,N'-(isopropylidenedi-p-phenylene)bismaleimide, polymethylene polyphenylene polymaleimides, the bismaleimide of 4,4'-diaminostilbene and the bismaleimide of 4,4'-diaminobenzanilide.

The polymaleimides can be prepared by reacting a stoichiometric quantity of a maleic anhydride per amine group with a polyamine in the presence of a suitable solvent, such as aromatic hydrocarbons, chlorinated hydrocarbons or N,N-dimethylformamide. The polymaleamic acid resulting from reaction of a maleic anhydride and a polyamine may be isolated and dehydrated to the desired polymaleimide. Alternately, the reaction may be performed in a single continuous step. Detailed procedures for preparing polymaleimides can be found in U.S. Patent Nos. 2,444,536 and 2,462,835; and Journal of Polymer Science: Part A: Polymer Chemistry, Vol. 27, pages 375-388 (1989).

### POLYAMINES SUITABLE FOR USE IN THE CURABLE AND CURED COMPOSITIONS

Suitable polyamines which can be employed to prepare the polymerizable mixtures of the present invention include those containing one or more of the mesogenic or rodlike structure(s) already described herein as well as any of the other known polyamines which do not contain mesogenic or rodlike structures. Typical representatives of said polyamines free of mesogenic or rodlike structures include, for example, 1,4-diamino-butane, 1,6-hexanediamine, 1,12-diaminododecane, 2-methyl-4-ethyl-1,8-diaminooctane, 1,4-diamino-cyclohexane, 4,4'-diaminodiphenyl methane and 1,4-diaminobenzene, tris(aminophenyl)methane, anilineformaldehyde condensation products.

### POLYPHENOLS SUITABLE FOR USE IN THE CURABLE AND CURED COMPOSITIONS

Suitable polyphenols which can be employed to prepare the polymerizable mixtures of the present invention include those containing one or more of the mesogenic or rodlike structure(s) already described herein as well as any of the other known polyphenols which do not contain mesogenic or rodlike structures. Typical representatives of said polyphenols free of mesogenic or rodlike structures include, for example, resorcinol, 4,4'-sulfonyldiphenol, 4,4'-dihydroxydiphenyl oxide, tris(hydroxyphenyl)methane and phenolformaldehyde condensation products.

### POLYMERIZABLE UNSATURATED MONOMERS SUITABLE FOR USE IN THE CURABLE AND CURED COMPOSITIONS

Suitable compounds containing one or more polymerizable ethylenically unsaturated group(s) which can be employed to prepare the polymerizable mixtures of the present invention include both those containing one or more mesogenic or rodlike structure(s) and those free of said structures.

Suitable polymerizable ethylenically unsaturated monomers containing one or moe mesogenic or rodlike moieties are cataloged by Alexandre Blumstein in Liquid Crystalline Order in Polymers, published by Academic Press, New York (1978) on pages 105-140; Mesomorphic Order in Polymers and Polymerization in Liquid Crystalline Media published by American Chemical Society (ACS Symposium Series 74), Washington, D.C. (1978) on pages 56-70; and N. A. Plate and V. P. Shibaev in Comb-Shaped Polymers and Liquid Crystals published by Plenum Press, New York (1987) on pages 1-415; V. Percec, et. al., Polymer Bulletin, 17, pages 347-352 (1987); R. Duran and P. Gramain, Makromol. Chem., 188, pages 2001 - 2009 (1987); A.M. Mousa, et. al., Polymer Bulletin, 6, pages 485 - 492 (1982); H. Finkelmann, et. al., Makromol. Chem., 179, pages 829 - 832 (1978); M. Portugall, et. al., Makromol. Chem., 183, pages 2311-2321 (1982) and U.S. Patent Nos. 4,637,896 and 4,614,619. Suitable polymerizable ethylenically unsaturated monomers containing one or more mesogenic or rodlike moieties per molecule are represented by the following Formulas XXXVI or XXXVII:

M-Q² FORMULA XXXVI

M-(Q³)ₙ-R³-Q² FORMULA XXXVII

wherein n is as hereinbefore defined, M is a group containing two or more aromatic rings bridged by a rigid central linkage, R³ is a divalent hydrocarbon group having from one to 12 carbon atoms and may be linear, branched, cyclic, aromatic or a combination thereof and may be substituted with one or more inert groups, such as a methoxy group, or may contain one or more inert heteroatom containing linkages, such as, for example, an ether linkage; Q³ is -O-, -NR¹-, -S-, -O-CO-, -CO-O-, -NR¹-CO-, -CO-NR¹-, -CO-, -O-CO-O-, -S-CO-, -CO-S-, -NR¹-CO-O-, -O-CO-NR¹-, -NR¹-CO-NR¹- wherein R¹ is as hereinbefore defined; and Q² is a polymerizable ethylenically unsaturated group. As a class, these monomers generally contain a -CH=CH₂, allyl, methallyl, propenyl, isopropenyl, acrylate or methacrylate group as the polymerizable ethylenically unsaturated group and a linear divalent aliphatic, aliphatic ether, aliphatic polyether, aliphatic thioether or cycloaliphatic flexible spacer connecting the polymerizable ethylenically unsaturated group and the mesogenic or rodlike group(s) through a heteroatom linkage. Typical mesogenic or rodlike groups include those wherein two or more aromatic rings are bridged by a rigid central linkage wherein said rigid central linkage is required to bridge the aromatic rings to provide at least about 80 percent para substitution. The aromatic rings can be inertly substituted, however, unsubstituted aromatic rings which maximize the molecular aspect ratio are preferred. Also preferred is a single inert substituent in the para position on the ring not connected to the polymerizable ethylenically unsaturated group (either directly or via a flexible spacer). This type of substituent can be used to enhance the molecular aspect ratio. Typical of these inert substituents are, for example, CH₃O-, Cl-, NO₂- and, -C≡N. The aromatic rings can also contain one or more heteroatoms selected from, for example, N, O or S. Typical rigid central linkage groups for bridging the aromatic rings include, for example, a direct bond, -CR¹=CR¹-, -C≡C-, -N=N-, -CR¹=N-, -CR¹=N-N=CR¹-, -CR¹=CR¹-CO-, -O-CO-, -NR¹-CO-, -CO-O-, -CO-NR¹-, -CO-CR¹=CR¹-, -CR¹=CR¹-O-CO-(CH₂)_{n′}-, -N=CR¹-, -(CH₂)_{n′}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n′}-, -S-CO-, -(CH₂)_{n′}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 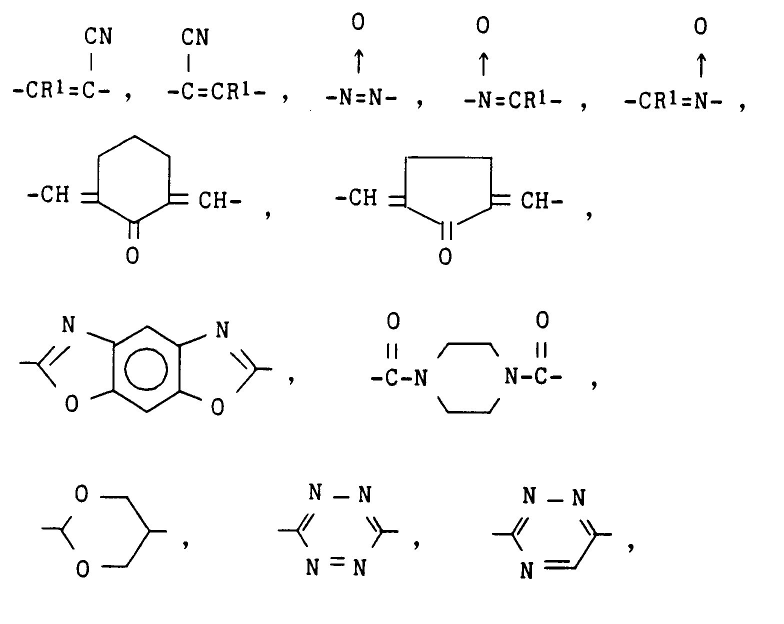 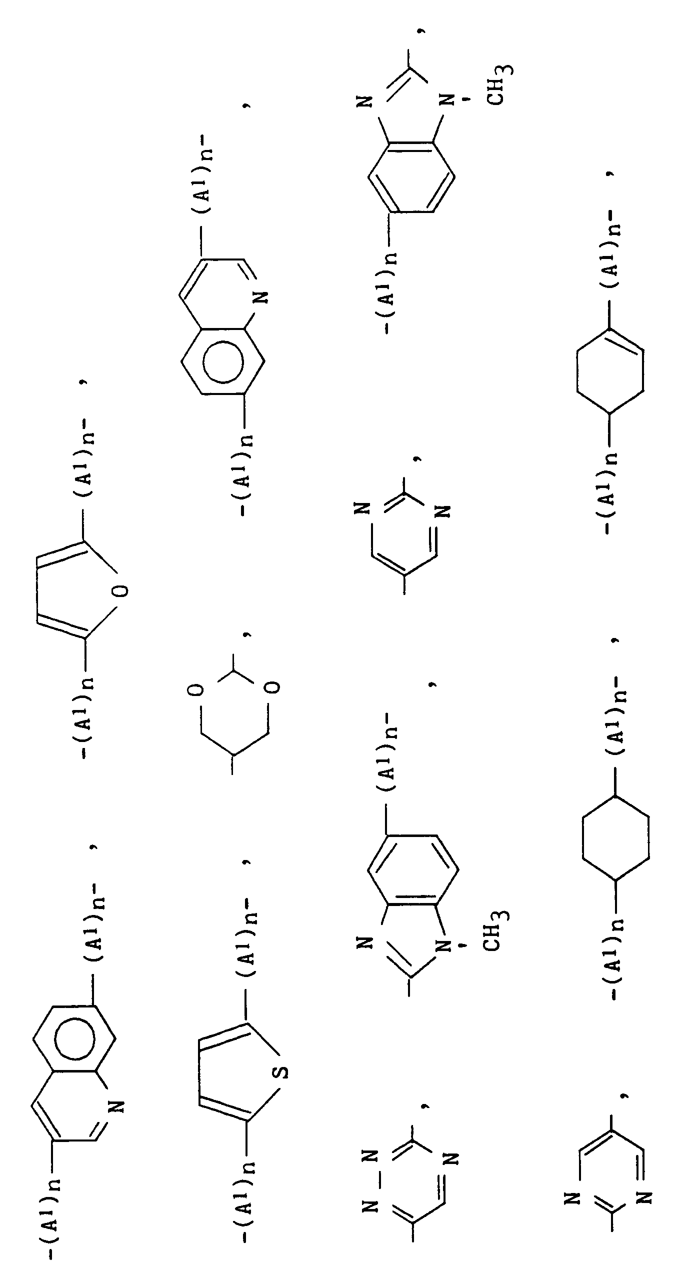 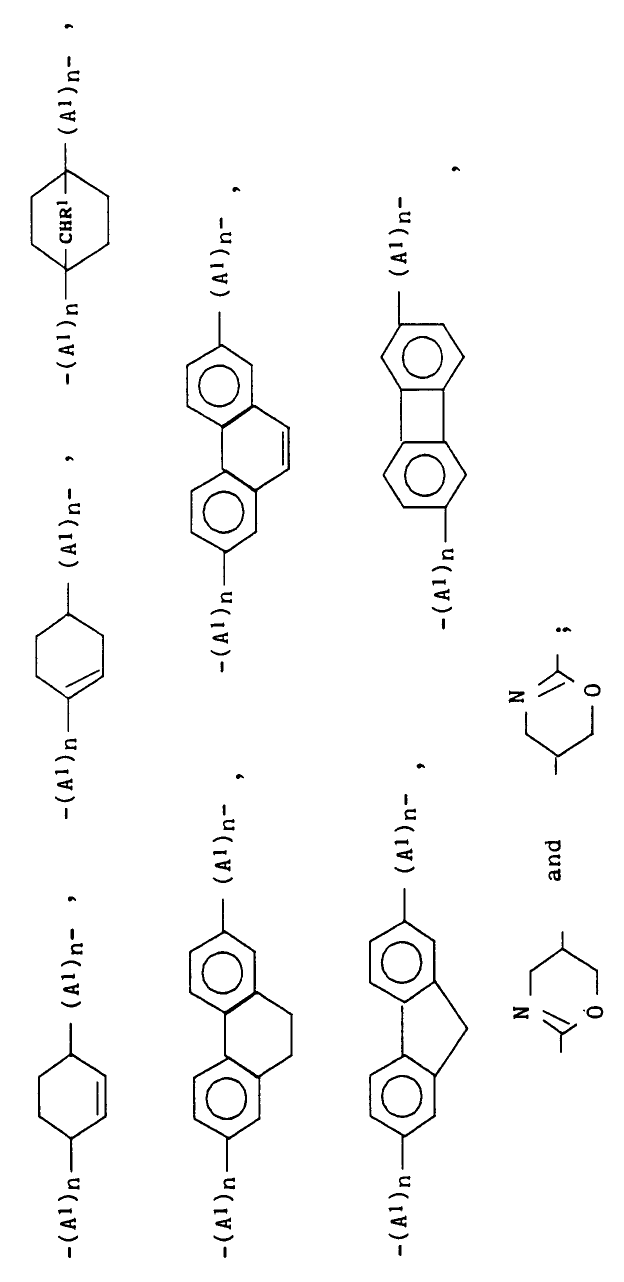 wherein R¹, A¹, n and n' are as hereinbefore defined. As is well known in the prior art, all or a part of the aromatic rings can be replaced with other promesogenic structures, such as the trans-cyclohexane ring or a cholesterol group. Additionally, it has been demonstrated in the prior art that efficacious mesogenic or rodlike group containing polymerizable ethylenically unsaturated monomers can be prepared with omission of the flexible spacer between the polymerizable ethylenically unsaturated group and the mesogenic or rodlike group(s).

Generally, the ethylenically unsaturated monomers containing -CH=CH₂, acrylate, allyl, methallyl, propenyl, isopropenyl or methacrylate as the polymerizable vinyl group and a linear divalent hydrocarbon group connecting the vinyl group and the mesogenic or rodlike group through heteroatom containing functional groups between the hydrocarbon spacer and the mesogenic group are most preferred. Thus, a mesogenic group ether linked to a -CH₂-CH₂- which is in turn linked to provide a methacrylate ester, that is, 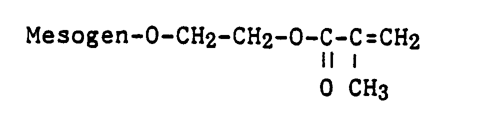 or a mesogenic group linked to a vinyl group, that is, 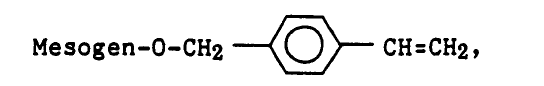 are examples of those species preferred as the ethylenically unsaturated monomer containing one or more mesogenic or rodlike moieties.

Particularly suitable ethylenically unsaturated monomers containing a mesogenic or rodlike moiety include, for example, 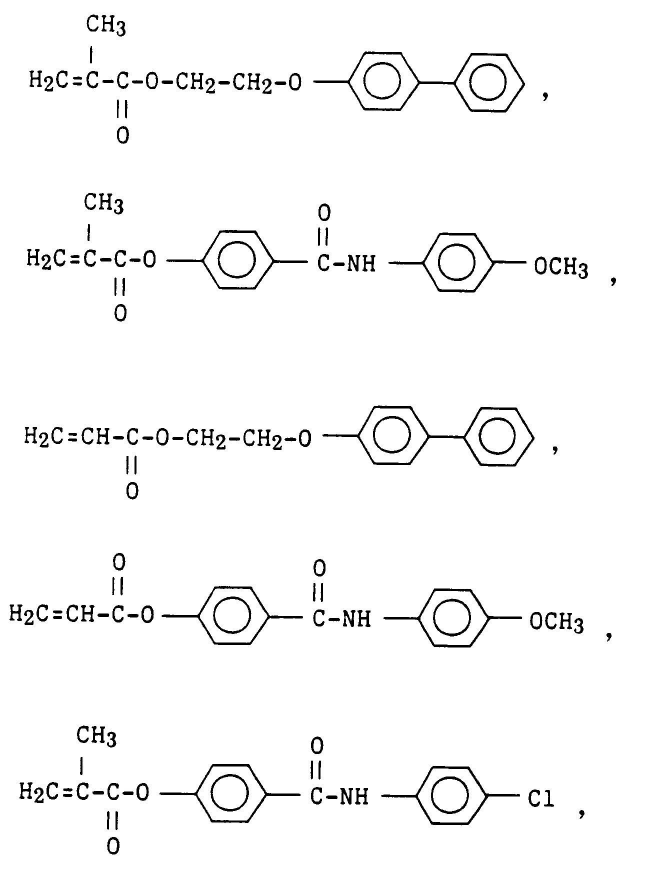 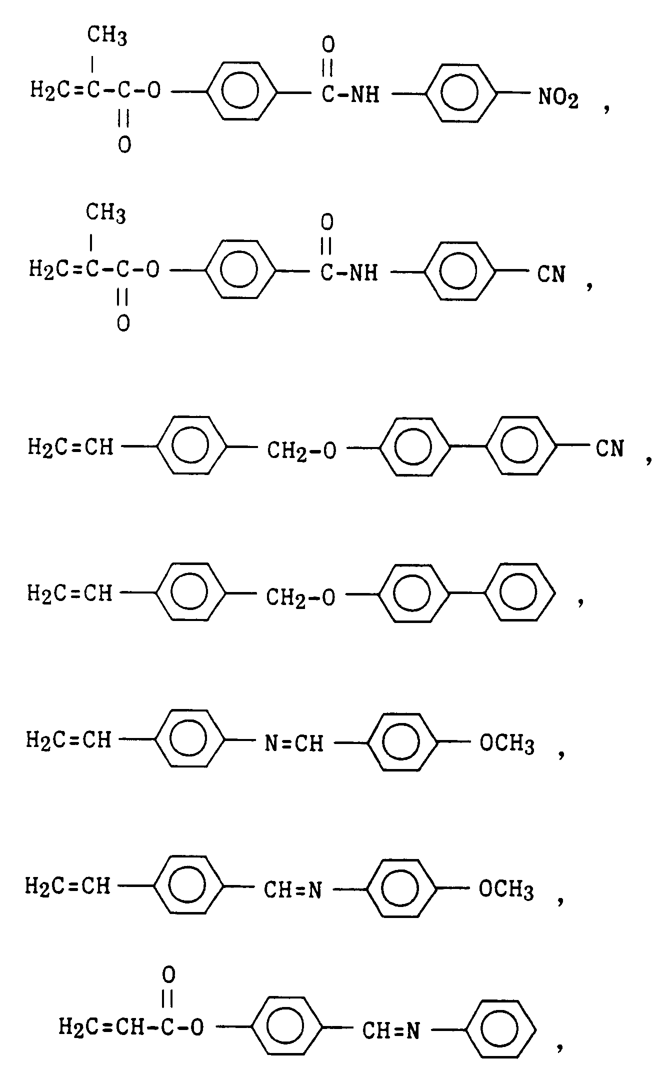 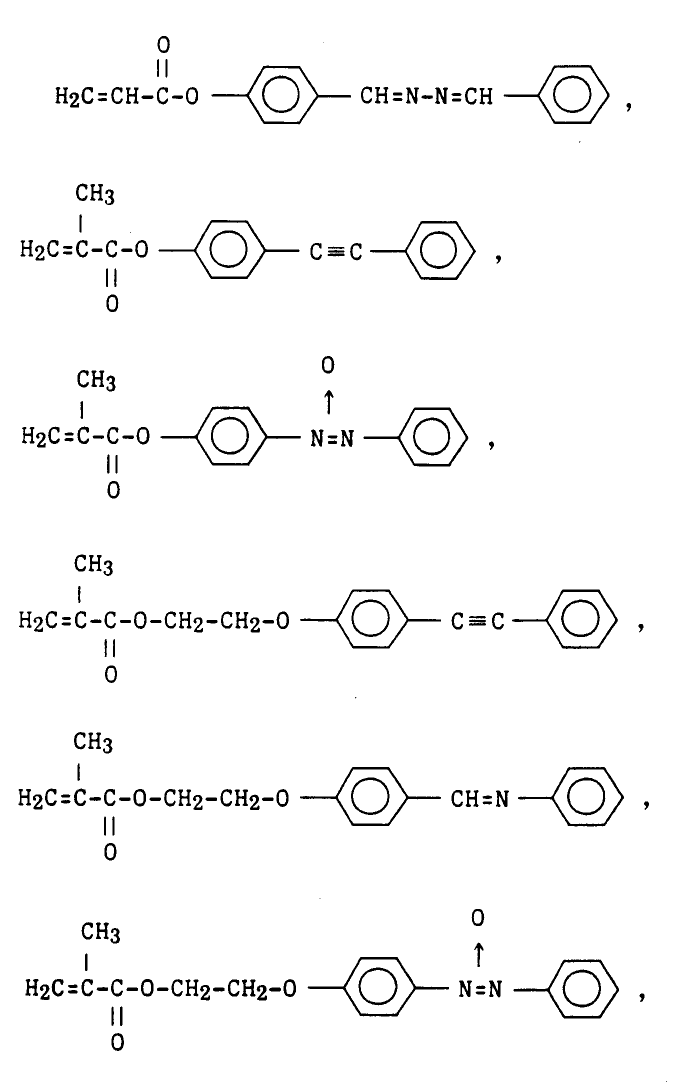 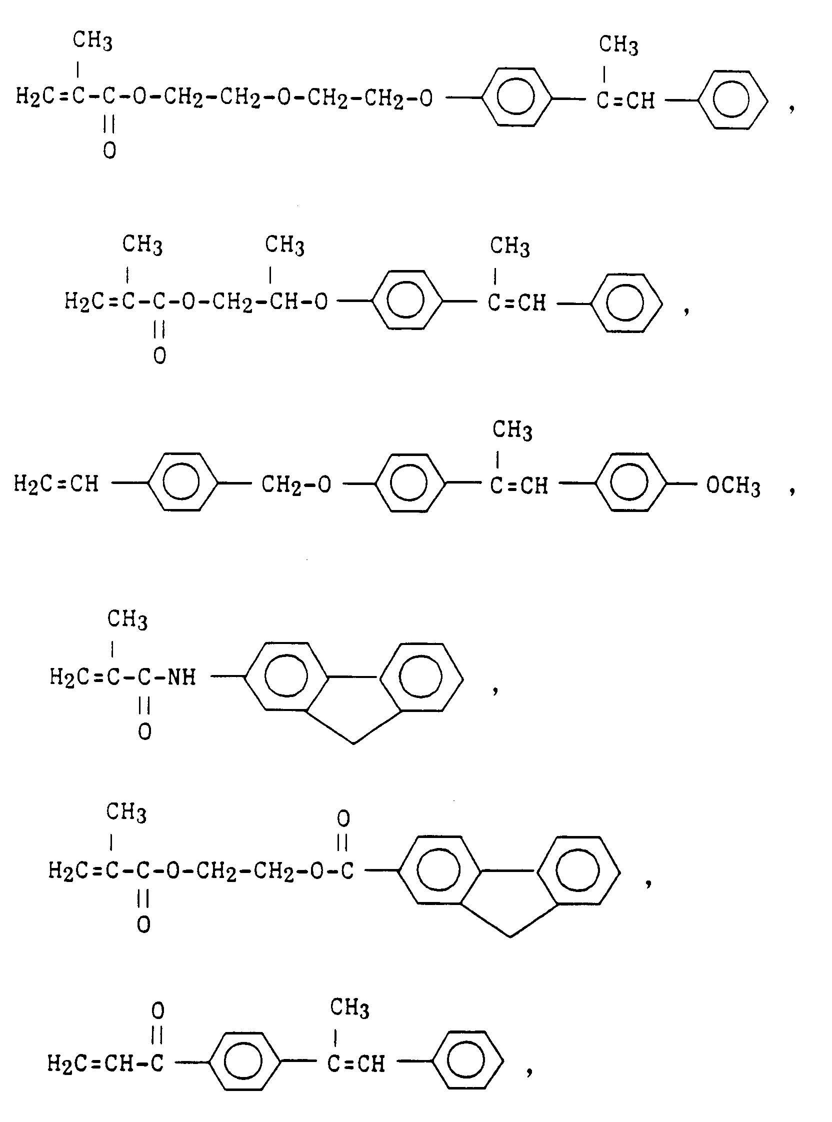 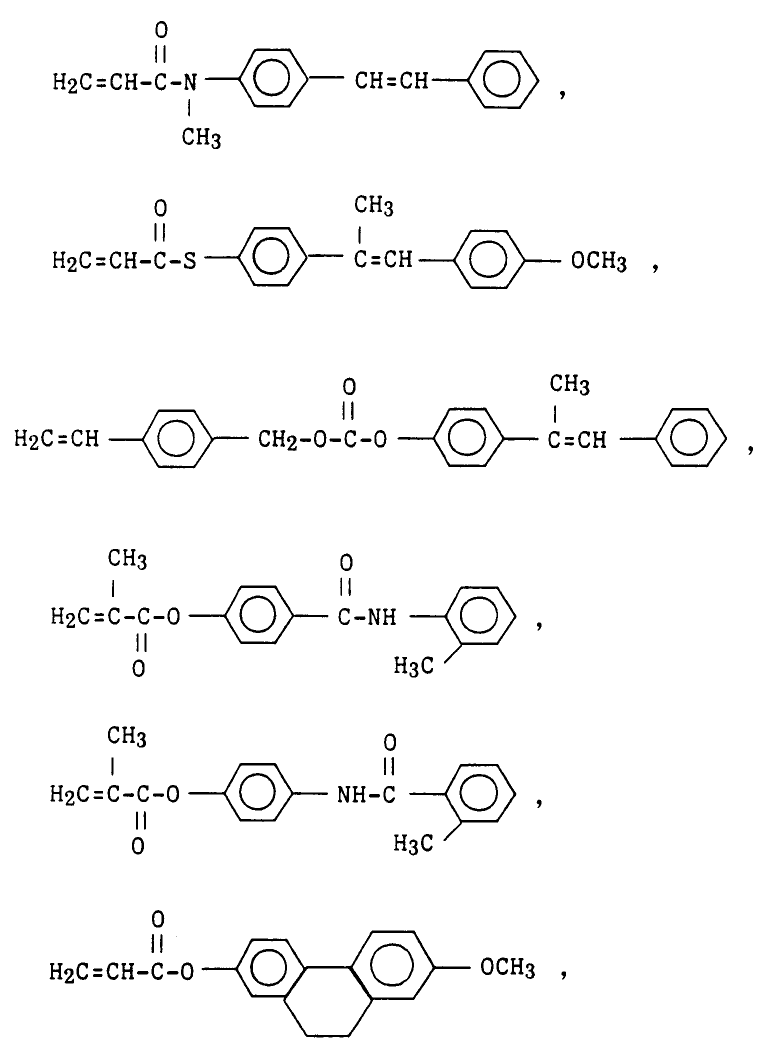 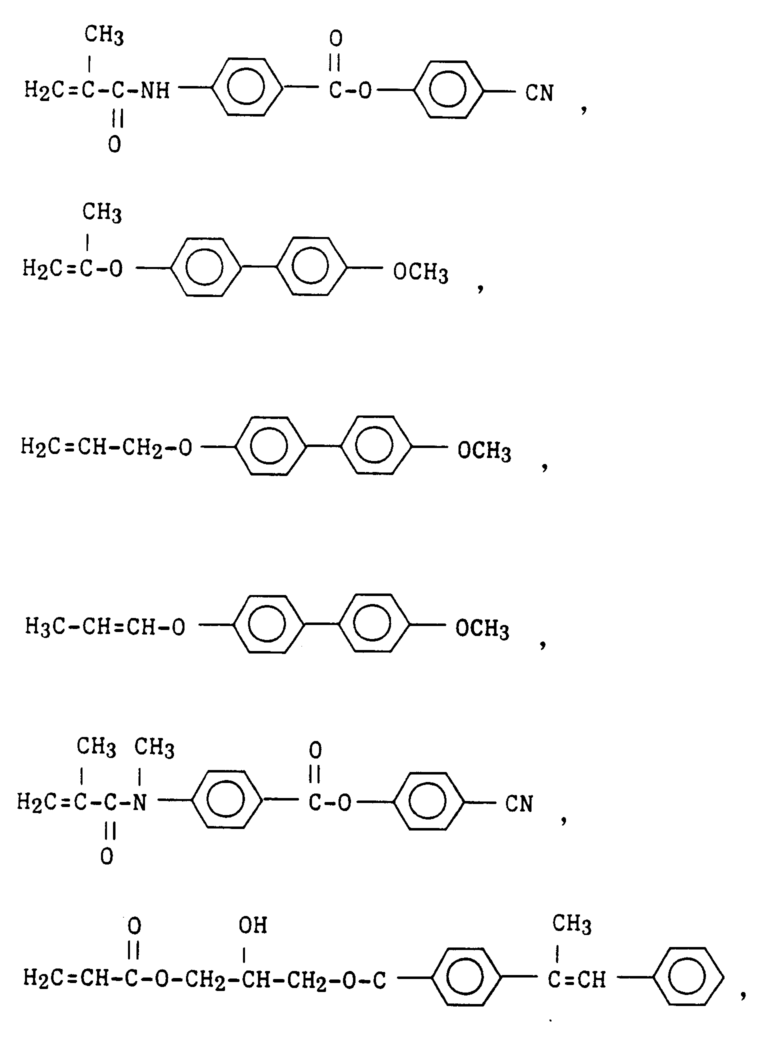 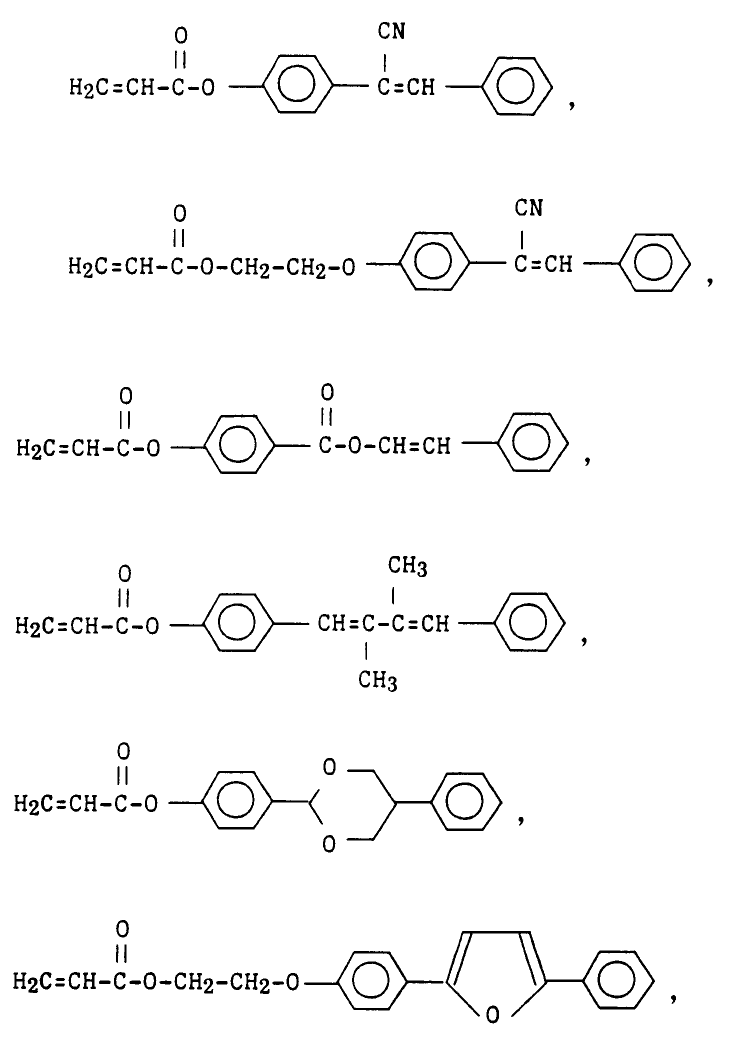 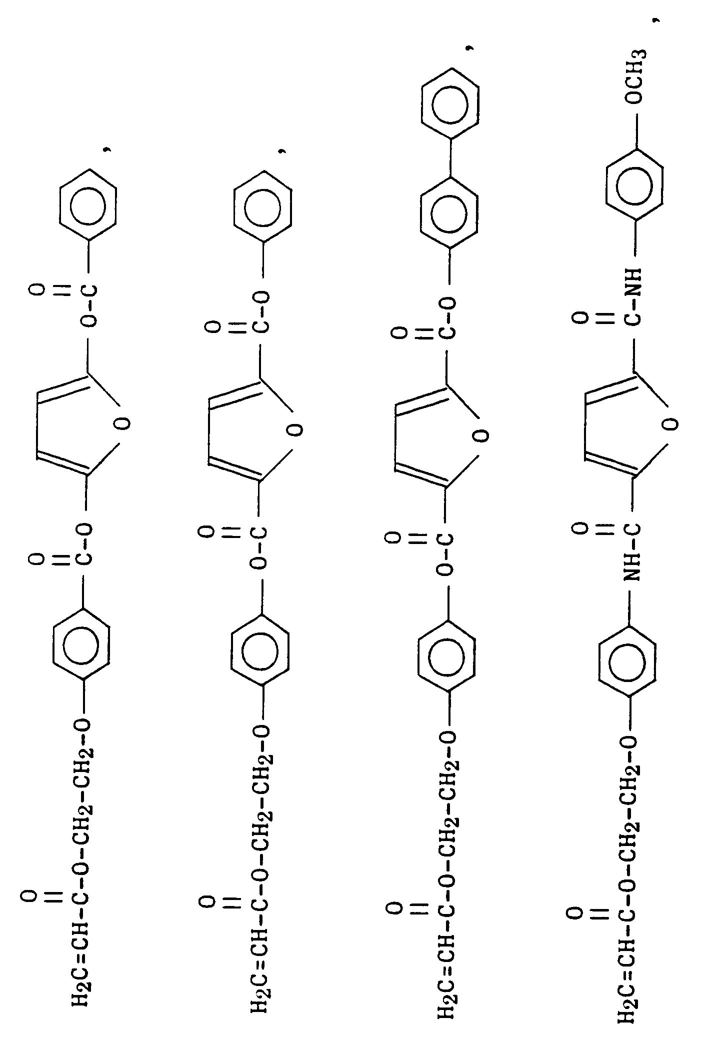 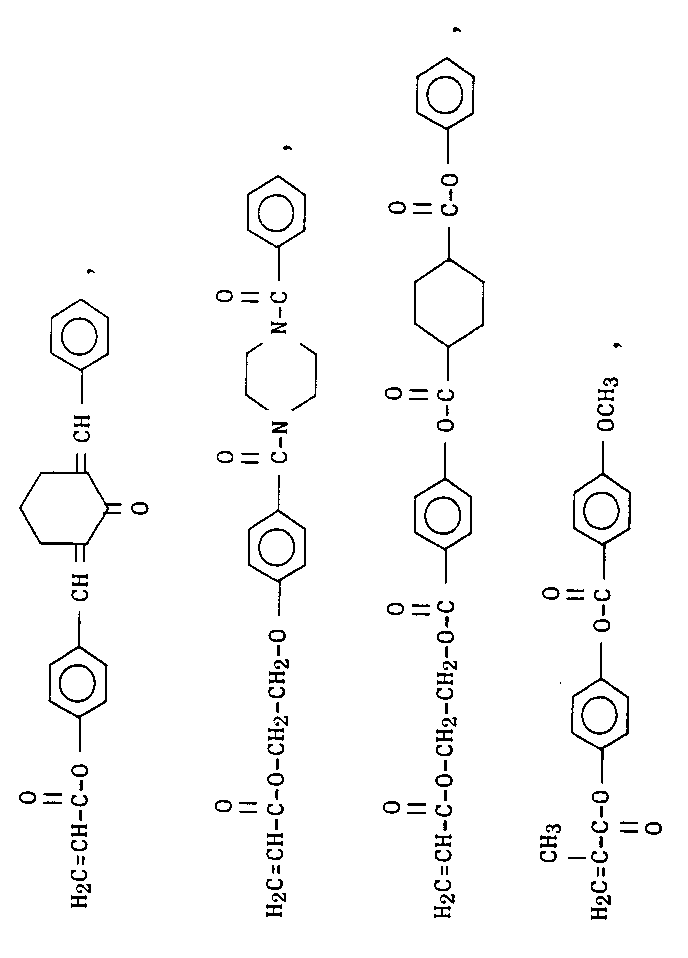 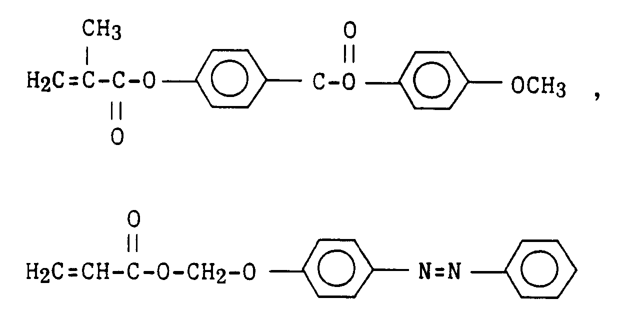 and
any combination thereof.

Suitable polymerizable ethylenically unsaturated monomers which do not contain mesogenic or rodlike structures can be selected from the many known classes of polymerizable vinyl monomers. Suitable such monomers include, for example, the vinyl aromatic compounds represented by the following Formula XXXVIII: 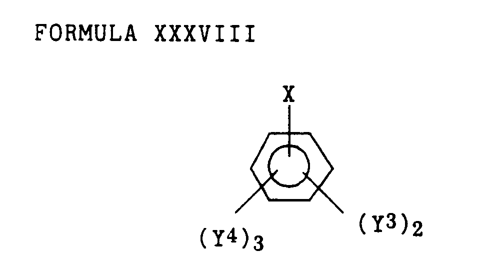 wherein each Y³ is independently hydrogen, a hydrocarbyl or hydrocarbyloxy group having from 1 to 5 carbon atoms, a vinyl group, an allyl group, a methallyl group, a propenyl group, a isopropenyl group, a nitro group, a nitrile group, a halogen, such as chlorine or bromine or fluorine, or a -CO-R¹ group; each Y⁴ is independently hydrogen, a hydrocarbyl or hydrocarbyloxy group having from 1 to 5 carbon atoms, or a halogen, such as chlorine or bromine or fluorine and X is 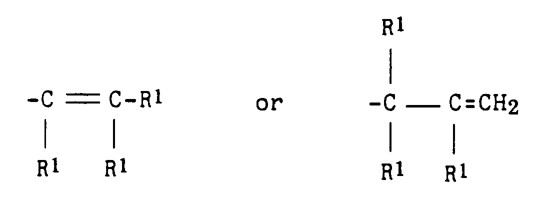 wherein each R¹ is as hereinbefore defined;
or the acrylate or methacrylate compounds represented by the following Formula XXXIX 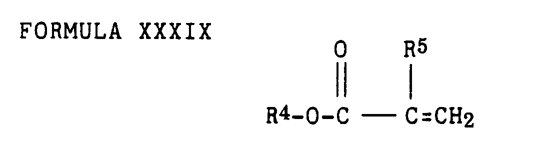 wherein R⁴ is a hydrocarbyl group having from 2 to 25 carbon atoms and may be branched, cyclic, polycyclic, saturated or unsaturated and R⁵ is hydrogen or a methyl group.

Typical polymerizable unsaturated monomers represented by Formula XXXVIII include, for example, styrene, alpha-methylstyrene, o-, m-, p-chlorostyrene; o-, m-, p-bromostyrene; o-, m-, p-tert-butylstyrene; o-, m-, p-methylstyrene; o-, m-, p-methoxystyrene; divinylbenzenes, trivinylbenzenes, o-, m-, p-isopropenylstyrene; o-, m-, p-allylstyrene; o-, m-, p-methallylstyrene; and allylbenzene, methallylbenzene, diallylbenzenes.

Typical acrylate (methacrylate) esters represented by Formula XXXIX include, for example, ethyl acrylate, n-butyl acrylate, n-butyl methacrylate, sec-butyl acrylate, 2-ethylhexyl acrylate, 2-ethylhexyl methacrylate, n-dodecyl acrylate, cyclohexyl acrylate, methylcyclohexyl acrylate, norbornyl acrylate, dicyclopentadiene acrylate and methyldicyclopentadiene acrylate.

Other suitable monomers include, for example, the acidic monomers, such as acrylic and methacrylic acid; the amide monomers, such as acrylamide and N-methyl-acrylamide; the allyl monomers, such as diallylphthalate, triallylisocyanurate, diallylmaleate and dimethallylfumarate; the vinyl halides, such as vinyl chloride and vinyl bromide; the vinyl esters, such as vinyl acetate; the vinyl di and polycyclic aromatics, such as vinyl naphthalene; the vinyl nitriles, such as acrylonitrile; and the hydroxyalkyl acrylates and methacrylates, such as 2-hydroxyethyl acrylate.

### COMPOUNDS CONTAINING BOTH A CYANATE OR CYANAMIDE GROUP AND A POLYMERIZABLE ETHYLENICALLY UNSATURATED GROUP FOR USE IN THE CURABLE AND CURED COMPOSITIONS

Suitable compounds which contain both a cyanate or cyanamide group and a polymerizable ethylenically unsaturated group in the same molecule that can be used to prepare the polymerizable mixtures of the present invention include, for example, those represented by the following Formula XXXX: 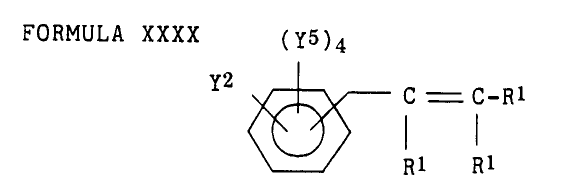 wherein each Y² and R¹ are as hereinbefore defined, Y⁵ is independently hydrogen, a hydrocarbyl or hydrocarbyloxy group having from 1 to 12 carbon atoms, a nitro group, a nitrile group, a halogen, such as chlorine or bromine or fluorine, or a -CO-R¹ group; or a compound represented by the following Formula XXXXI: 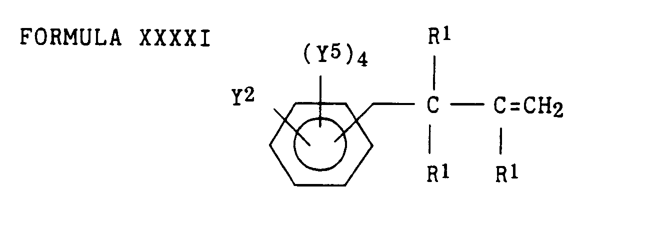 wherein each Y², Y⁵ and R¹ are as hereinbefore defined.

Suitable compounds which contain a cyanate or cyanamide group and a polymerizable ethylenically unsaturated group in the same molecule represented by Formulas XXXX and XXXXI include, for example, o-, m-, p-isopropenylphenyl cyanate; o-, m-, p-vinylphenyl cyanate; methyl-p-isopropenylphenyl cyanates; 3-chloro-4-isopropenylphenyl cyanate; o-, m-, p-propenylphenyl cyanate; o-, m-, p-allylphenyl cyanate; and o-, m-, p-methallylphenyl cyanate. Some of the alkenylphenol precursors to the alkenylphenyl cyanates represented by Formula XXXX, notably the vinylphenols, have a tendency to dimerize or oligomerize thus leading to poly(alkenylphenyl)cyanates. It is most preferred that the alkenylphenyl cyanate be substantially free of dimeric and/or oligomeric components, although it is operable to use an alkenylphenyl cyanate containing substantial (up to 90 percent by weight) dimeric and/or oligomeric components. A specific preparation of p-isopropenylphenyl cyanate is taught in Example 1 of U.S. Patent No. 4,559,399.

### COMPOUNDS CONTAINING BOTH A 1,2-EPOXIDE GROUP AND A POLYMERIZABLE ETHYLENICALLY UNSATURATED GROUP FOR USE IN THE CURABLE AND CURED COMPOSITIONS

Suitable compounds which contain both a 1,2-epoxide group and a polymerizable ethylenically unsaturated group in the same molecule that can be used to prepare the polymerizable mixtures of the present invention include, for example, those represented by the following Formulas XXXXII or XXXXIII: 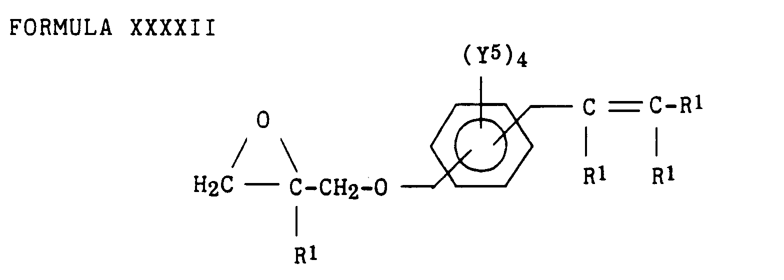 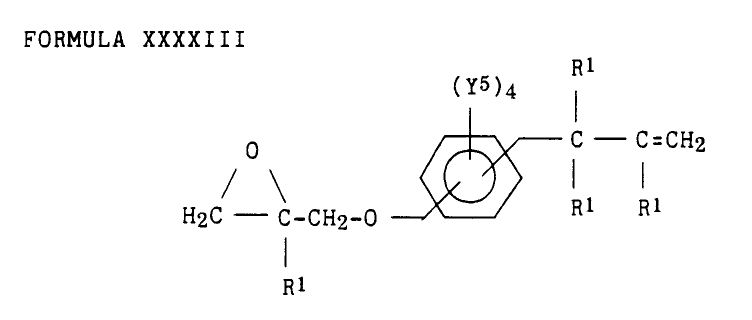 wherein each Y⁵ and R¹ are as hereinbefore defined.

Suitable compounds which contain a 1,2-epoxide group and a polymerizable ethylenically unsaturated group in the same molecule represented by Formulas XXXXII and XXXXIII include, for example, o-, m-, p-isopropenylphenyl glycidyl ether; o-, m-, p-vinylphenyl glycidyl ether; methyl-p-isopropenylphenyl glycidyl ethers; 3-chloro-4-isopropenylphenyl glycidyl ether; o-, m-, p-propenylphenyl glycidyl ether; o-, m-, p-allylphenyl glycidyl ether; and o-, m-, p-methallyphenyl glycidyl ether. Some of the alkenylphenol precursors to the alkenylphenyl glycidyl ethers represented by Formula XXXXII, notably the vinylphenols, have a tendency to dimerize or oligomerize thus leading to poly(alkenylphenyl)glycidyl ethers. It is most preferred that the alkenylphenyl glycidyl ether be substantially free of dimeric and/or oligomeric components, although it is operable to use an alkenylphenyl glycidyl ether containing substantial (up to 90 percent by weight) dimeric and/or oligomeric components. The compounds which contain a 1,2-epoxide group and a polymerizable ethylenically unsaturated group in the same molecule are prepared using the corresponding phenol containing a polymerizable ethylenically unsaturated group and the hereinbefore described chemistry used in the preparation of epoxy resins.

### COMPOUNDS CONTAINING BOTH A MALEIMIDE GROUP AND A CYANATE GROUP AND NO MESOGENIC OR RODLIKE STRUCTURES SUITABLE FOR USE IN THE CURABLE AND CURED COMPOSITIONS

Suitable compounds which contain both a maleimide group and a cyanate group in the same molecule and do not contain mesogenic or rodlike structures that can be used to prepare the polymerizable mixtures of the present invention include, for example, those represented by the following Formulas XXXXIV or XXXXV: 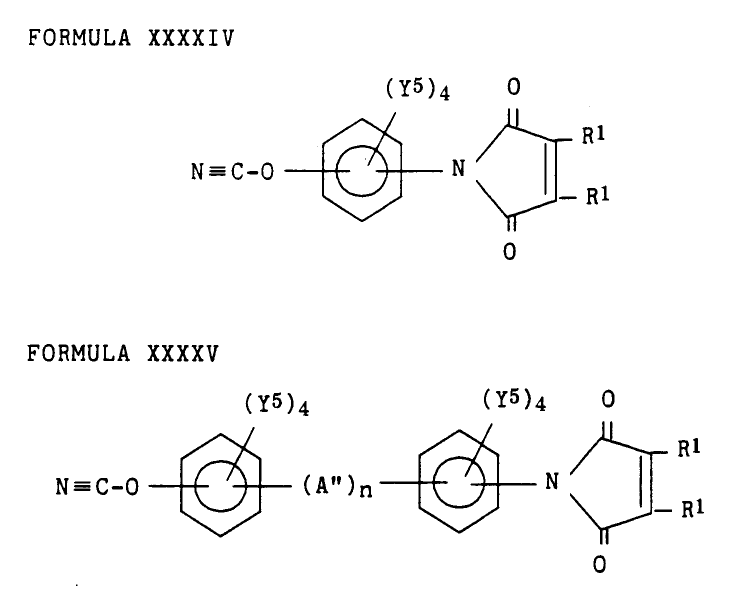 wherein each Y⁵, R¹, A'' and n are as hereinbefore defined.

Suitable compounds which contain a maleimide group and a cyanate group in the same molecule and do not contain mesogenic or rodlike structures represented by Formulas XXXXIV and XXXXV include, for example, 4-(1-(3-(2,5-dihydro-2,5-dioxo-1H-pyrrol-1-yl)phenyl)-1-methylethyl)phenyl cyanate; 4-(1-(4-(2,5-dihydro-2,5-dioxo-1H-pyrrol-1-yl)phenyl)-1-methylethyl)phenyl cyanate; 4-(1-(4-(2,5-dihydro-2,5-dioxo-1H-pyrrol-1-yl)phenyl)ethyl)phenyl cyanate; 4-(4-(2,5-dihydro-2,5-dioxo-1H-pyrrol-1-yl)phenoxy)phenyl cyanate; 4-((4-(2,5-dihydro-2,5-dioxo-1H-pyrrol-1-yl)phenyl)thio)phenyl cyanate; 4-(4-(2,5-dihydro-2,5-dioxo-1H-pyrrol-1-yl)benzoyl)phenyl cyanate; 4-((4-(2,5-dihydro-2,5-dioxo-1H-pyrrol-1-yl)phenyl)sulfonyl)phenyl cyanate; 4-(1-(4-(2,5-dihydro-3-methyl-2,5-dioxo-1H-pyrrol-1-yl)phenyl)-1-methylethyl)phenyl cyanate; 2,6-dibromo-4-(1-(3,5-dibromo-4-(2,5-dihydro-2,5-dioxo-1H-pyrrol-1-yl)phenyl)-1-methylethyl)phenylcyanate; 4-(2,5-dihydro-2,5-dioxo-1H-pyrrol-1-yl)phenyl cyanate; and 3-(2,5-dihydro-2,5-dioxo-1H-pyrrol-1-yl)phenyl cyanate. Preparation of compounds which contain a maleimide group and a cyanate group in the same molecule and do not contain mesogenic or rodlike structures is taught in U.S. Patent No. 4,683,276.

### COMPOUNDS CONTAINING ONE CYANATE OR CYANAMIDE GROUP PER MOLECULE AND ONE OR MORE MESOGENIC OR RODLIKE MOIETIES WHICH CAN BE EMPLOYED IN THE CURABLE AND CURED COMPOSITIONS

Suitable compounds which contain one or more mesogenic or rodlike structure(s) and an average of one cyanate or cyanamide group per molecule that can be used to prepare the polymerizable mixtures of the present invention include, for example, those represented by the following Formulas XXXXVI, XXXXVII, XXXXVIII or XXXXIX: 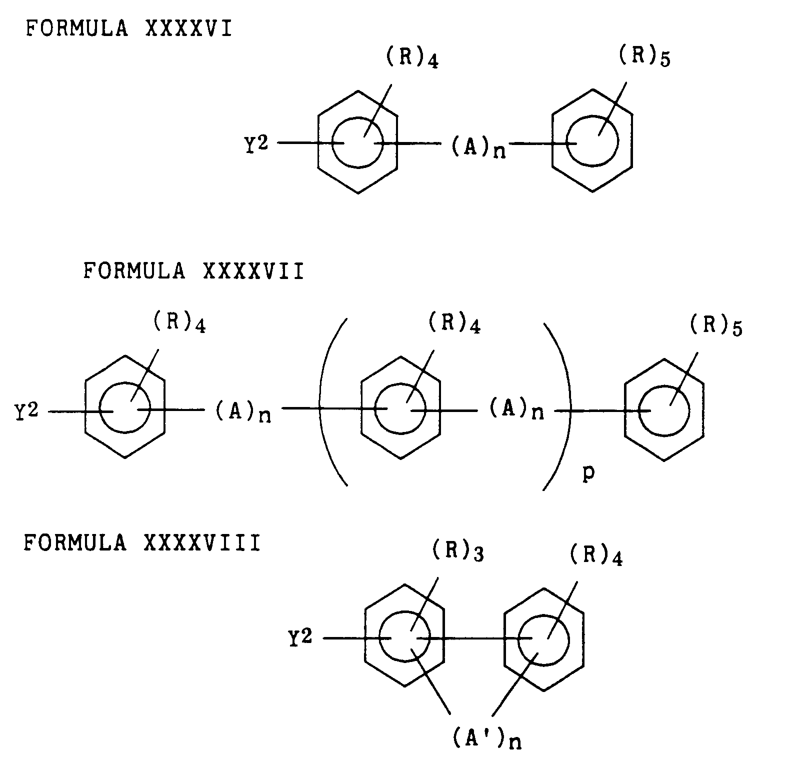 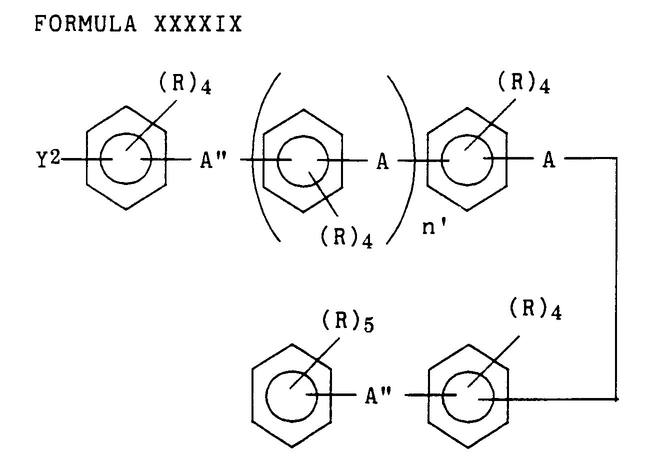 wherein at least 80 percent of the -A- linkages in Formulas XXXXVI, XXXXVII and XXXXIX and the direct bond in Formula XXXXVIII and the Y² groups are in the para position with respect to each other and each A, A', A'', R, Y², p, n and n' are as hereinbefore defined.

Suitable compounds which contain one or more mesogenic or rodlike structure(s) and an average of one cyanate or cyanamide group per molecule represented by formulas XXXXVI, XXXXVII, XXXXVIII and XXXXIX include, for example, the cyanates of 4-hydroxystilbene, 4-hydroxy-4'-methoxystilbene, 4-hydroxy-4'-chlorostilbene, 4-hydroxy-4'-nitrostilbene, 4-hydroxy-4'-cyanostilbene, 4-hydroxy-alpha-methylstilbene, 4-hydroxychalcone, 1-(4-hydroxyphenyl)-2-phenylacetylene, 1-(4-hydroxyphenyl)-2-phenylazomethine, 4-hydroxyphenylazobenzene, 4-hydroxyphenylazoxybenzene, 4-(4-hydroxyphenoxy)diphenyl, 4-hydroxydiphenyl, 4-hydroxy-alpha-cyanostilbene, 4-hydroxy-alpha-ethylstilbene, 4-hydroxybenzanilide, 4-hydroxy-4'-methoxybenzanilide, 4-hydroxy-3,3',5,5'-tetramethyl-alpha-methylstilbene, N-methyl-4-hydroxybenzamide, N-phenyl-4-hydroxybenzamide, 4-hydroxy-3,3',5,5'-tetrabromo-alpha-methylstilbene, 4-hydroxyphenylbenzoate, phenyl-4-hydroxybenzoate, the cyanamides of 4-aminostilbene, 4-amino-alpha-methylstilbene and 4-aminobenzanilide. The compounds which contain one or more mesogenic or rodlike structure(s) and an average of one cyanate or cyanamide group per molecule are prepared using the corresponding monophenol (monoamine) containing one or more mesogenic or rodlike structure(s) and the hereinbefore described chemistry used in the preparation of polycyanates (polycyanamides).

### METHOD FOR FORMING THE MIXTURES OF THE PRESENT INVENTION

The mixtures of the present invention can be prepared by directly combining one or more of the desired component(s) with one or more cyanate functional maleimides containing one or more mesogenic or rodlike structures or by addition of one or more of the desired components to one or more of the cyanate functional maleimides containing one or more mesogenic or rodlike structures in increments or stages. When a single component is to be added to one or more of the cyanate functional maleimides containing one or more mesogenic or rodlike structures, said component may be prepolymerized (B-staged) or fully homopolymerized, prior to the addition. When two or more components are to be added to one or more of the cyanate functional maleimides containing one or more mesogenic or rodlike structures, said components may be partially or totally copolymerized or reacted together, prior to the addition. Additionally, when two or more components are to be added to one or more of the cyanate functional maleimides containing one or more mesogenic or rodlike structures, one component may be prepolymerized or fully homopolymerized in the presence of the other components, prior to the addition. It is understood that one or more catalysts or accelerators may be included where desired to facilitate the aforementioned copolymerization, prepolymerization, homopolymerization or reaction of one or more specific components.

The mixtures can comprise any amount of the compound or compounds containing at least one cyanate group, at least one maleimide group and at least one mesogenic or rodlike moiety and the other component or components; however, the mixtures suitably contain from 1 to 99, more suitably from 99 to 40, most suitably from 95 to 70 percent by weight of the compound or compounds containing at least one cyanate group, at least one maleimide group and at least one mesogenic or rodlike moiety and suitably from 99 to 1, more suitably from 60 to 1, most suitably from 30 to 5 percent by weight of the other component or components.

### POLYMERIZATION (CURING) OF THE POLYMERIZABLE MIXTURES

The mixtures of the present invention may be polymerized by heating from 50°C to 400°C, preferably by heating from 100°C to 250°C, optionally in the presence of one or more suitable catalysts. For the mixtures containing one or more of the cyanate functional maleimides containing one or more mesogenic or rodlike structures, whenever one or more polymaleimides, compounds containing one or more polymerizable ethylenically unsaturated group(s), compounds which simultaneously contain both a cyanate group or cyanamide group and a polymerizable ethylenically unsaturated group, compounds which simultaneously contain both a 1,2-epoxide group and a polymerizable ethylenically unsaturated group or compounds which simultaneously contain both a maleimide group and a cyanate group and no mesogenic or rodlike structures are present, it is often desirable to utilize one or more free radical forming catalysts for the purpose of polymerizing all or a part of said unsaturated groups. Said free radical forming catalysts include the organic peroxides and hydroperoxides as well as the azo and diazo compounds. Preferred free radical forming catalysts include benzoylperoxide, t-butylhydroperoxide, t-butylperoxybenzoate, azobisisobutyronitrile, dicumylperoxide, di-tert-butylperoxide and cumene hydroperoxide. The quantity of catalyst used, if any, depends on, for example, the structure of the particular catalyst, the structure of the components used in the polymerizable mixture, the cure structure desired, the cure time and the cure temperature. Generally, catalyst concentrations of from 0.001 to 2 percent by weight are preferred. B-staging or prepolymerization of the mixtures of the present invention can be accomplished by using lower temperatures and/or shorter curing times. Curing of the thus formed B-staged (prepolymerized) mixture can then be accomplished at a later time or immediately following B-staging (prepolymerization) by increasing the temperature and/or curing time.

The polymerized (cured) mixtures possess a variety of curing structures which depend, in part, for example, upon the amounts and types of individual components used to prepare said mixture, the sequence of component addition and procedure used to prepare said mixture, the amounts and types of catalysts, if any, employed, the reaction times and temperatures.

Mixtures of (A), one or more cyanate functional maleimides containing one or more mesogenic or rodlike structures and no other moieties reactive with the cyanate or maleimide group, and (B-1), one or more polycyanates which do not contain mesogenic or rodlike structures, and/or (B-3), one or more polymaleimides and/or prepolymers of any of the aforementioned types of compounds, polymerize to produce the aforementioned curing structures delineated for the (A), cyanate functional maleimides containing one or more mesogenic or rodlike structures. It should be noted; however, that the relative mole ratio of cyanate groups to maleimide groups can influence the amounts of the various cure structures in the cured product. For example, a large excess of cyanate groups, provided by using an (B-1) aromatic polycyanate in the copolymerizable compositions increases the amount of the triazine cure structure in the cured product.

Mixtures of (A), one or more cyanate functional maleimides containing one or more mesogenic or rodlike structures and no other moieties reactive with the cyanate or maleimide group, and (B-2), one or more epoxy resins, polymerize to produce a complex structure, including that derived from the copolymerization reaction of the cyanate group and the glycidyl ether group 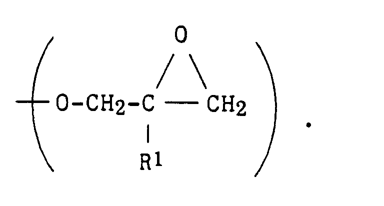 Additionally, curing structures derived from homopolymerization of the maleimide groups, homopolymerization of the cyanate groups (triazine), as well as copolymerization of the cyanate and maleimide groups can be present.

Mixtures of (A), one or more cyanate functional maleimides containing one or more mesogenic or rodlike structures and no other moieties reactive with the cyanate or maleimide group, and (B-4), one or more polyamines, polymerize to produce a complex structure including the iminocarbamic acid ester moiety derived from the copolymerization reaction of the cyanate group and the amine group, as well as the addition structure derived from the copolymerization reaction of the amine group and the maleimide unsaturation. Additionally, curing structures derived from homopolymerization of the maleimide groups, homopolymerization of the cyanate groups (triazine), as well as copolymerization of the cyanate and maleimide groups can be present.

Mixtures of (A), one or more cyanate functional maleimides containing one or more mesogenic or rodlike structures and no other moieties reactive with the cyanate or maleimide group, and (B-5), one or more polyphenols, polymerize to produce a complex structure including iminocarbonic acid ester moiety derived from the copolymerization reaction of the cyanate group and the phenolic hydroxyl group, as well as the addition structure derived from the copolymerization reaction of the phenolic hydroxyl group and the maleimide unsaturation. Additionally, curing structures derived from homopolymerization of the maleimide groups, homopolymerization of the cyanate groups (triazine), as well as copolymerization of the cyanate and maleimide groups can be present.

Mixtures of (A), one or more cyanate functional maleimides containing one or more mesogenic or rodlike structures and no other moieties reactive with the cyanate or maleimide group, and (B-6), one or more polymerizable ethylenically unsaturated compounds, polymerize to produce a complex curing structure including structure derived from the copolymerization reaction of the maleimide group and the polymerizable ethylenically unsaturated group(s), as well as structure derived from the copolymerization reaction of the cyanate group and the polymerizable ethylenically unsaturated group(s). Additionally, curing structures derived from homopolymerization of the polymerizable ethylenically unsaturated groups, from homopolymerization of the maleimide groups, from homopolymerization of the cyanate groups (triazine), as well as copolymerization of the cyanate and maleimide groups can be present.

Mixtures of (A), one or more cyanate functional maleimides containing one or more mesogenic or rodlike structures and no other moieties reactive with the cyanate or maleimide group, and (B-7), one or more compounds which simultaneously contain both a cyanate group and a polymerizable ethylenically unsaturated group, or (B-8), one or more compounds which simultaneously contain both a 1,2-epoxide group and a polymerizable ethylenically unsaturated group, or (B-9), one or more compounds which simultaneously contain both a maleimide group and a cyanate group and does not contain mesogenic or rodlike structures, can polymerize to produce a complex variety of structures, including those previously mentioned for the various respective functional groups.

Mixtures of (A), one or more cyanate functional maleimides containing one or more mesogenic or rodlike structures, and (B-10), one or more compounds which contain one or more mesogenic or rodlike structures and only one cyanate or cyanamide group per molecule, polymerize to produce the aforementioned curing structures delineated for the (A), cyanate functional maleimides containing one or more mesogenic or rodlike structures; providing that no other moieties reactive with cyanate or maleimide groups are present in (A) or (B-10). Increasing the amount of the aforementioned cyanate compound containing an average of one cyanate group per molecule with respect to the amount of cyanate functional maleimide can be used as a convenient method for lowering the crosslink density of the thermoset product thereof.

### ORIENTATION OF THE POLYMERIZED PRODUCT CONTAINING MESOGENIC OR RODLIKE STRUCTURES

During processing and/or curing of the cyanate functional maleimides containing one or more mesogenic or rodlike structures or the mixtures containing said cyanate functional maleimides, electric or magnetic fields or shear stresses can be applied for the purpose of orienting the mesogenic or rodlike moieties contained or developed therein. As specific examples of these methods, Finkelmann, et. al., Macromol. Chem., 180, 803-806 (March, 1979), induced orientation in an electric field, of thermotropic methacrylate copolymers containing mesogenic side chain groups decoupled from the main chain via flexible spacers. Orientation in a magnetic field of mesogenic side chain groups decoupled from the main chain via flexible spacers has been demonstrated by Roth and Kruecke, Macromol. Chem., 187, 2655-2662 (November, 1986). Magnetic field induced orientation of mesogenic main chain containing polymers has been demonstrated by Moore, et. al., ACS Polymeric Material Sciences and Engineering, 52, 84-86 (April-May, 1985). Magnetic and electric field induced orientation of low molecular weight mesogenic compounds is discussed by W. Krigbaum in Polymer Liquid Crystals, pages 275-309 (1982), published by Academic Press, Inc. The use of shear to induce orientation is also discussed therein. When the curing is to be performed in an electric or magnetic field, it is frequently of value to conduct simple preliminary experiments that allow for balancing of cure kinetics versus induction of orientation under the particular experimental conditions being employed (i.e. catalyst(s) level being used, temperature used, inherent dielectric or diamagnetic susceptibility of the specific mesogenic or rodlike structure(s) used). This is done recognizing the relatively greater ease of inducing orientation in low molecular weight materials versus polymeric materials containing mesogenic moieties.

In addition to orientation by electric or magnetic fields, the cyanate functional maleimides containing one or more mesogenic or rodlike structures or mixtures containing said cyanate functional maleimides can be oriented by shear forces which are induced by flow through dies, orifices and mold gates. A general discussion of orientation of thermotropic liquid crystalline polymers by this method is given by S.K. Garg and S. Kenig in High Modulus Polymers, pages 71-103 (1988) published by Marcel Dekker, Inc. For the mesomorphic cyanate functional maleimides or mixtures containing said cyanate functional maleimides, this shear orientation can conveniently be produced by or during processing methods such as injection molding, extrusion, pultrusion, filament winding, filming and prepreging.

### OTHER COMPONENTS WHICH CAN BE EMPLOYED

The cyanate functional maleimides containing one or more mesogenic or rodlike structures or mixtures containing said cyanate functional maleimides can be blended with other materials such as solvents or diluents, fillers including those comprising a liquid crystalline polymer, pigments, dyes, flow modifiers, thickeners, reinforcing agents, mold release agents, wetting agents, stabilizers, fire retardant agents, surfactants, low profile additives, shrinkage control agents, other resinous products and combinations thereof.

These additives are added in functionally effective amounts, for example, the pigments and/or dyes are added in quantities which will provide the composition with the desired color; however, they are suitably employed in amounts of from zero to 20, more suitably from 0.5 to 5, most suitably from 0.5 to 3 percent by weight based on the total weight of the composition.

Solvents or diluents which can be employed herein include, for example, hydrocarbons, ketones, aliphatic ethers, cyclic ethers, esters, chlorinated hydrocarbons and combinations thereof. Particularly suitable solvents or diluents include, for example, toluene, xylenes, methylethyl ketone, methylisobutyl ketone, methylamyl ketone, chloroform, acetone, perchloroethylene, methylene chloride, tetrahydrofuran, 1,4-dioxane, ethyl acetate, butyl acetate and combinations thereof.

The modifiers such as thickeners, flow modifiers, shrinkage control agents, low profile additives and the like can be suitably employed in amounts from 0.05 to 15, more suitably from 0.1 to 10, most suitably from 0.1 to 5 percent by weight based on the total weight of the composition.

Reinforcing materials which can be employed herein include, for example, natural and synthetic fibers in the form of woven fabric, mats, monofilament, multifilament, unidirectional fibers, rovings, random fibers or filaments, inorganic fillers or whiskers and hollow spheres. Suitable reinforcing materials include, for example, glass, ceramics, nylon, rayon, cotton, aramid, graphite, polyalkylene terephthlates, polyethylene, polypropylene, polyesters, carbon, boron, asbestos, combinations and hybrids thereof.

Suitable fillers which can be employed herein include, for example, inorganic oxides, ceramic microspheres, plastic microspheres, glass microspheres, inorganic whiskers, calcium carbonate, graphite powder, sand, metal powders and combinations thereof. The fillers can be employed in amounts from 0.1 to 95, more suitably from 5 to 80, most suitably from 10 to 50 percent by weight of the total composition.

### USES FOR THE COMPOSITIONS

The compositions of the present invention can be employed, for example, in the preparation of laminates, prepregs, composites, coatings, castings, pultruded products, filament wound products, films, molding and potting formulations and injection molded products.

The following examples are illustrative of the invention but are not to be construed as to limiting the scope thereof in any manner.

### Example 1

### A. Synthesis of 4-Hydroxy-4'-nitrobenzanilide

p-Hydroxybenzoic acid (59.05 grams, 0.4275 mole), sodium ethoxide catalyst (0.133 gram, 0.225 percent weight of the p-hydroxybenzoic acid used) and N,N-dimethyl-acetamide solvent (404 grams) were added to a reactor equipped with a reflux condenser and stirred under a nitrogen atmosphere at 80°C. p-Nitrophenylisocyanate (73.85 grams, 0.450 mole) was initially added in an aliquot of 25.00 grams, followed by 25.00 and 23.85 gram aliquots eleven then nine minutes later, respectively, and so as to maintain a 80 to 82°C reaction temperature. After the last aliquot of p-nitrophenylisocyanate was added, heating of the reactor commenced and a 160°C reaction temperature was achieved 24 minutes later. After three hours at the 160°C reaction temperature, the reactor was cooled to 30°C then the contents poured into 3.8 liters (one gallon) of deionized water. A precipitated yellow powder was recovered via filtration of the aqueous slurry then dissolved into 1900 milliliters of boiling methanol and refluxed therein (65°C). After cooling the methanol solution to 5°C and maintaining therein for twelve hours, a first crop of pale yellow colored crystalline product was filtered off and dried at 110°C under vacuum to a constant weight of 92.5 grams (79.6 percent isolated yield). No attempt was made to recover a second crop of crystalline product from the mother liquor. Fourier transform infrared spectrophotometric analysis of a nujol mull of a portion of the product on a sodium chloride plate revealed the presence of the expected secondary amide N-H stretching (solid state) at 3385 cm⁻¹ (sharp), the secondary amide carbonyl stretching (solid state) at 1655 cm⁻¹ (sharp), the hydroxyl group O-H stretching centered at 3232 cm⁻¹ (broad) and the conjugated nitro group absorbances at 1537 and 1339 cm⁻¹ (sharp). Proton magnetic resonance spectroscopy (250 MHz) further confirmed the product structure as 4-hydroxy-4'-nitrobenzanilide.

### B. Synthesis of 4-Hydroxy-4'-aminobenzanilide

A portion (44.1 grams, 0.1708 mole) of 4-hydroxy-4'-nitrobenzanilide from A. above and ethanol (300 milliliters) were added to a 400 milliliter heavy walled glass bottle then sparged with nitrogen. After removal of air by nitrogen sparging, Raney nickel catalyst (5.5 grams of a 75 percent weight slurry in water at pH 10) was added to the slurry in the glass bottle which was then stoppered and multiply purged with hydrogen to replace the nitrogen atmosphere. The bottle was then placed on a shaking type agitator, and pressurized to 331 kPa (48 psig) hydrogen. Shaking of the pressurized slurry at room temperature (25°C) commences until 23.3 hours later, the hydrogen pressure reading indicated that 324 kPa (47 psi) of hydrogen has been consumed. By the completion of the hydrogenation, the light yellow colored reactant slurry became a light pink tan colored product slurry. The product slurry was recovered, diluted into dimethylsulfoxide (300 milliliters) to provide a solution of product containing precipitated Raney nickel, then filtered through a medium porosity fritted glass funnel. The recovered dimethylsulfoxide product solution was rotary evaporated at 130°C under vacuum to provide a powder product. The powder product was further dried at 120°C under vacuum to a constant weight of 38.94 grams (99.88 percent isolated yield). Fourier transform infrared spectrophotometric analysis of a nujol mull of a portion of the product on a sodium chloride plate revealed the presence of absorbances at 3376 (shoulder), 3351 (shoulder), 3316 (sharp) and 3282 (shoulder) cm⁻¹ due to secondary amide group N-H stretching (solid state), primary amine N-H group stretching and hydroxyl group O-H stretching; the secondary amide carbonyl stretching (solid state) at 1645 cm⁻¹ (sharp); and complete disappearance of the conjugated nitro group absorbances at 1537 and 1339 cm⁻¹ (sharp). Proton magnetic resonance spectroscopy (250 MHz) further confirmed the product structure as 4-hydroxy-4'-aminobenzanilide.

### C. Synthesis of Monomaleimide of 4-Hydroxy-4'-aminobenzanilide

A portion (38.50 grams, 0.1687 mole) of 4-hydroxy-4'-aminobenzanilide from B. above and dry acetic acid (800 milliliters) were added to a reactor and maintained under a nitrogen atmosphere with stirring. The stirred slurry was maintained at 25°C while maleic anhydride (16.54 grams, 0.1687 mole) dissolved in dry acetic acid (100 milliliters) was added to the reactor. One minute after the addition, a maximum exotherm of 27°C was achieved, then heating was started. Fifty nine minutes later, the slurry reaches a reaction temperature of 110°C and was maintained therein for fourteen hours. The recovered product slurry was rotary evaporated at 80°C under vacuum to provide a powder product. The powder product was then added to refluxing acetone (500 milliliters) and stirred therein as a slurry for five minutes. After cooling the acetone slurry to room temperature (25°C), the first crop of light yellow green colored product was filtered off and dried at 80°C under vacuum to a constant weight of 43.87 grams (84.38 percent isolated yield). No attempt was made to recover a second crop of product from the mother liquor. Fourier transform infrared spectrophotometric analysis of a nujol mull of a portion of the product on a sodium chloride plate revealed the presence of absorbances at 3349 (sharp), 3289 (sharp) and 3087 (broad) cm⁻¹ due to secondary amide group N-H stretching (solid state), hydroxyl group O-H stretching; the secondary amide carbonyl stretching (solid state) at 1649 cm⁻¹ (sharp); and the maleimide carbonyl group stretching at 1702 cm⁻¹ (sharp). Proton magnetic resonance spectroscopy (250 MHz) further confirmed the product structure as the maleimide of 4-hydroxy-4'-aminobenzanilide.

### D. Synthesis of the Cyanate of 4-Hydroxy-4'-aminobenzanilide Maleimide

A portion (10.30 grams, 0.0334 mole) of the maleimide of 4-hydroxy-4'-aminobenzanilide from C. above, cyanogen bromide (3.72 grams, 0.0351 mole) and acetone (400 milliliters) were added to a reactor and maintained under a nitrogen atmosphere with stirring. The stirred slurry was cooled to -3°C, then triethylamine (3.40 grams, 0.0336 mole) was added to the reactor over a 10 minute period and so as to maintain the reaction temperature at -3 to -1°C. After completion of the triethylamine addition, the reactor was maintained at -3 to -1°C for an additional 35 minutes followed by addition of the reactor contents to deionized water (2000 milliliters). After five minutes, the water and product mixture was multiply extracted with three 500 milliliter volumes of methylene chloride. The combined methylene chloride extract was washed with three 250 milliliter portions of deionized water, then dried over anhydrous sodium sulfate. The dry methylene chloride extract was filtered and solvent removed by rotary evaporation under a vacuum at 60°C to provide a powder product. The remaining precipitated product not recovered by the methylene chloride extractions was directly filtered off then washed with two 250 milliliter portions of deionized water. After drying under vacuum at 65°C, a constant weight of 3.00 and 6.61 grams of light yellow colored powder was recovered from the methylene chloride extraction and the precipitation/filtration, respectively (86.31 percent isolated yield for the combined product). Fourier transform infrared spectrophotometric analysis of a nujol mull of a portion of the product on a sodium chloride plate revealed the presence of the expected secondary amide group N-H stretching (solid state) at 3348 cm⁻¹ (sharp), the secondary amide group carbonyl stretching (solid state) at 1659 cm⁻¹ (sharp), the maleimide group carbonyl stretching at 1706 cm⁻¹ (sharp) and the cyanate group absorbance at 2264 and 2242 cm⁻¹ (sharp) (the analysis was essentially identical for both product fractions). Proton magnetic resonance spectroscopy (250 MHz) further confirmed the product structure as the cyanate of 4-hydroxy-4'-aminobenzanilide maleimide.

### E. Characterization of the Cyanate of 4-Hydroxy-4'-aminobenzanilide Maleimide for Liquid Crystallinity

A portion (9.00 milligrams) of the cyanate of 4-hydroxy-4'-aminobenzanilide maleimide from D. above was analyzed by differential scanning calorimetry using a heating rate of 10°C per minute under a stream of nitrogen flowing at 35 cubic centimeters per minute and the indicated temperature ranges. The following results were obtained:

| CYCLE DESIGNATION | OBSERVED TRANSITION TEMPERATURES (°C) midpoint/range | ENTHALPY (J/G) | COMMENTS |
|---|---|---|---|
| First heating | 197/140-214 | 141.8 | Single peak exotherm |
| (30 to 375°C) | 269/219-366 | 169.1 | Single peak exotherm |

A repeat of the above first heating using a fresh sample (8.20 milligrams) and a range of 30°C to 300°C revealed a 273°C midpoint for the second exothermic peak without a return to baseline by the 300°C end of the analysis. A second heating of this second sample from 30°C to 300°C revealed only a slight and gradual exothermic shift of the baseline starting at 258°C.

The cured products were recovered from the differential scanning calorimetry as golden brown colored solids and were used to prepare nujol mulls. Fourier transform infrared spectrophotometric analysis of a film of the nujol mulls on a sodium chloride plate revealed that complete disappearance of the cyanate absorbance had occurred with retention of the secondary amide group carbonyl stretching (solid state) at 1653 cm⁻¹ (sharp) and the maleimide group carbonyl stretching at 1706 cm⁻¹ (sharp). The ratio of the amide group N-H absorbance to the amide group carbonyl group absorbance for both the cured product and the uncured product (from D. above) was found to be identical, hence substantial retention of the secondary amide group N-H stretching (3348 cm⁻¹) was implied. The appearance of a new absorbance at 1565 cm⁻¹ was observed in the cured product. The cured products from both of the differential scanning analysis gave essentially identical infrared spectrophotometric analysis.

Analysis of the maleimide cyanate via cross polarized light microscopy was completed using a microscope equipped with a programmable hot stage using a heating rate of 10°C per minute and 35X magnification. The following results were obtained:

| CYCLE DESIGNATION | OBSERVED TRANSITION TEMPERATURES (°C) | COMMENTS |
|---|---|---|
| First heating | 25 | Immobile crystals. |
| | 196 | First fluidity noted as birefringent nematic droplets. |
| | 227 | Viscosity increasing, forms birefringent streaks in shear direction as sheared. |
| | 239 | Cures to a birefringent solid. |

### COMPARATIVE EXPERIMENT A

### 1. Synthesis of the Cyanate of 3-Aminophenol Maleimide

A portion (11.45 grams, 0.0605 mole) of the maleimide of 3-aminophenol prepared using the method of U.S. Patent No. 4,683,276, Example 1-A, cyanogen bromide (6.73 grams, 0.0636 mole) and acetone (300 milliliters) are added to a reactor and maintained under a nitrogen atmosphere with stirring. The stirred solution was cooled to -5°C, then triethylamine (6.16 grams, 0.0608 mole) was added to the reactor over a 10 minute period and so as to maintain the reaction temperature at -5°C to -1°C. After completion of the triethylamine addition, the reactor was maintained at -3°C to -1°C for an additional 35 minutes followed by addition of the reactor contents to deionized water (1500 milliliters). After five minutes, the water and product mixture was multiply extracted with three 200 milliliter volumes of methylene chloride. The combined methylene chloride extract was washed with three 250 milliliter portions of deionized water, then dried over anhydrous sodium sulfate. The dry methylene chloride extract was filtered and solvent removed by rotary evaporation under a vacuum at 60°C to provide a powder product. After drying under vacuum at 65°C, a constant weight of 11.08 grams light tan colored powder was recovered (85.47 percent isolated yield). Fourier transform infrared spectrophotometric analysis of a neat film of a portion of the product on a sodium chloride plate revealed the presence of the expected maleimide group carbonyl stretching at 1716 cm⁻¹ (sharp) and the cyanate group absorbance 2265 and 2234 cm⁻¹ (sharp). Proton magnetic resonance spectroscopy (250 MHz) further confirmed the product structure as the cyanate of 3-aminophenol maleimide.

### 2. Characterization of the Cyanate of 3-Aminophenol Maleimide for Liquid Crystallinity

Results of the analysis of the cyanate of 3-aminophenol maleimide by differential scanning calorimetry are reported in U.S. Patent No. 4,680,378, Comparative Experiment A. Analysis of the maleimide cyanate using cross polarized light microscopy as per the method of Example 1-E above demonstrated that the product became fluid upon heating without any birefringence. Shearing of the fluid produced no birefringence, and the product cured to a non-birefringent solid.

### Example 2

### A. Preparation and Curing of a Blend of the Cyanate of 4-Hydroxy-4'-aminobenzanilide Maleimide and Bisphenol A Dicyanate

A portion (2.35 grams, 25 percent weight) of the cyanate of 4-hydroxy-4'-aminobenzanilide maleimide from Example 1-D and bisphenol A dicyanate (7.05 grams, 75 percent weight) were ground together to form a homogeneous powder blend. A portion (5.0 grams) of the resultant blend was heated to 125°C to provide a homogeneous paste which was then catalyzed by vigorously mixing in cobalt naphthenate (6.0 percent active) (0.005 grams, 0.10 percent weight) dissolved in methylene chloride (0.30 milliliter). The catalyzed blend was then placed in an oven which was preheated to 140°C and held for ten minutes at this temperature. The molten blend was removed, degassed in a vacuum bell jar, then poured into the reservoir of an injection molder preheated to 140°C. Blend remaining after filling of the reservoir was poured into an aluminum dish and placed back into the 140°C oven. After 10 minutes in the reservoir, the blend was injected through a 0.0825 inch (2.1 millimeters) square orifice into a mold preheated to 140°C and having the following dimensions: 3.0 by 0.5 by 0.125 inches (76.2 by 12.7 by 3.2 millimeters). The filled mold was immediately transferred to the 140°C oven which also contains the blend in the aluminum dish. Heating of both the mold and the aluminum dish to 177°C commences and this temperature was then maintained for 2 hours. The oven temperature was then increased to 200°C and maintained therein for 2 hours followed by increasing to 240°C. After 2 hours at the 240°C temperature, the oven was slowly cooled to room temperature (25°C) then the casting was recovered from the mold and the film was recovered from the aluminum dish.

### B. Dynamic Mechanical Analysis and Thermal Mechanical Analysis of the Cured Blend

Dynamic mechanical analysis of the molded cured blend was completed using a heating rate of 5°C per minute and a range of 30°C to 300°C. The tensile storage modulus measured at 40°C, 80°C, 120°C, 160°C and 200°C is reported in Table I. Thermal mechanical analysis of the film of the cured blend was completed with glass transition temperature and the mean linear thermal coefficient of expansion over the range of 30°C to Tg evaluated. In this analysis, a constant probe force of 0.1 Newtons and a heating rate of 10°C per minute was used over a range of 25°C to 330°C. The results are reported in Table I.

### COMPARATIVE EXPERIMENT B

### 1. Preparation and Curing of a Blend of the Cyanate of 3-Aminophenol Maleimide and Bisphenol A Dicyanate

A portion (9.40 grams, 25 percent by weight) of the cyanate of 3-aminophenol maleimide from Comparative Experiment A-1 and bisphenol A dicyanate (28.20 grams, 75 percent by weight) were ground together to form a homogeneous powder blend. A portion (5.0 grams) of the resultant blend was heated to 125°C to provide a homogeneous paste which was then catalyzed by vigorously mixing in cobalt naphthenate (6.0 percent active) (0.005 grams, 0.10 percent by weight) dissolved in methylene chloride (0.30 milliliter). The catalyzed blend was then used to prepare a molded test piece and a film using the method of Example 2-A.

### 2. Dynamic Mechanical Analysis and Thermal Mechanical Analysis of the Cured Blend

Dynamic mechanical analysis of the molded cured blend was completed using the method of Example 2-B. Thermal mechanical analysis of the film of the cured blend was completed with glass transition temperature and the mean linear thermal coefficient of expansion over the range of 30°C to Tg evaluated using the method of Example 2. The results are reported in Table I.

**TABLE I**

| | DESIGNATION OF SAMPLE | |
|---|---|---|
| | EXAMPLE 2 | COMPARATIVE. EXPERIMENT. B* |
| Dynamic Mechanical Analysis: | | |
| Tensile Storage Modulus (GPa) | | |
| 40°C | 1.430 | 0.877 |
| 80°C | 1.284 | 0.826 |
| 120°C | 1.220 | 0.770 |
| 160°C | 1.146 | 0.726 |
| 200°C | 1.022 | 0.634 |

| Thermal Mechanical Analysis: | | |
|---|---|---|
| Tg (°C) | 228.5 | 202.8 |
| Mean linear Coefficient of Thermal Expansion (ppm/°K) | 48 | 63 |

| | | |
|---|---|---|
| *Not an example of the present invention. | | |

## Claims

1. A compound containing at least one cyanate group, at least one maleimide group and at least one mesogenic or rodlike moiety represented by the following Formulas I, II, III or IV: 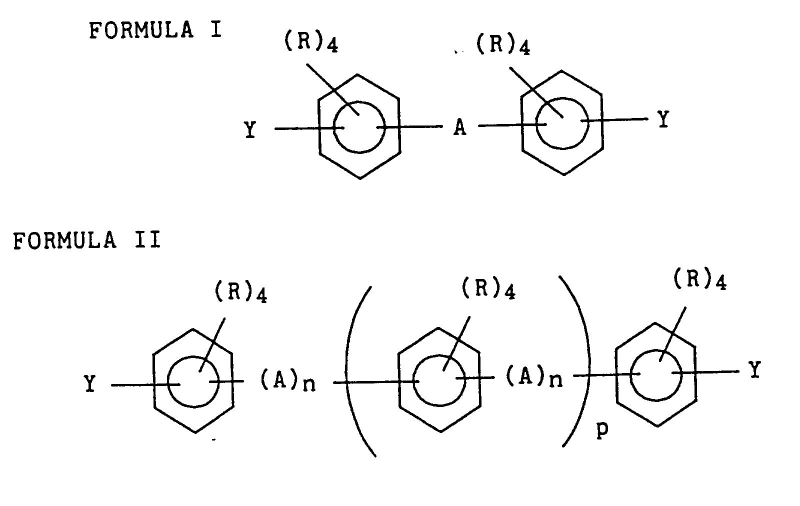 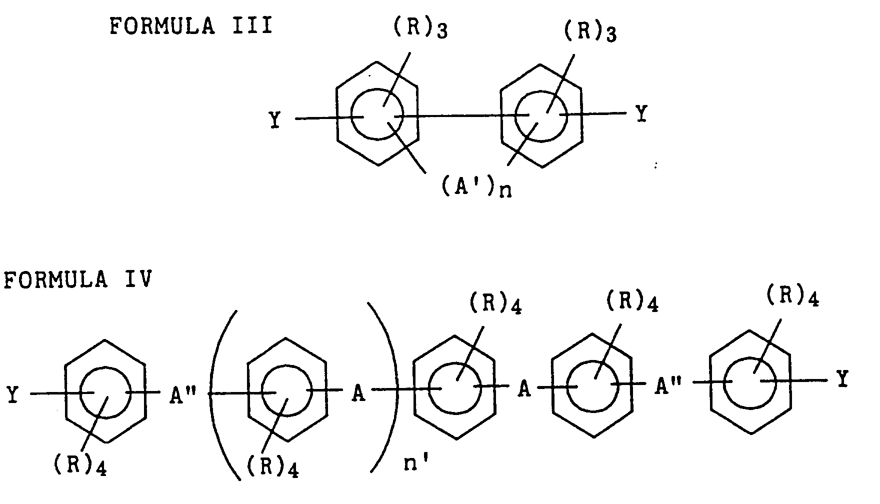 wherein at least about 80 percent of the -A- linkages in Formulas I, II and IV and the direct bond in Formula III and the Y groups are in the para position with respect to each other; one Y group is a cyanate, -O-C≡N, group and the other Y group is a maleimide group represented by the formula 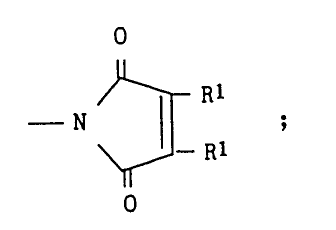 each A is independently -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 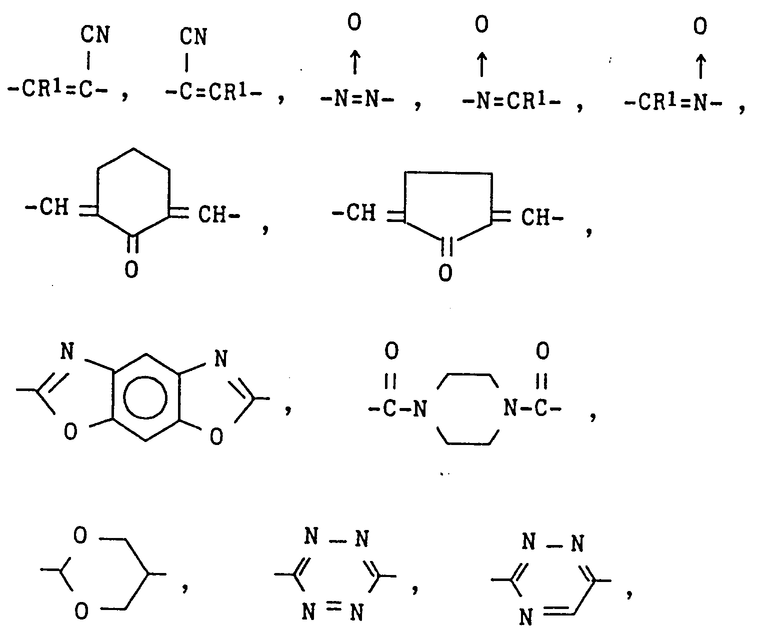 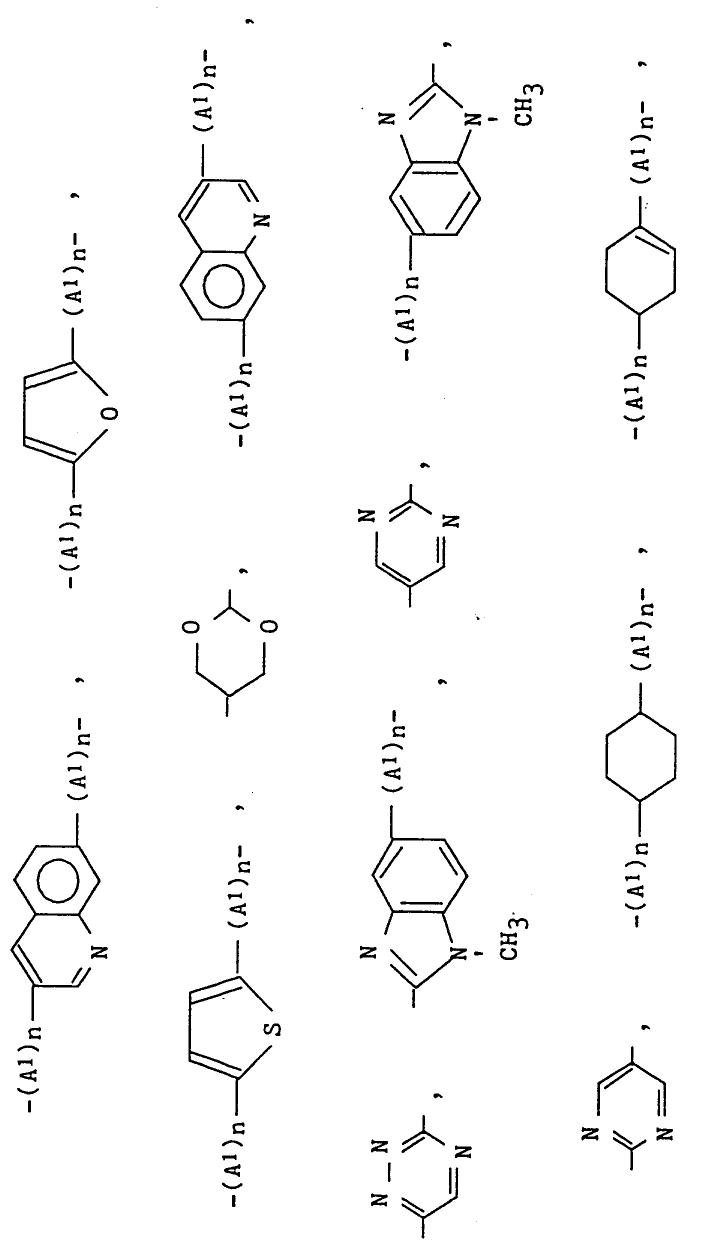 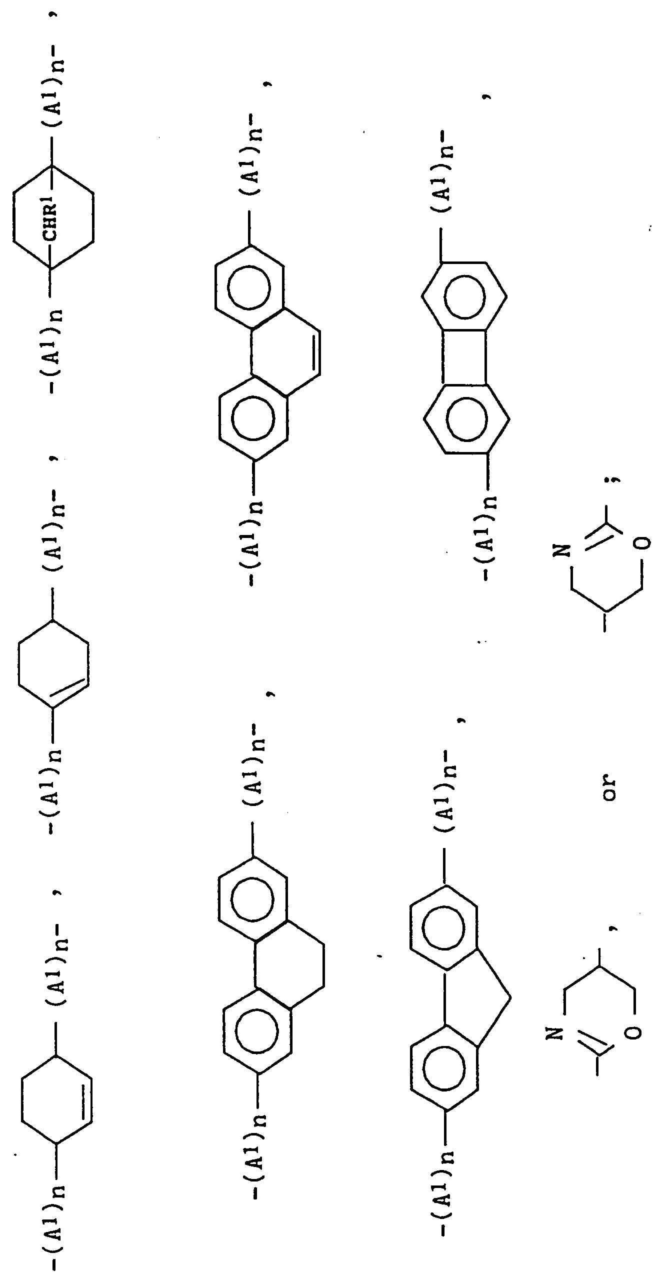 each A' is independently a divalent hydrocarbyl group having from 1 to 10 carbon atoms; each A'' is independently an alkylene group having from 1 to 10 carbon atoms, a direct bond, -O-, -CO-, -S-, -S-S-, -SO-, -SO₂- or -O-CO-O-; each A¹ is independently a -CO-, -O-CO-, -CO-O-, -CO-NR¹-, or -NR¹-CO- group; each R is independently hydrogen or a hydrocarbyl or hydrocarbyloxy group having from 1 to 10 carbon atoms, a halogen atom, a nitro group, a nitrile group, a phenyl group or a -CO-R¹ group; each R¹ is independently hydrogen or a hydrocarbyl group having 1 to 3 carbon atoms; n has a value of zero or one; n' has an average value from zero to 6; and p has an average value from 1 to 30; with the proviso that any of the aromatic rings can, if desired, contain a nitrogen, oxygen or sulfur heteroatom.

2. A compound of Claim 1 wherein each A' independently has from 1 to 4 carbon atoms; when A'' is an alkylene group, it has from 1 to 4 carbon atoms; each R is independently hydrogen or a hydrocarbyl group having from 1 to 4 carbon atoms, chlorine or bromine; n' has an average value from zero to 3; and p has an average value from 1 to 3.

3. A compound of Claim 1 selected from the group consisting of 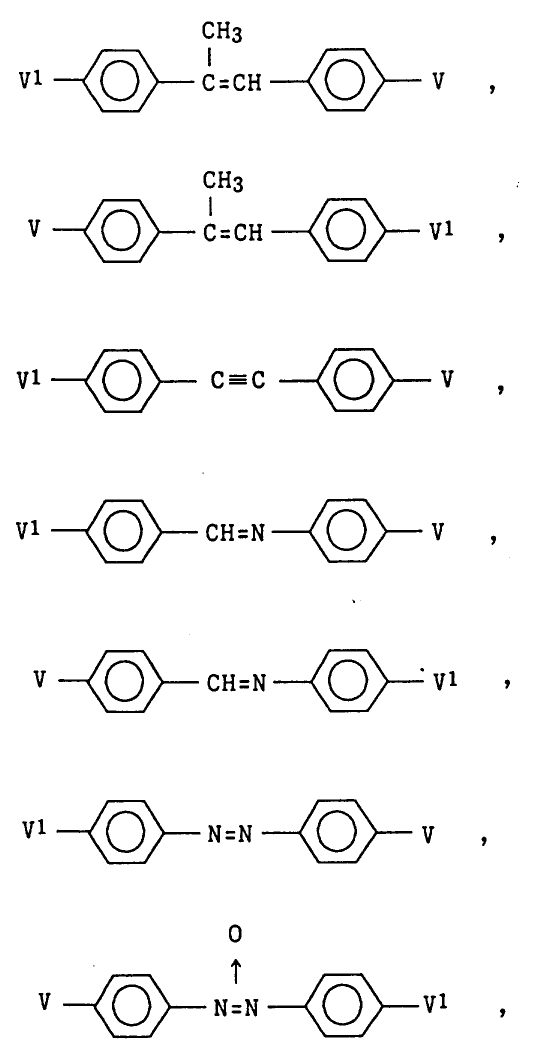 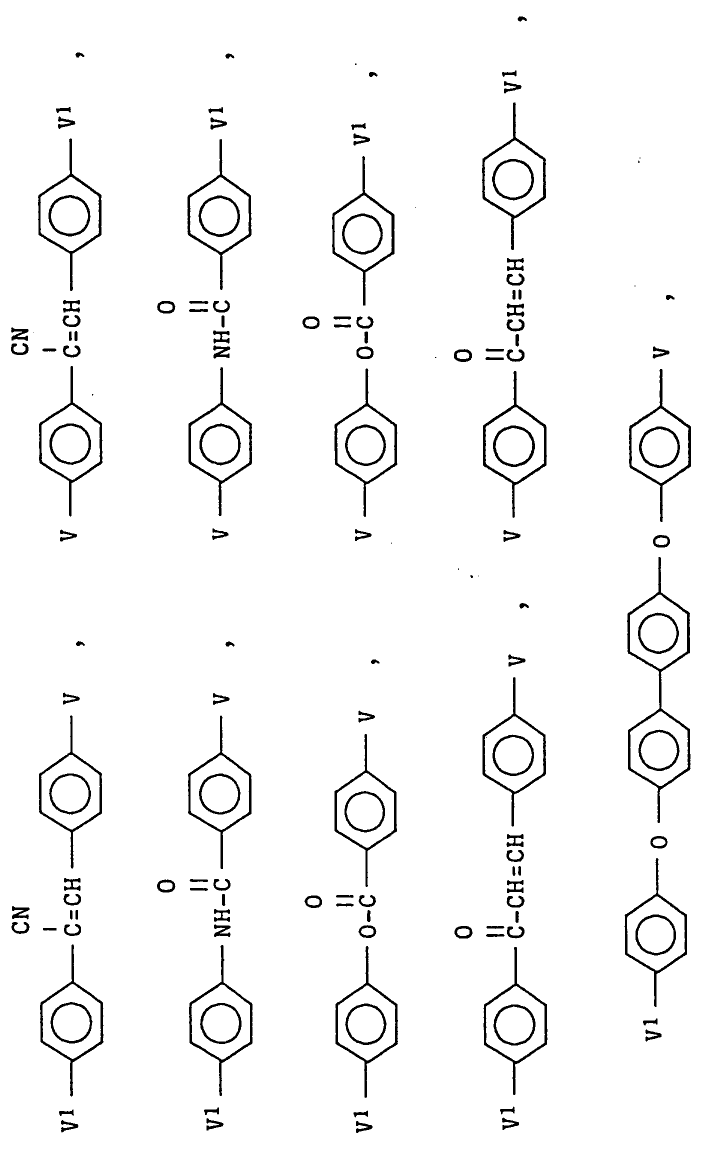 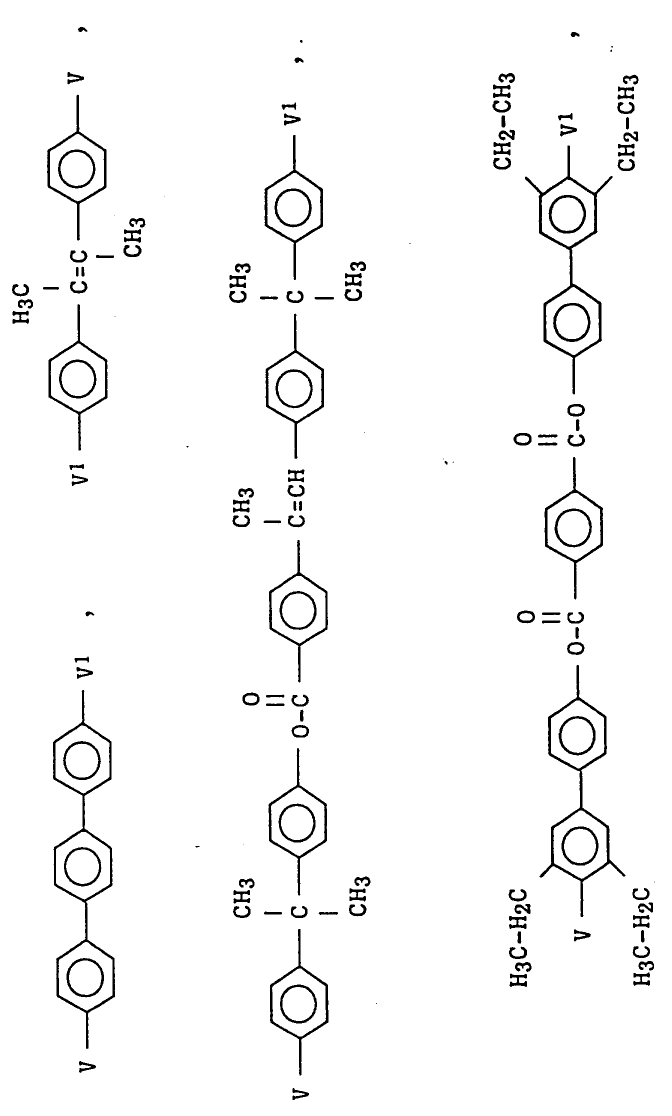 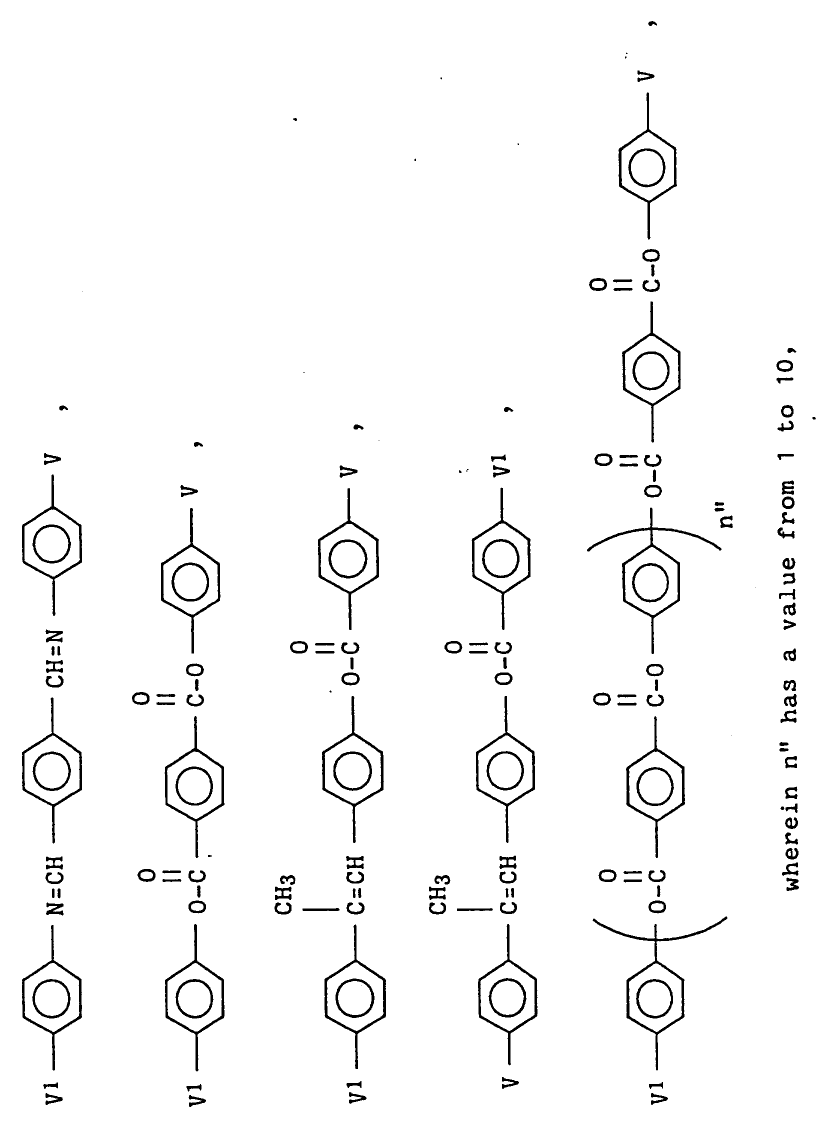 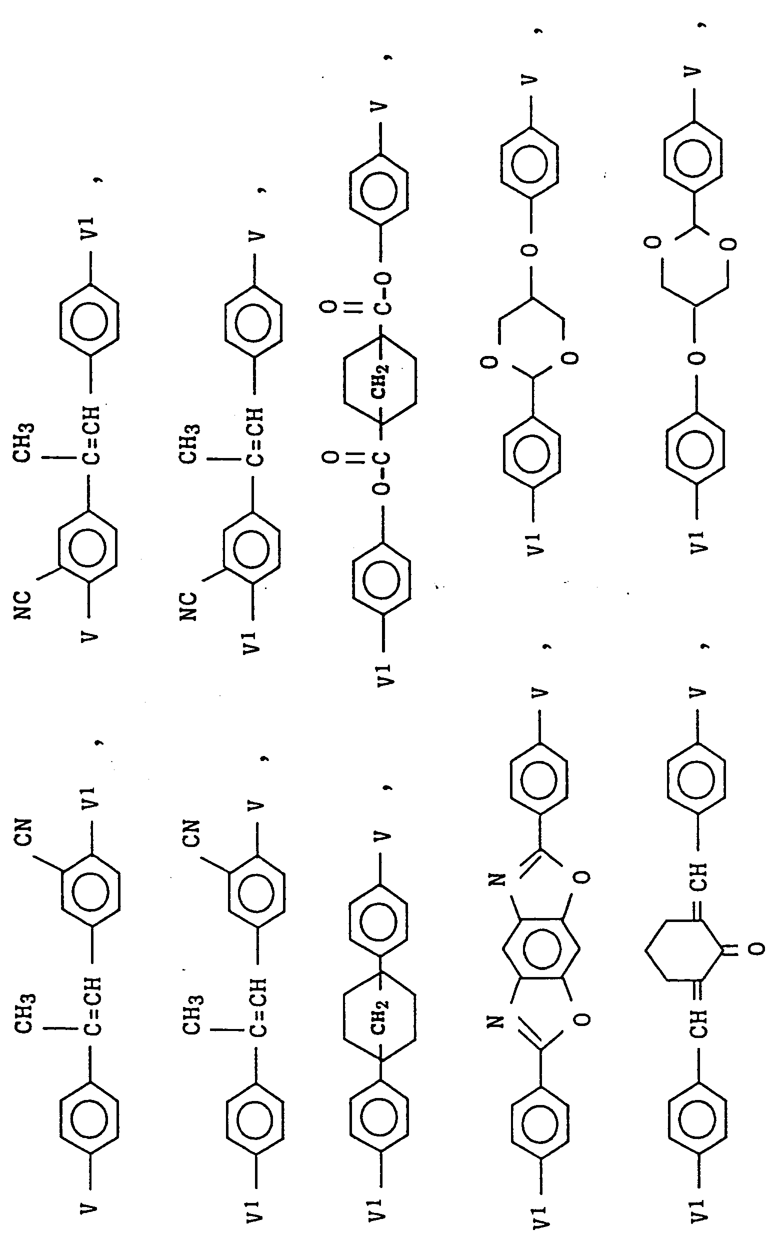 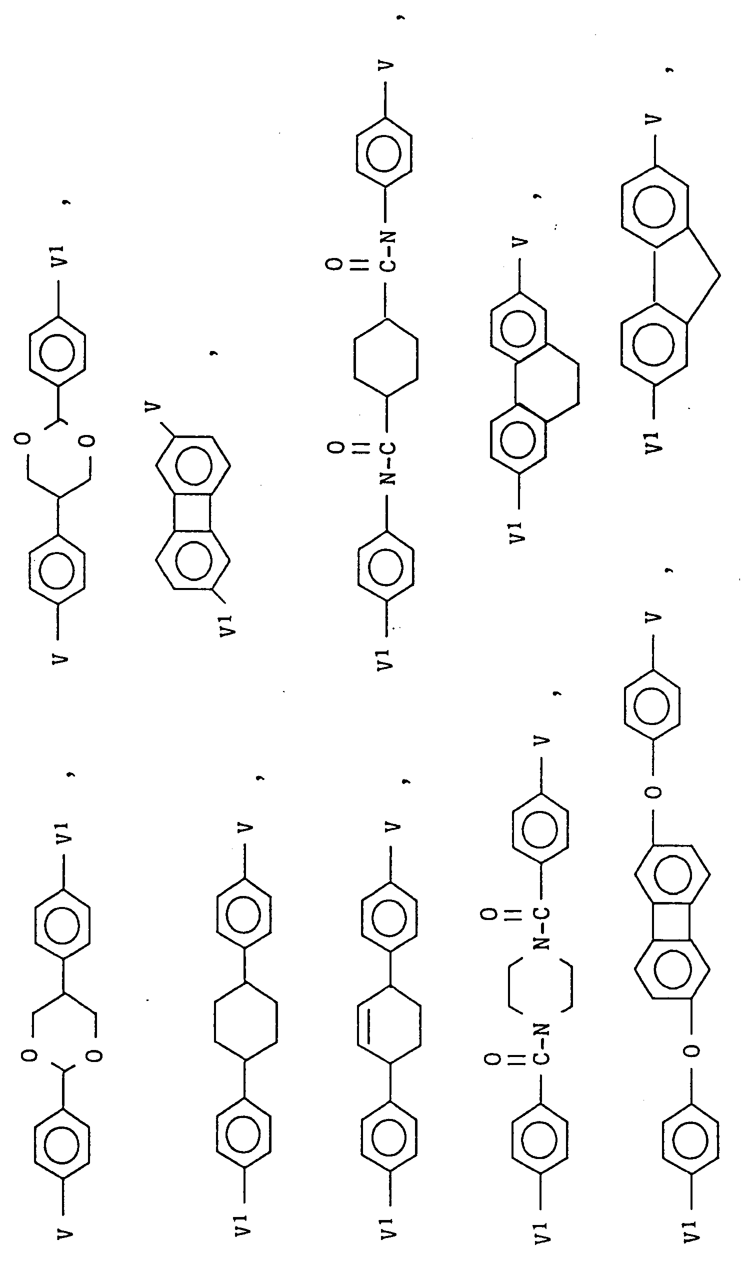 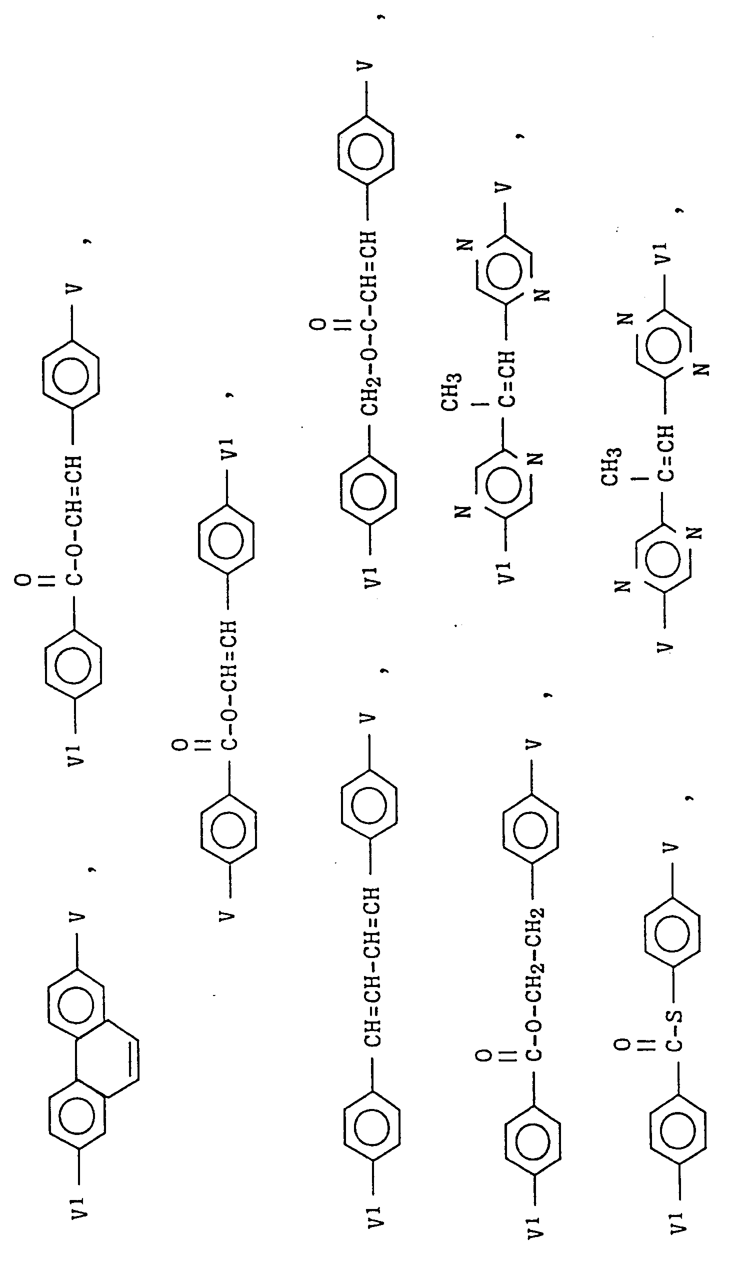 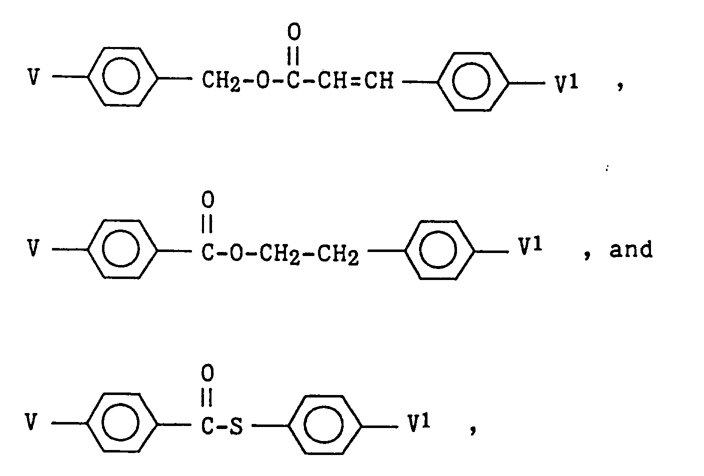 where V¹ is a maleimide group represented by the formula 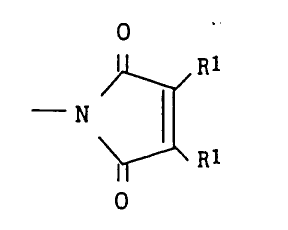 and V is a cyanate group, -O-C≡N, and where R¹ is independently hydrogen or a hydrocarbyl group having 1 to 3 carbon atoms.

4. The product resulting from curing a composition containing one or more compounds of Claim 1, 2 or 3, optionally in the presence of one or more catalysts.

5. A curable composition comprising a mixture containing
(A) at least one compound containing at least one cyanate group, at least one maleimide group and at least one mesogenic or rodlike moiety as defined in claim 1; and
(B) at least one of
(1) at least one polycyanate or polycyanamide which does not contain mesogenic or rodlike structures;
(2) at least one epoxy resin;
(3) at least one polymaleimide;
(4) at least one polyamine;
(5) at least one polyphenol;
(6) at least one compound containing one or more polymerizable ethylenically unsaturated group(s);
(7) at least one compound which contains in the same molecule both a cyanate or cyanamide group and a polymerizable ethylenically unsaturated group;
(8) at least one compound which contains in the same molecule both a 1,2-epoxide group and a polymerizable ethylenically unsaturated group;
(9) at least one compound which contains in the same molecule both a maleimide group and a cyanate group and does not contain mesogenic or rodlike structures;
(10) at least one compound which contains one or more mesogenic or rodlike moieties and only one cyanate or cyanamide group per molecule;
(11) at least one prepolymer of any of the aforesaid components (1) through (10) or any combination of any two or more of said components; or
(12) a mixture of any two or more of components (1) through (11) in any proportion and any combination.

6. A composition of Claim 5 wherein component (A) is present in an amount of from 1 to 99 weight percent of the combined weight of components (A) and (B) and component (B) is present in an amount of from 99 to 1 weight percent of the combined weight of components (A) and (B).

7. A composition of Claim 5 or 6 wherein component (A) is one or more compounds represented by the following general Formulas I, II, III or IV: 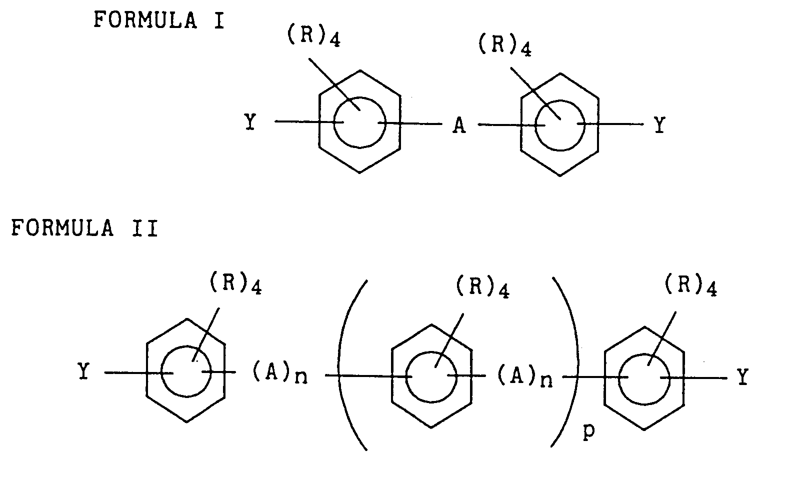 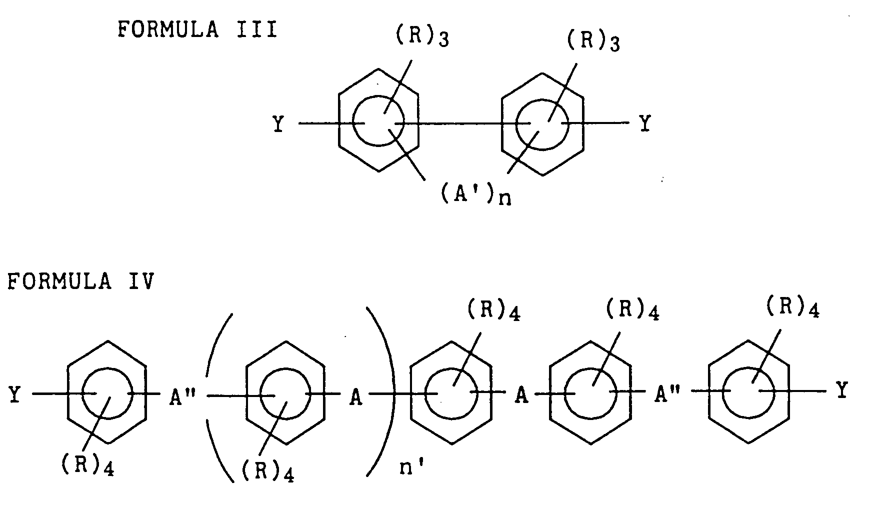 wherein at least about 80 percent of the -A- linkages in Formulas I, II and IV and the direct bond in Formula III and the Y groups are in the para position with respect to each other; one Y group is a cyanate, -0-C≡N, group and the other Y group is a maleimide group represented by the formula 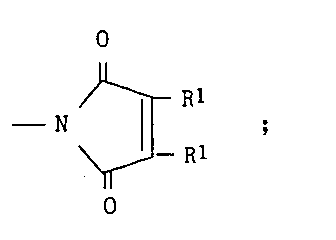 each A is independently -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 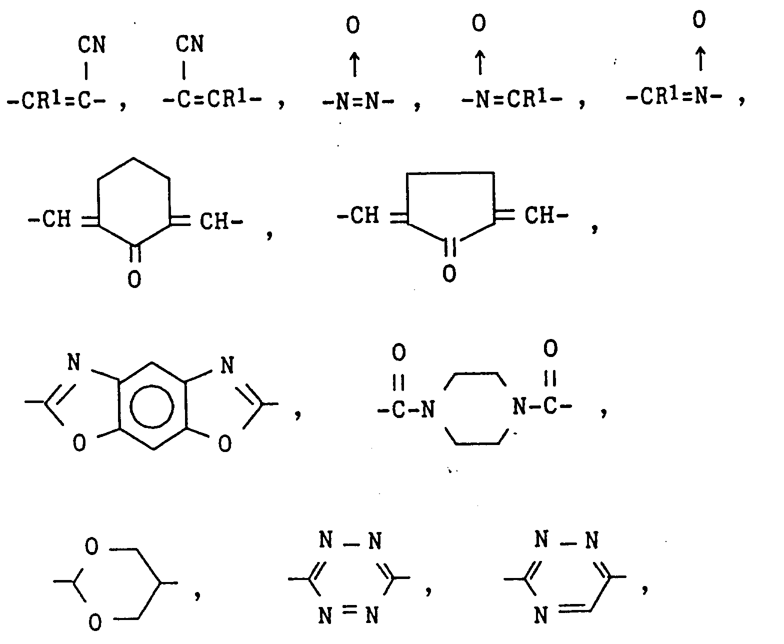 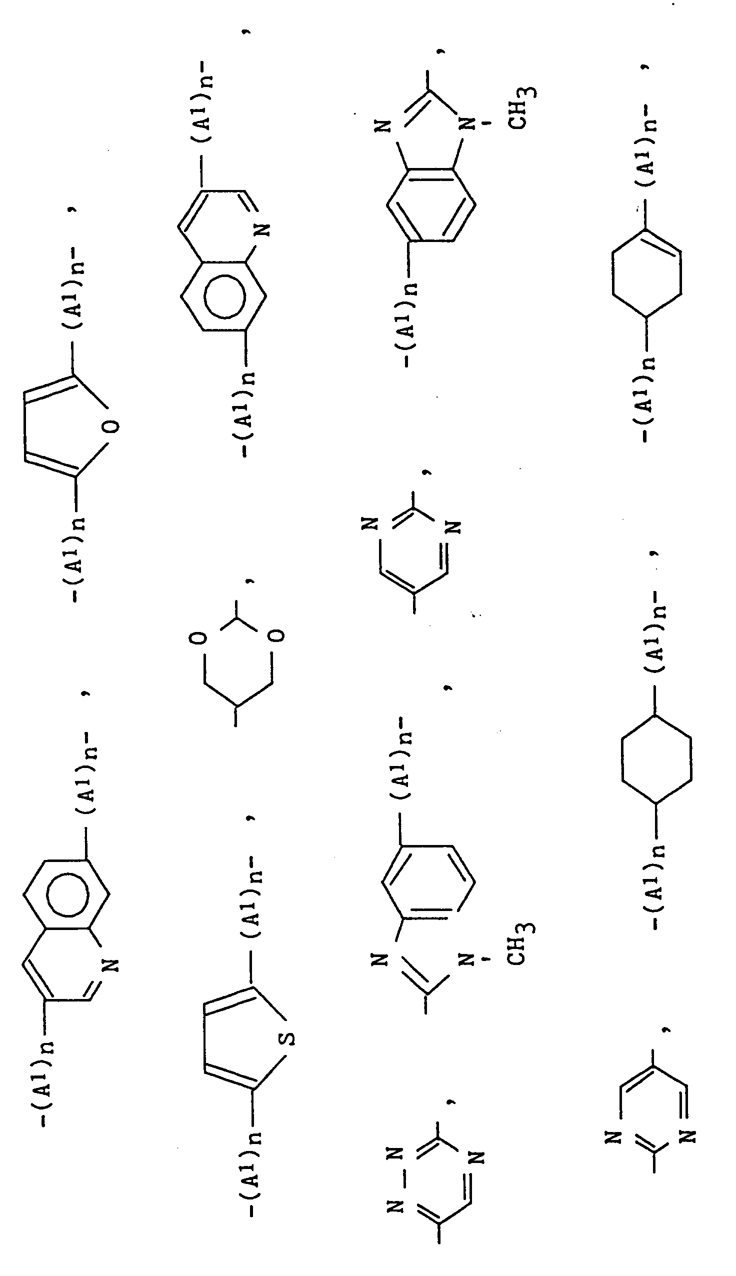 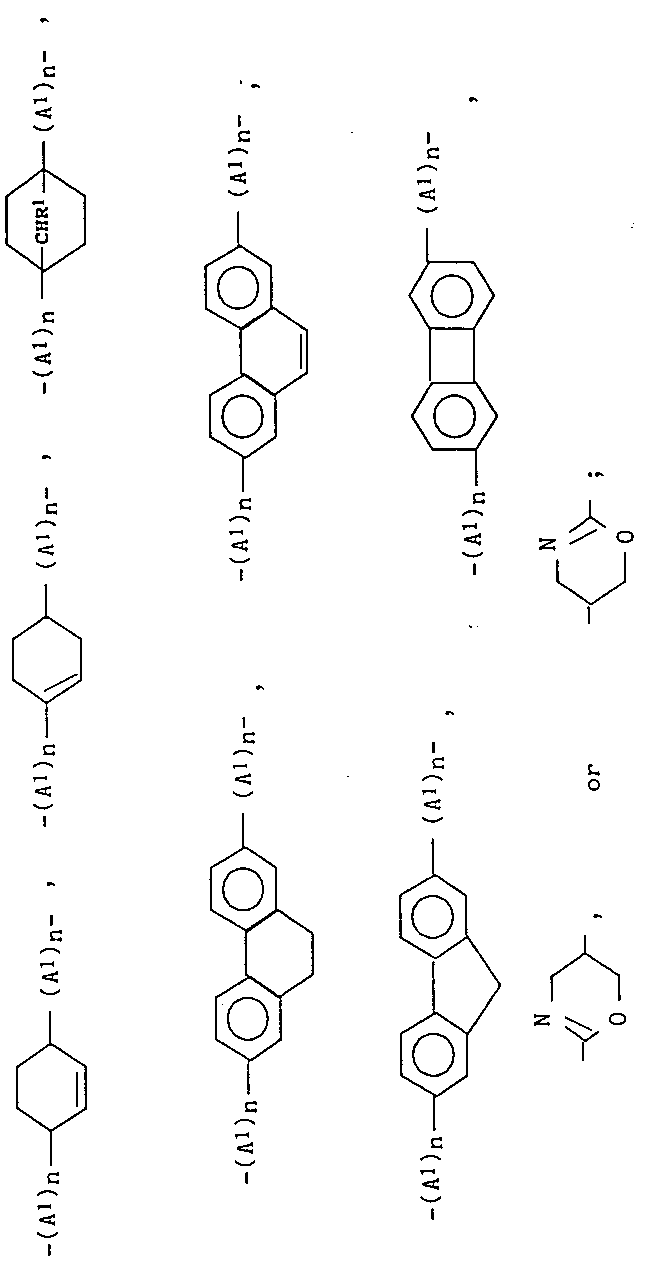 each A' is independently a divalent hydrocarbyl group having from 1 to 10 carbon atoms; each A'' is independently an alkylene group having from 1 to 10 carbon atoms, direct bond, -O-, -CO-, -S-, -S-S-, -SO-, -SO₂- or -O-CO-O-; each A¹ is independently a -CO-, -O-CO-, -CO-O-, -CO-NR¹-, or -NR¹-CO- group; each R is independently hydrogen or a hydrocarbyl or hydrocarbyloxy group having from 1 to 10 carbon atoms, a halogen atom, a nitro group, a nitrile group, a phenyl group or a -CO-R¹ group; each R¹ is independently hydrogen or a hydrocarbyl group having 1 to 3 carbon atoms; n has a value of zero or one; n' has an average value from zero to 6; and p has an average value from 1 to 30; with the proviso that any of the aromatic rings can, if desired, contain a nitrogen, oxygen or sulfur heteroatom.

8. A composition of Claim 7 wherein in component (A) each A' independently has from 1 to 4 carbon atoms; when A'' is an alkylene group, it has from 1 to 4 carbon atoms; each R is independently hydrogen or a hydrocarbyl group having from 1 to 4 carbon atoms, chlorine or bromine; n' has an average value from zero to 3; and p has an average value from 1 to 3.

9. A composition of Claim 5 or 6 wherein component (A) is one or more compounds selected from the group consisting of 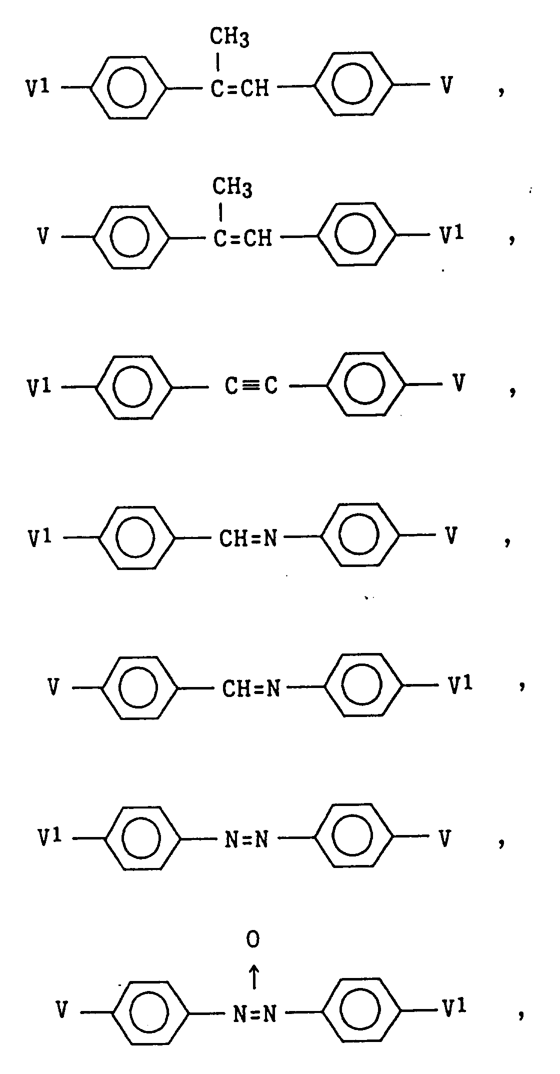 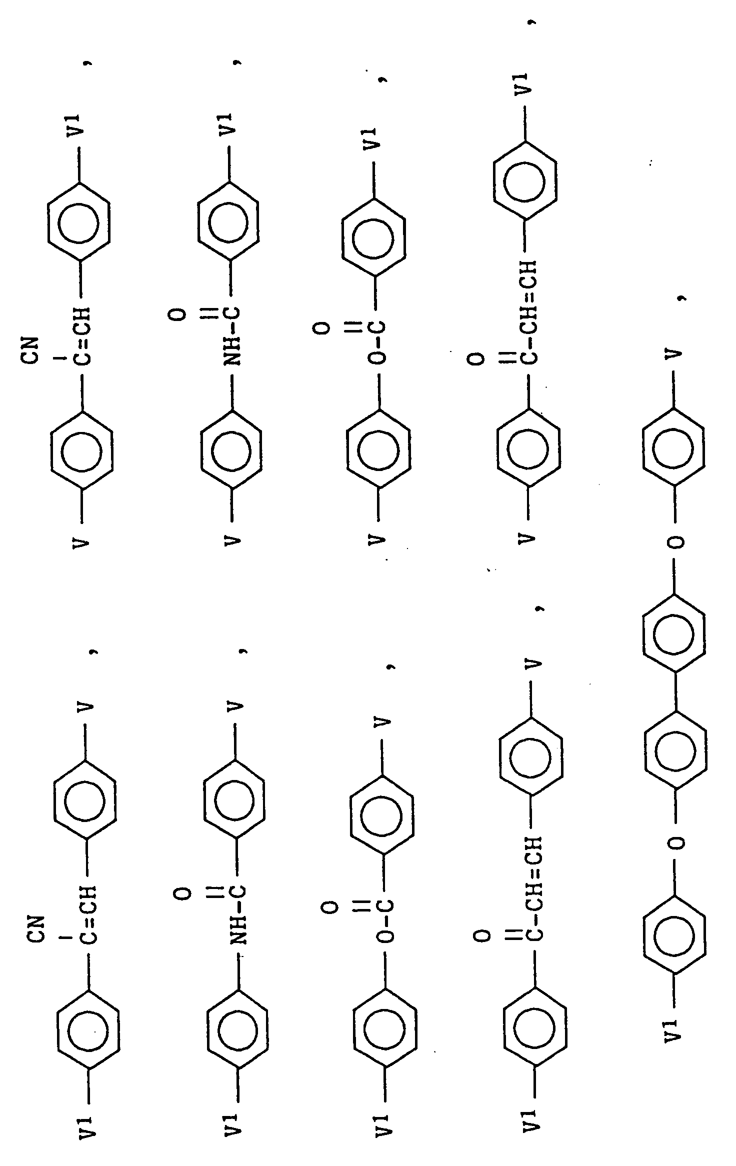 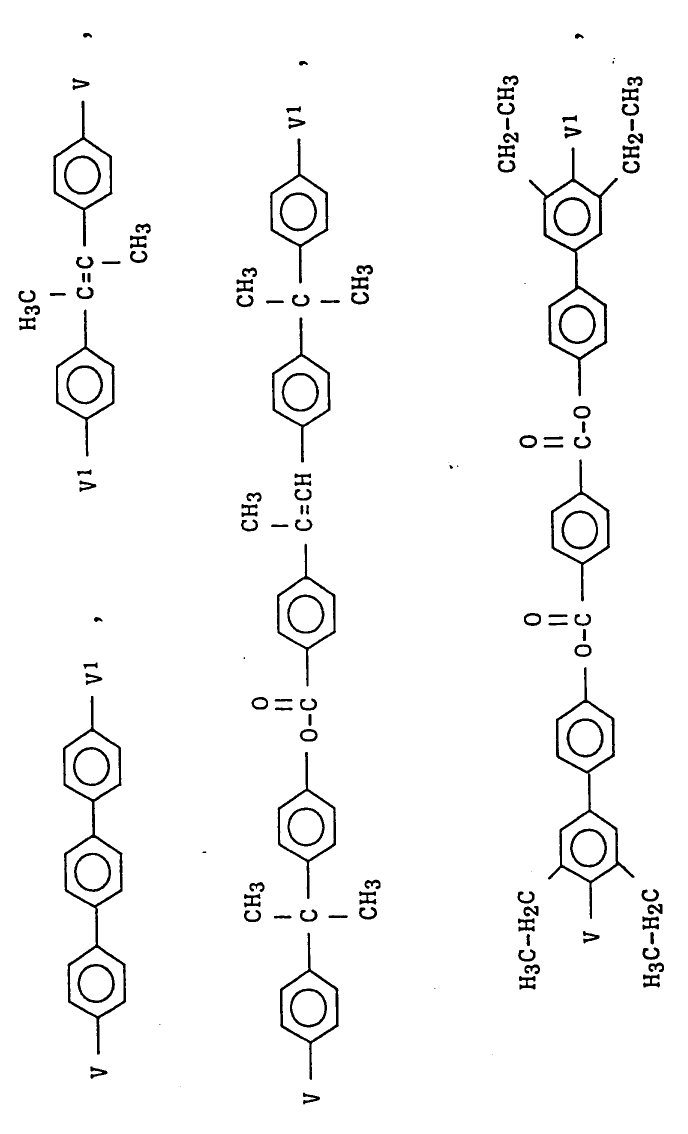 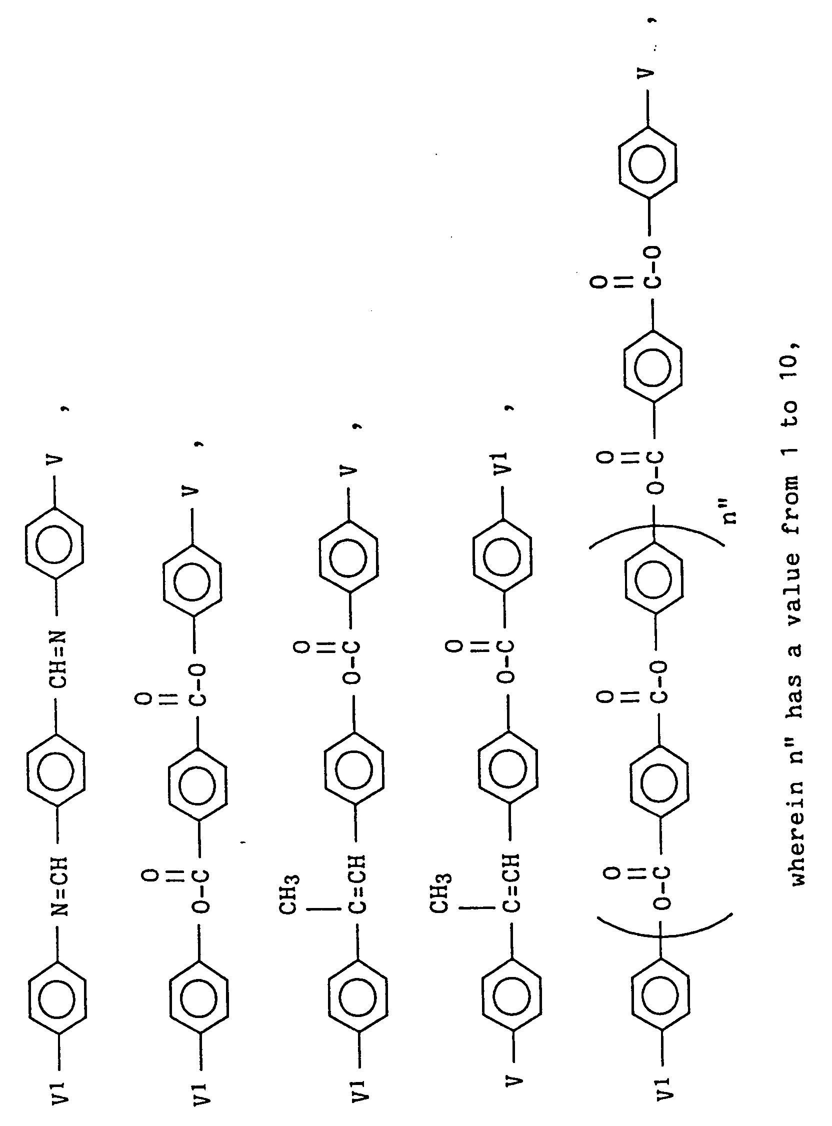 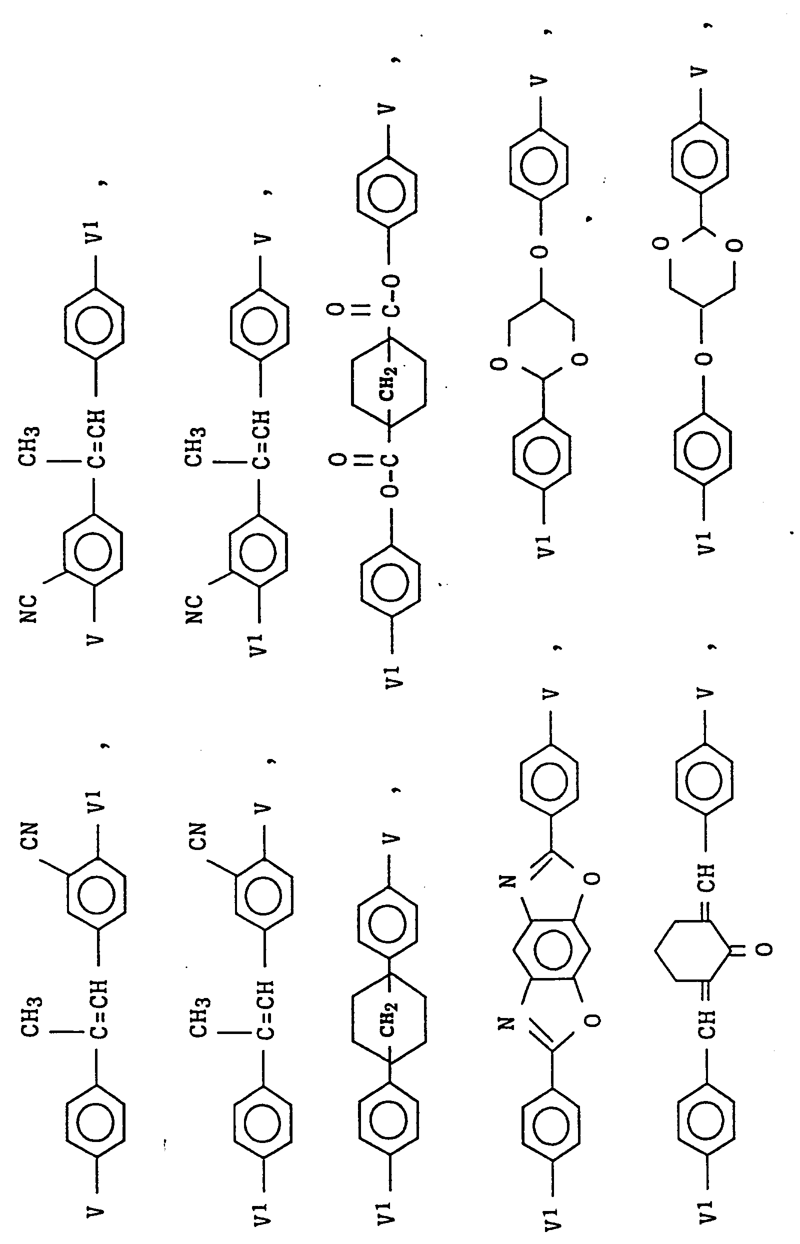 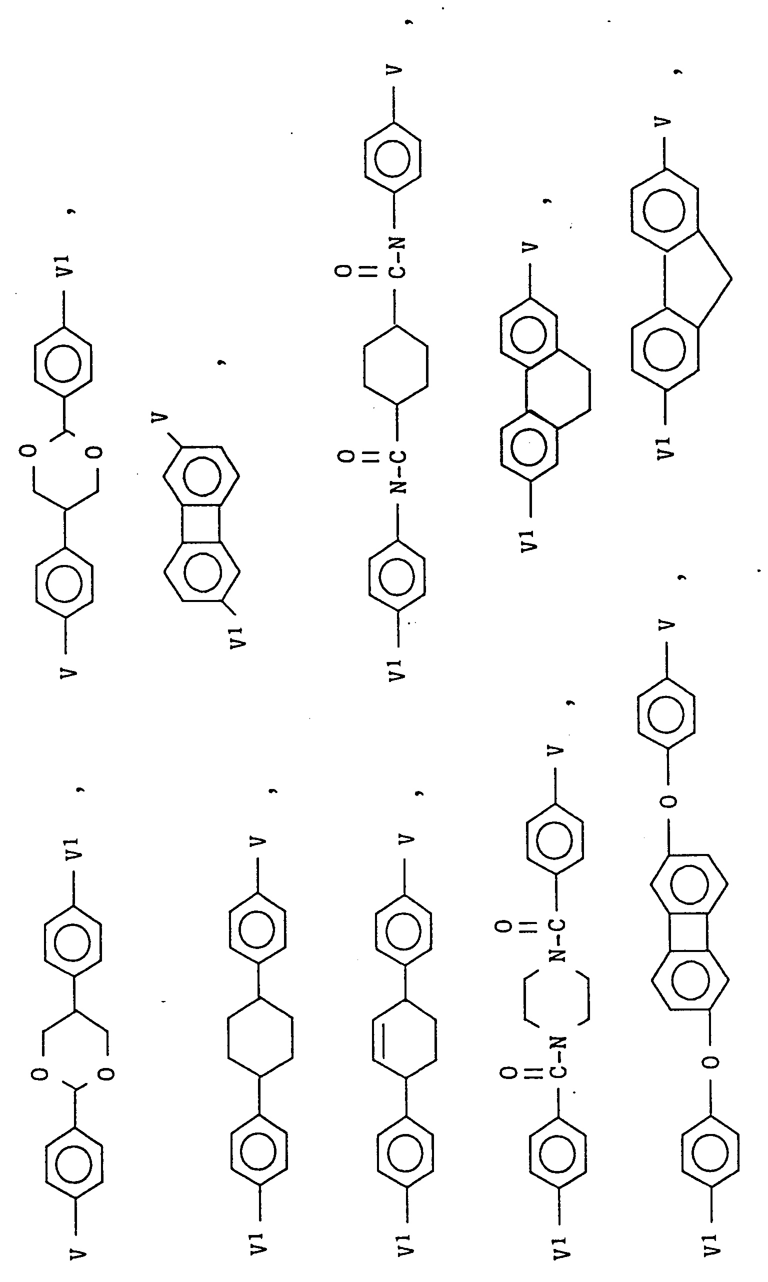 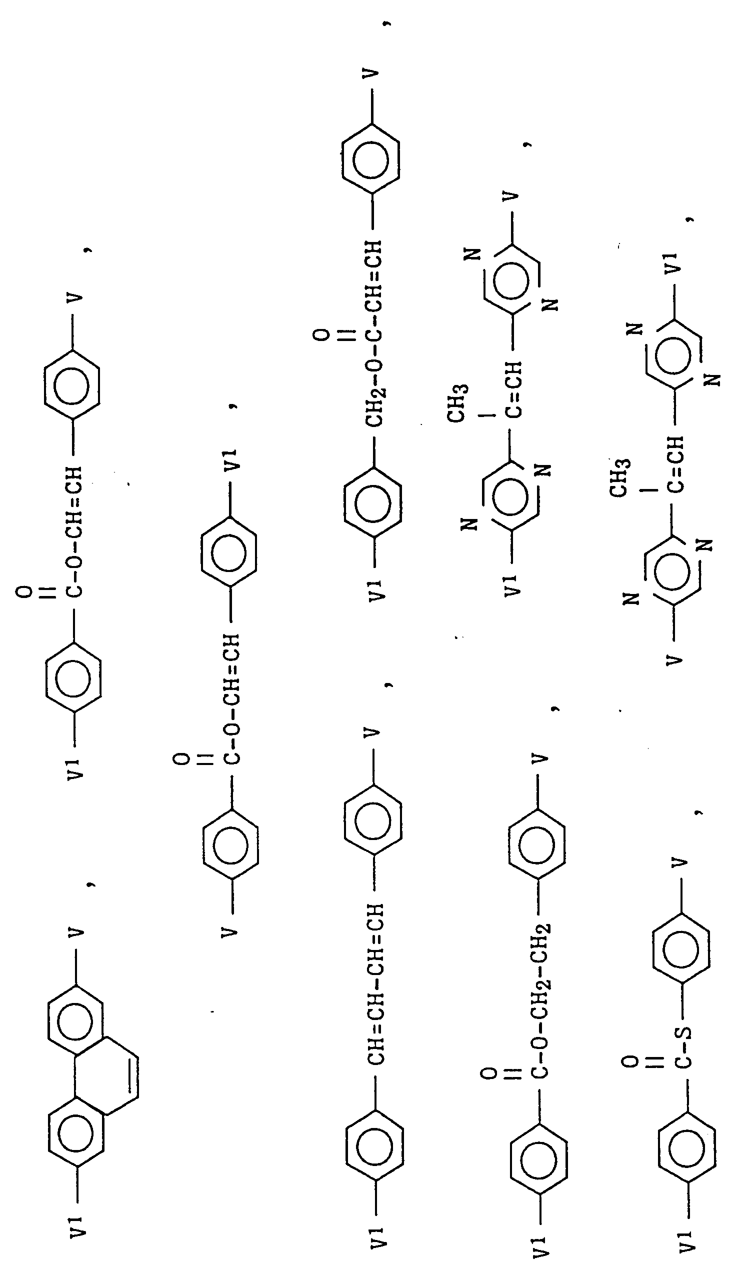 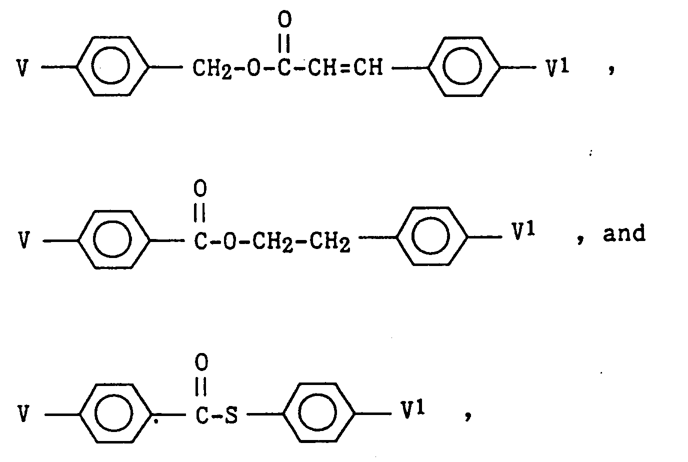 where V¹ is a maleimide group represented by the formula 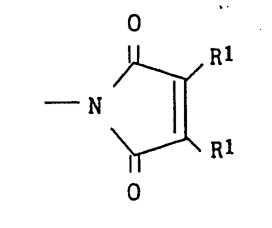 and V is a cyanate group, -O-C≡N, and where R¹ is independently hydrogen or a hydrocarbyl group having 1 to 3 carbon atoms.

10. The product resulting from curing the curable composition of Claim 5, 6, 7, 8 or 9.

11. The product of Claim 4 or 9 wherein the mesogenic or rodlike moieties are oriented prior to or during curing.

12. The product of Claim 11 wherein said orientation is accomplished by means of an electric or magnetic field or by shear forces.

## Patentansprüche

1. Verbindung, welche wenigstens eine Cyanatgruppe, wenigstens eine Maleinimidgruppe und wenigstens eine mesogene oder stabförmige Einheit enthält, wiedergegeben durch die folgenden Formeln I, II, III oder IV: 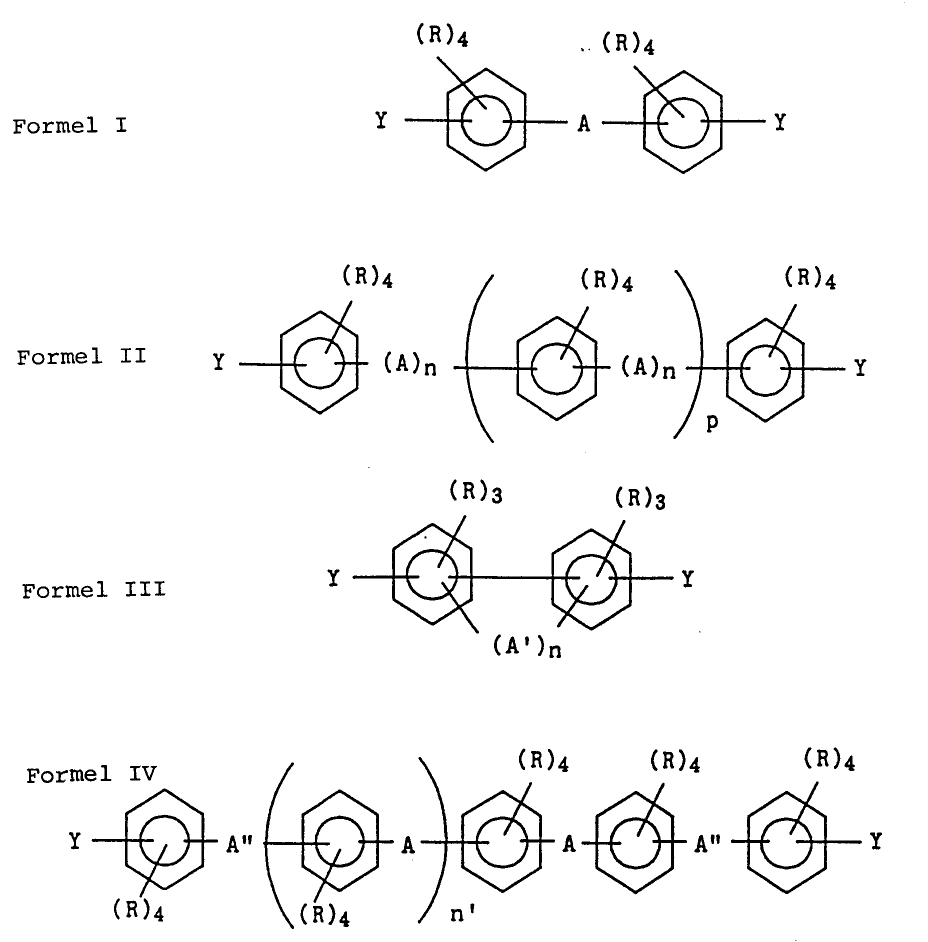 worin wenigstens etwa 80 Gew.-% der -A- Bindungen in den Formeln I, II und IV und die direkte Bindung in der Formel III und die Y-Gruppen in der para-Stellung bezogen aufeinander stehen; eine Y-Gruppe eine Cyanatgruppe, -O-C≡N, ist, und die andere Y-Gruppe eine Maleinimidgruppe ist, welche durch die Formel wiedergegeben wird 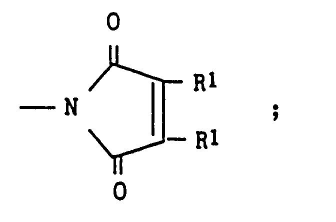 jedes A unabhängig ist: -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 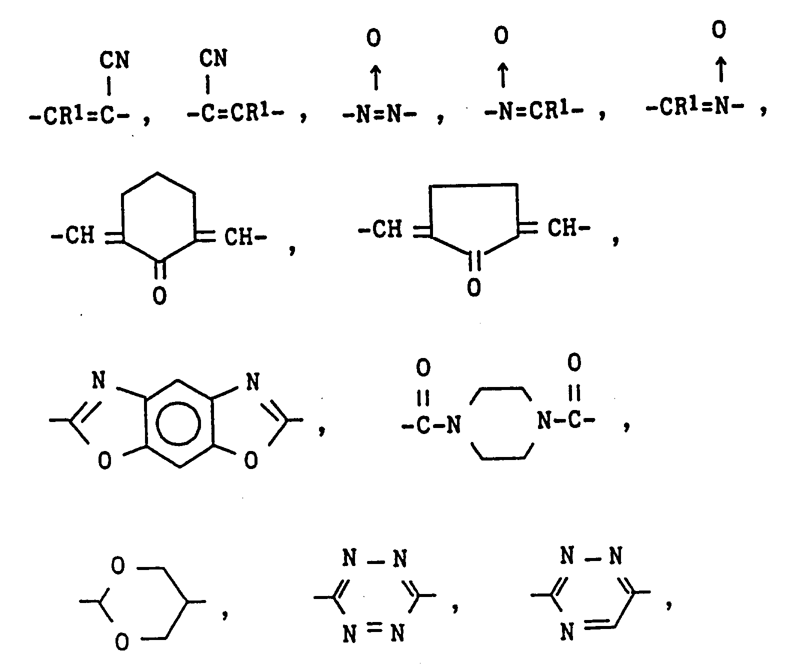 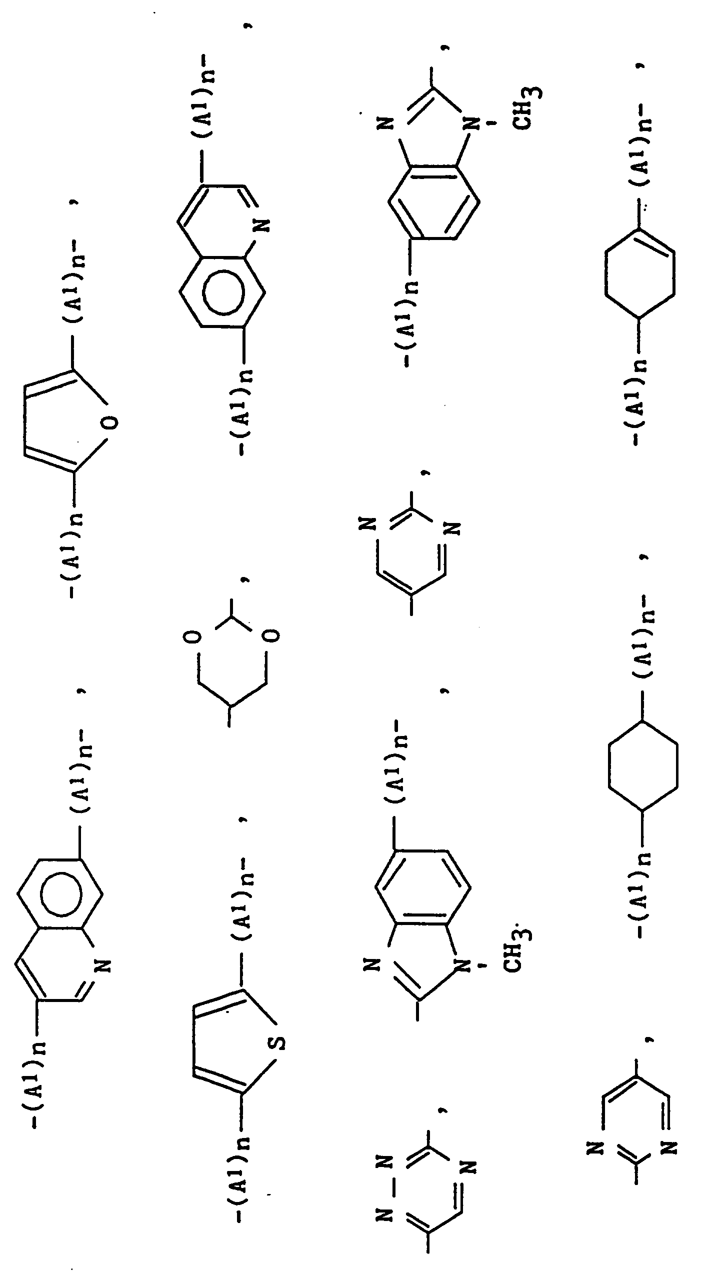 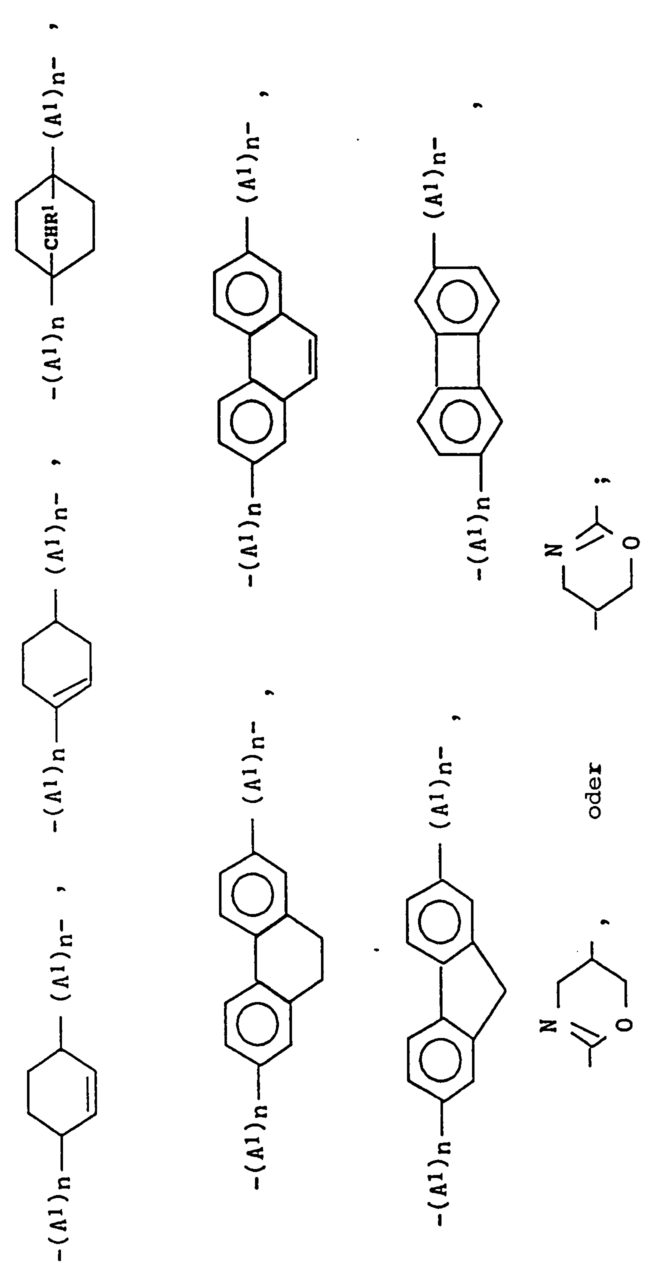 jedes A' unabhängig eine zweiwertige Hydrocarbylgruppe mit 1 bis 10 Kohlenstoffatomen ist; jedes A'' unabhängig eine Alkylengruppe mit 1 bis 10 Kohlenstoffatomen, eine direkte Bindung, -O-, -CO-, -S-, -S-S-, -SO-, -SO₂- oder O-CO-O- ist; jedes A¹ unabhängig eine Gruppe -CO-, -O-CO-, -CO-O-, -CO-NR¹- oder -NR¹-CO- ist; jedes R unabhängig Wasserstoff oder eine Hydrocarbyl- oder Hydrocarbyloxygruppe mit 1 bis 10 Kohlenstoffatomen, ein Halogenatom, eine Nitrogruppe, eine Nitrilgruppe, eine Phenylgruppe oder eine Gruppe -CO-R¹- ist; jedes R¹ unabhängig Wasserstoff oder eine Hydrocarbylgruppe mit 1 bis 3 Kohlenstoffatomen ist; n einen Wert von null oder eins besitzt; n' einen Durchschnittswert von null bis 6 besitzt; und p einen Durchschnittswert von 1 bis 30 besitzt; mit der Maßgabe, daß ein beliebiger der aromatischen Ringe, falls gewünscht, ein Stickstoff-, Sauerstoff- oder Schwefelheteroatom enthalten kann.

2. Verbindung nach Anspruch 1, worin jedes A' unabhängig von 1 bis 4 Kohlenstoffatome hat; falls A'' eine Alkylengruppe ist, sie von 1 bis 4 Kohlenstoffatome hat; jedes R unabhängig Wasserstoff oder eine Hydrocarbylgruppe mit 1 bis 4 Kohlenstoffatomen, Chlor oder Brom ist; n' einen Durchschnittswert von null bis 3 besitzt; und p einen Durchschnittswert von 1 bis 3 besitzt.

3. Verbindung nach Anspruch 1, ausgewählt aus der Gruppe, welche besteht aus: 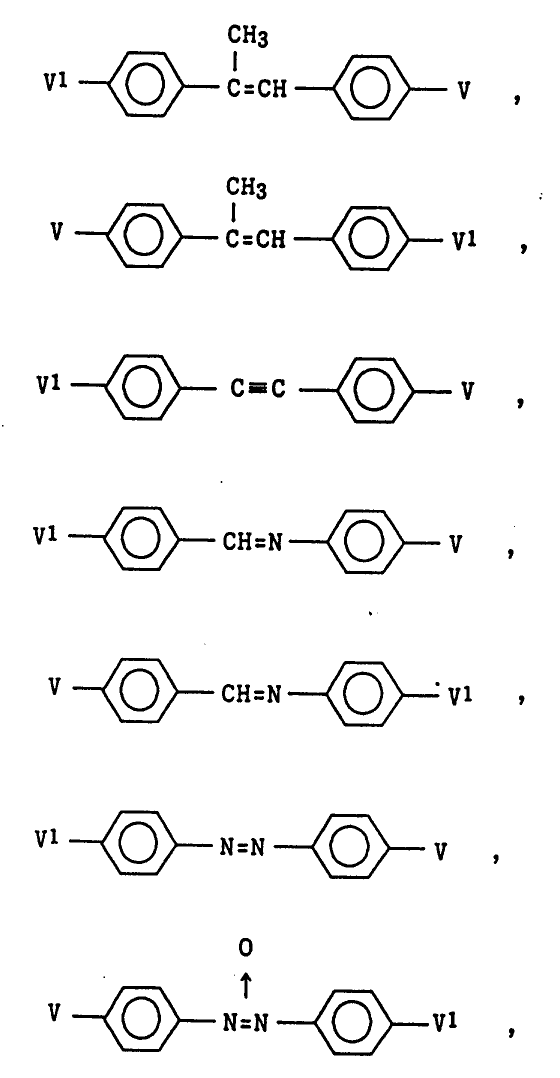 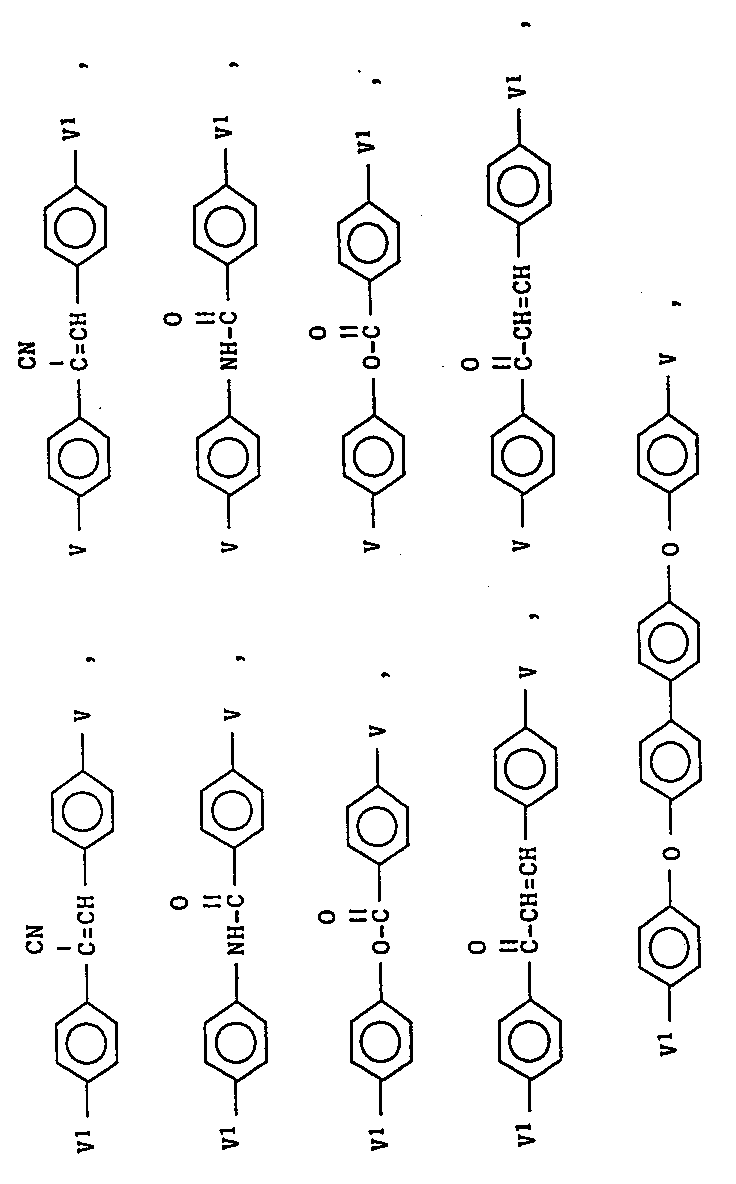 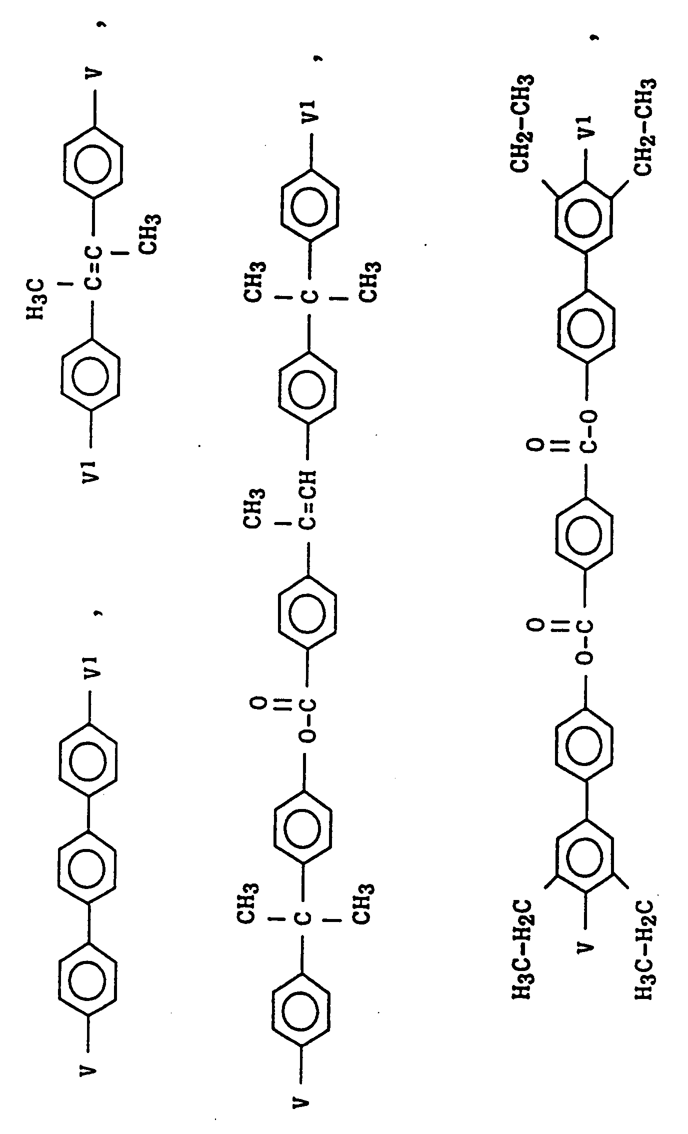 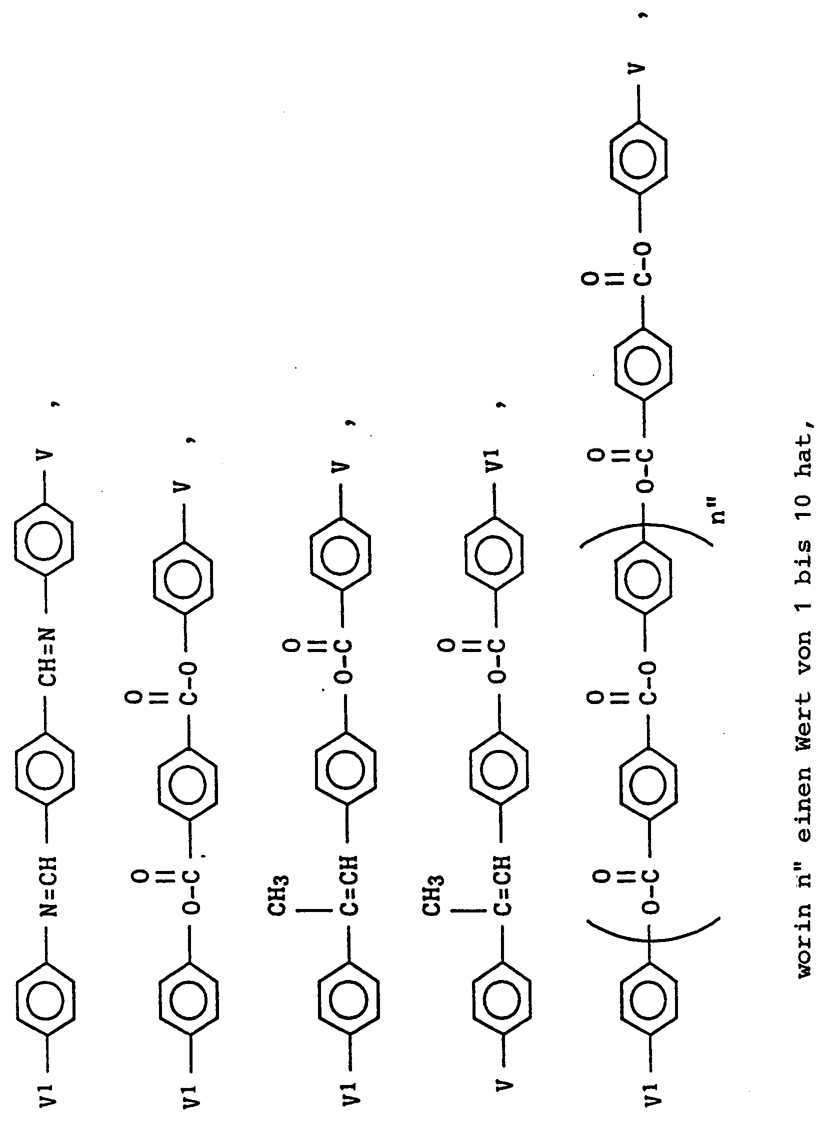 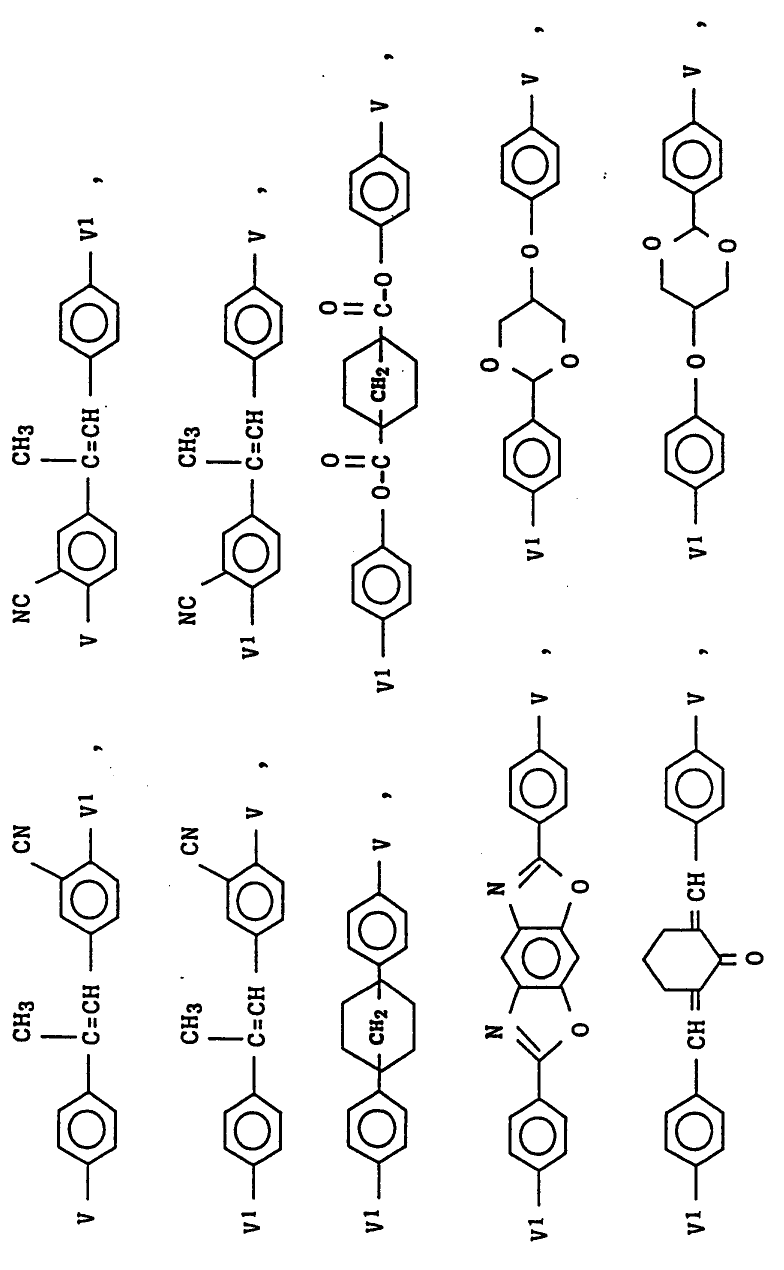 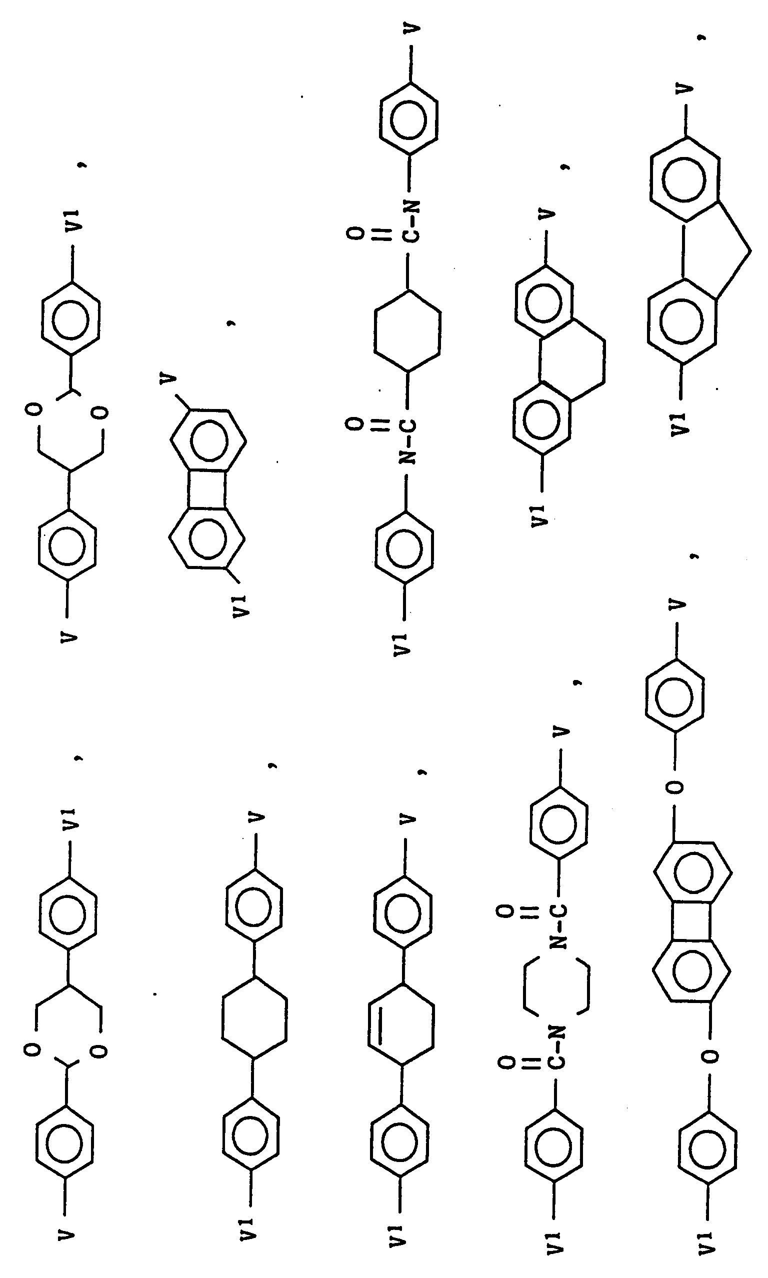 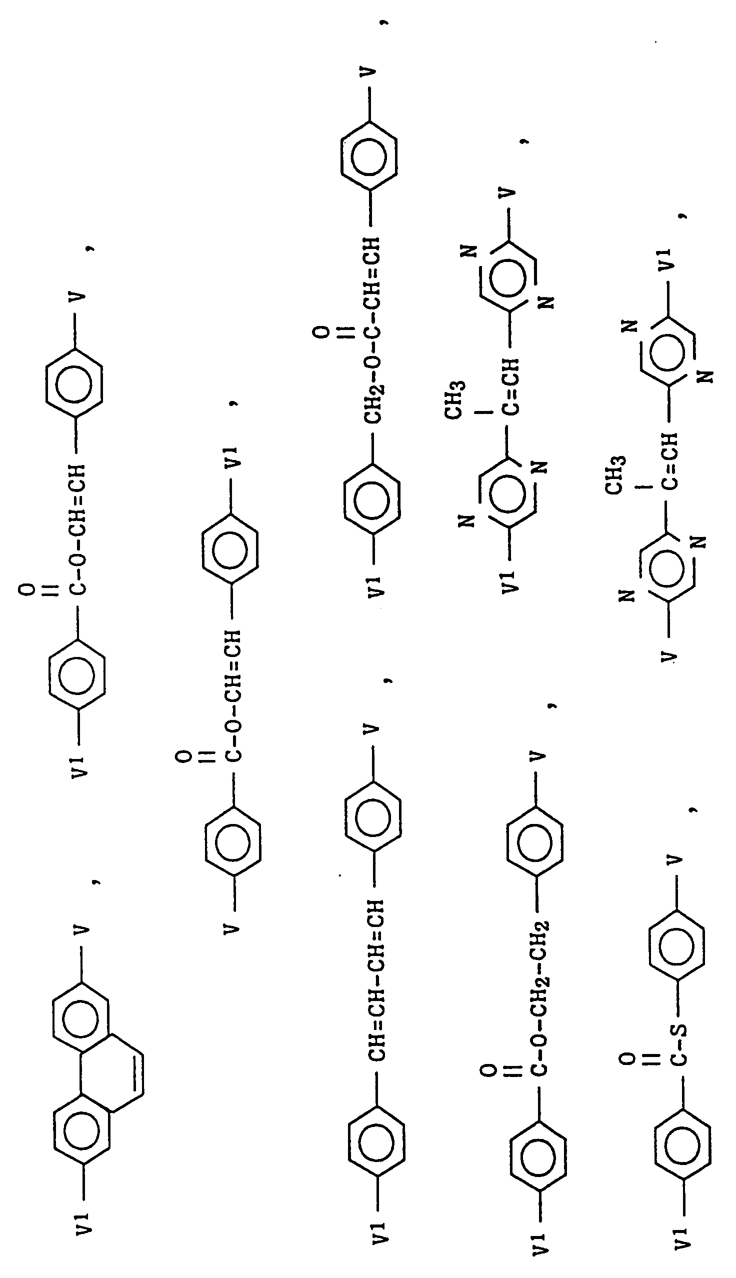 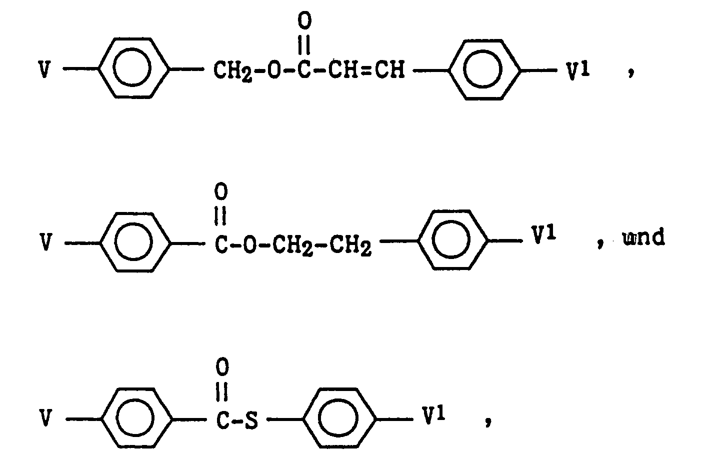 worin V¹ eine Maleinimidgruppe ist, welche durch die Formel wiedergegeben wird: 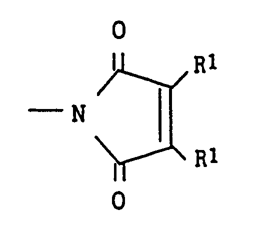 und V eine Cyanatgruppe, -O-C≡N, ist, und worin R¹ unabhängig Wasserstoff oder eine Hydrocarbylgruppe mit 1 bis 3 Kohlenstoffatomen ist.

4. Produkt, resultierend aus dem Aushärten einer Zusammensetzung, welche eine oder mehrere Verbindungen von Anspruch 1, 2 oder 3 enthält, wahlweise in Anwesenheit von einem oder mehreren Katalysatoren.

5. Aushärtbare Zusammensetzung, umfassend eine Mischung, welche enthält:
(A) wenigstens eine Verbindung, welche wenigstens eine Cyanatgruppe, wenigstens eine Maleinimidgruppe und wenigstens eine mesogene oder stabförmige Einheit enthält, wie in Anspruch 1 definiert; und
(B) wenigstens eine Substanz aus:
(1) wenigstens einem Polycyanat oder Polycyanamid, welches keine mesogenen oder stabförmigen Strukturen enthält;
(2) wenigstens einem Epoxyharz;
(3) wenigstens einem Polymaleinimid;
(4) wenigstens einem Polyamin;
(5) wenigstens einem Polyphenol;
(6) wenigstens einer Verbindung, welche eine oder mehrere polymerisierbare ethylenartig ungesättigte Gruppe/n enthält;
(7) wenigstens eine Verbindung, welche in demselben Molekül sowohl eine Cyanat- oder Cyanamidgruppe als auch eine polymerisierbare ethylenartig ungesättigte Gruppe enthält;
(8) wenigstens eine Verbindung, welche in demselben Molekül sowohl eine 1,2-Epoxidgruppe als auch eine polymerisierbare ethylenartig ungesättigte Gruppe enthält;
(9) wenigstens eine Verbindung, welche in demselben Molekül sowohl eine Maleinimidgruppe als auch eine Cyanatgruppe enthält, und keine mesogene oder stabförmige Strukturen enthält;
(10) wenigstens eine Verbindung, welche eine oder mehrere mesogene oder stabförmige Strukturen und nur eine Cyanat- oder Cyanamidgruppe pro Molekül enthält;
(11) wenigstens ein Prepolymer einer beliebigen der zuvorgenannten Komponenten (1) bis (10) oder eine beliebige Kombination von beliebigen zwei oder mehr dieser Komponenten; oder
(12) eine Mischung von beliebigen zwei oder mehr der Komponenten (1) bis (11) in einem beliebigen Verhältnis und in einer beliebigen Kombination.

6. Zusammensetzung nach Anspruch 5, worin die Komponente (A) in einer Menge von 1 bis 99 Gew.-% des kombinierten Gewichtes der Komponenten (A) und (B) vorhanden ist, und die Komponente (B) in einer Menge von 99 bis 1 Gew.-% des kombinierten Gewichtes der Komponenten (A) und (B) vorhanden ist.

7. Zusammensetzung nach Anspruch 5 oder 6, worin die Komponente (A) eine oder mehrere Verbindungen ist, welche durch die folgenden allgemeinen Formeln I, II, III oder IV wiedergegeben werden: 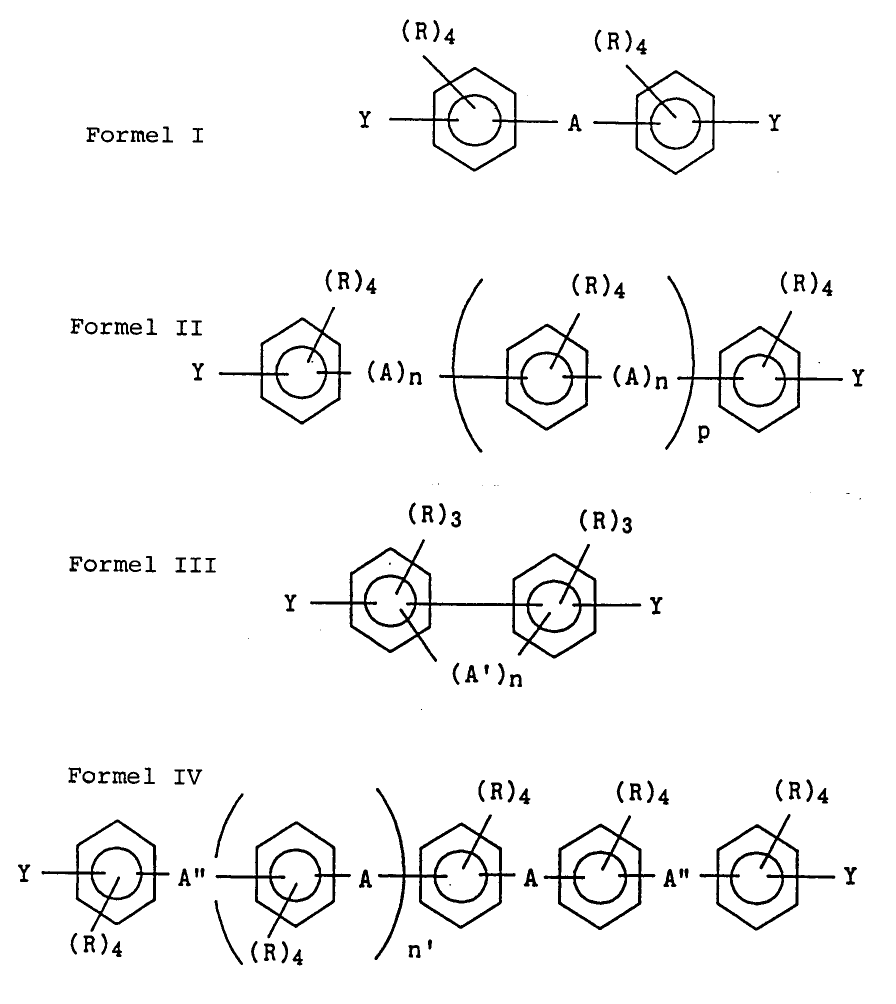 worin wenigstens etwa 80 Gew.-% der -A- Bindungen in den Formeln I, II und IV und die direkte Bindung in der Formel III und die Y-Gruppen in der para-Stellung bezogen aufeinander stehen; eine Y-Gruppe eine Cyanatgruppe, -O-C≡N, ist, und die andere Y-Gruppe eine Maleinimidgruppe ist, welche durch die Formel wiedergegeben wird 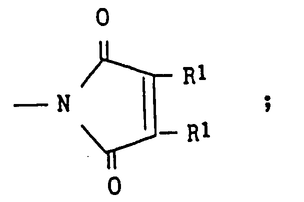 jedes A unabhängig ist: -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 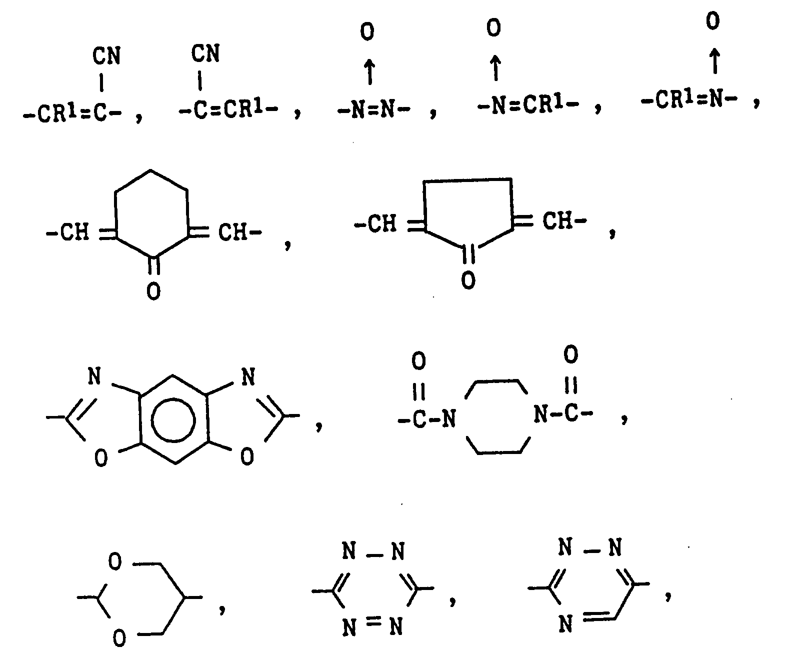 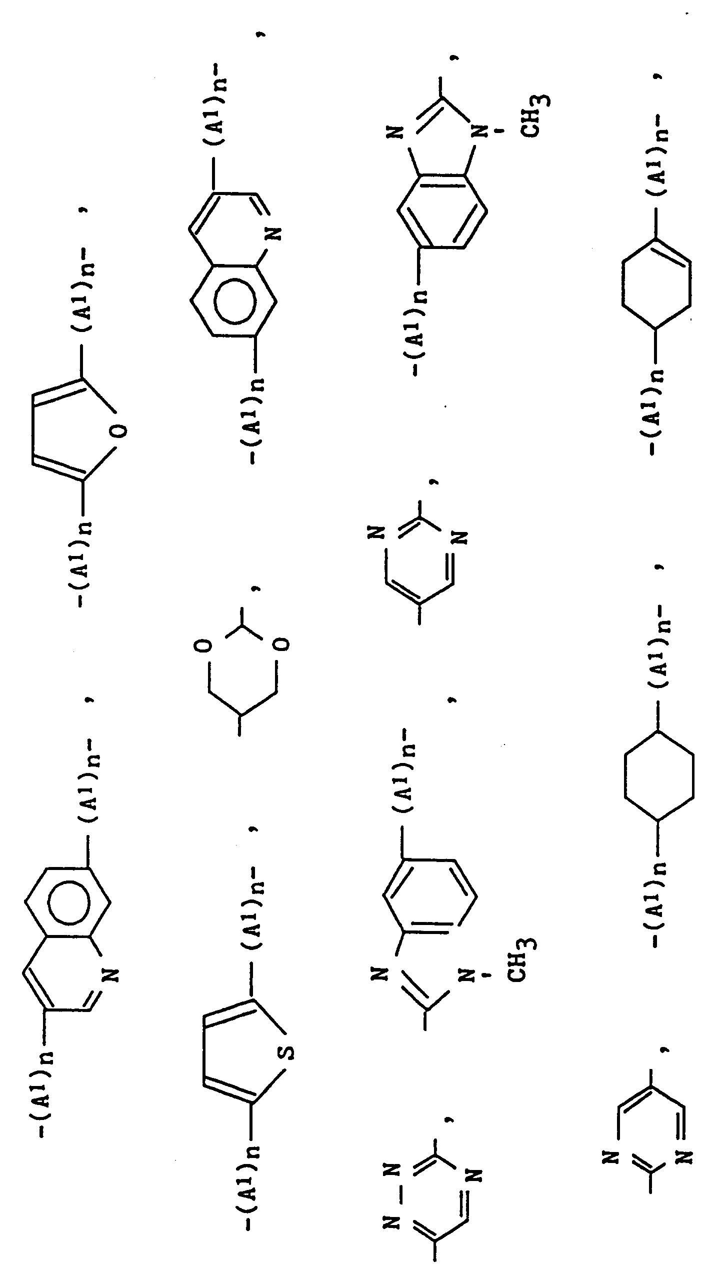 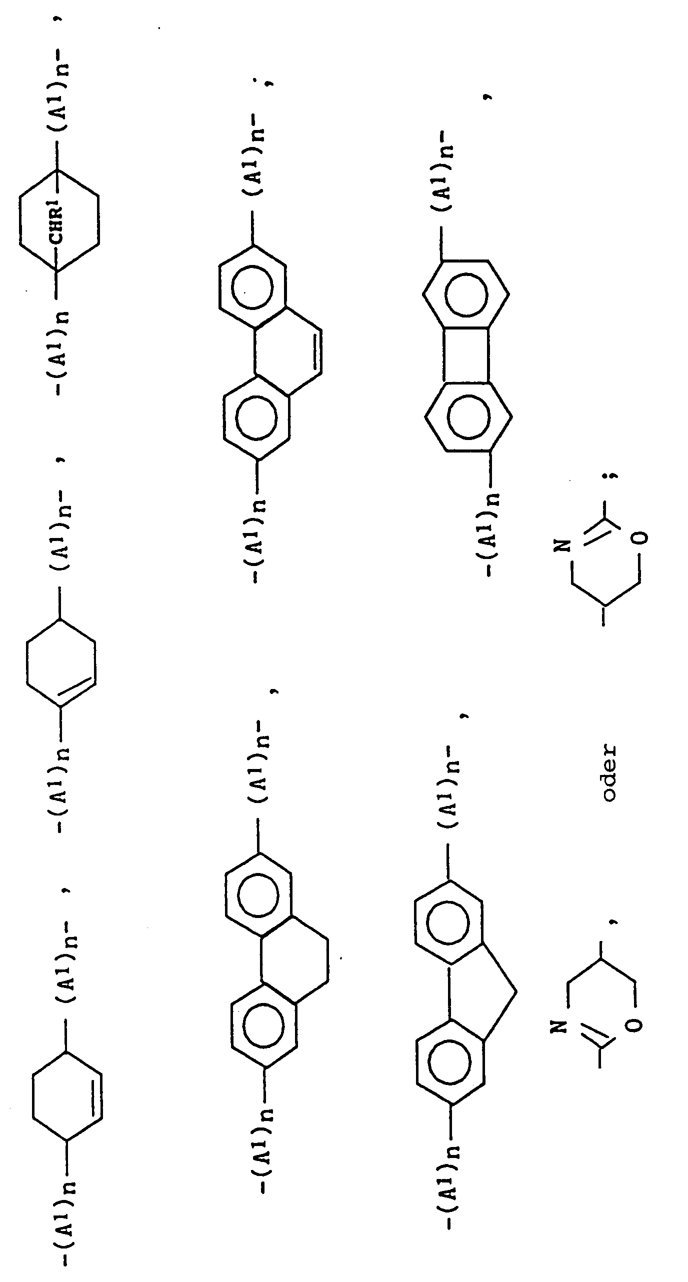 jedes A' unabhängig eine zweiwertige Hydrocarbylgruppe mit 1 bis 10 Kohlenstoffatomen ist; jedes A'' unabhängig eine Alkylengruppe mit 1 bis 10 Kohlenstoffatomen, eine direkte Bindung, -O-, -CO-, -S-, -S-S-, -SO-, -SO₂- oder O-CO-O- ist; jedes A¹ unabhängig eine Gruppe -CO-, -O-CO-, -CO-O-, -CO-NR¹- oder -NR¹-CO- ist; jedes R unabhängig Wasserstoff oder eine Hydrocarbyl- oder Hydrocarbyloxygruppe mit 1 bis 10 Kohlenstoffatomen, ein Halogenatom, eine Nitrogruppe, eine Nitrilgruppe, eine Phenylgruppe oder eine Gruppe -CO-R¹- ist; jedes R¹ unabhängig Wasserstoff oder eine Hydrocarbylgruppe mit 1 bis 3 Kohlenstoffatomen ist; n einen Wert von null oder eins besitzt; n' einen Durchschnittswert von null bis 6 besitzt; und p einen Durchschnittswert von 1 bis 30 besitzt; mit der Maßgabe, daß ein beliebiger der aromatischen Ringe, falls gewünscht, ein Stickstoff-, Sauerstoff- oder Schwefelheteroatom enthalten kann.

8. Zusammensetzung nach Anspruch 7, worin in der Komponente A jedes A' unabhängig von 1 bis 4 Kohlenstoffatome hat; falls A'' eine Alkylengruppe ist, sie von 1 bis 4 Kohlenstoffatome hat; jedes R unabhängig Wasserstoff oder eine Hydrocarbylgruppe mit 1 bis 4 Kohlenstoffatomen, Chlor oder Brom ist; n' einen Durchschnittswert von null bis 3 besitzt; und p einen Durchschnittswert von 1 bis 3 besitzt.

9. Zusammensetzung nach Anspruch 5 oder 6, worin die Komponente (A) eine oder mehrere Verbindungen ist, ausgewählt aus der Gruppe, welche besteht aus: 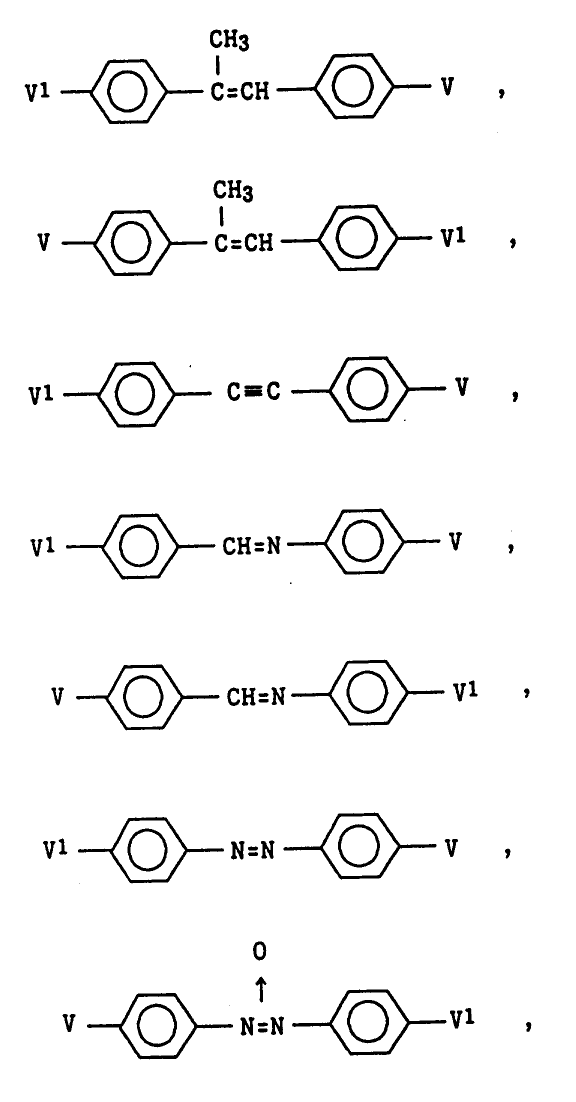 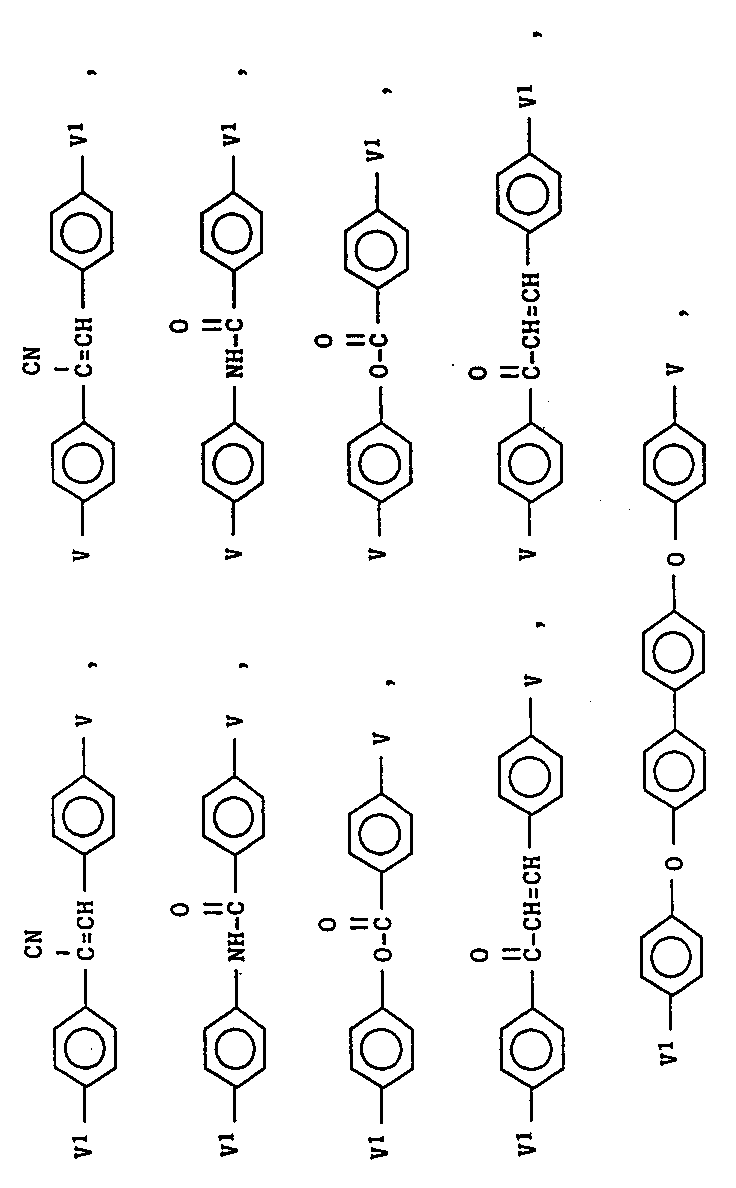 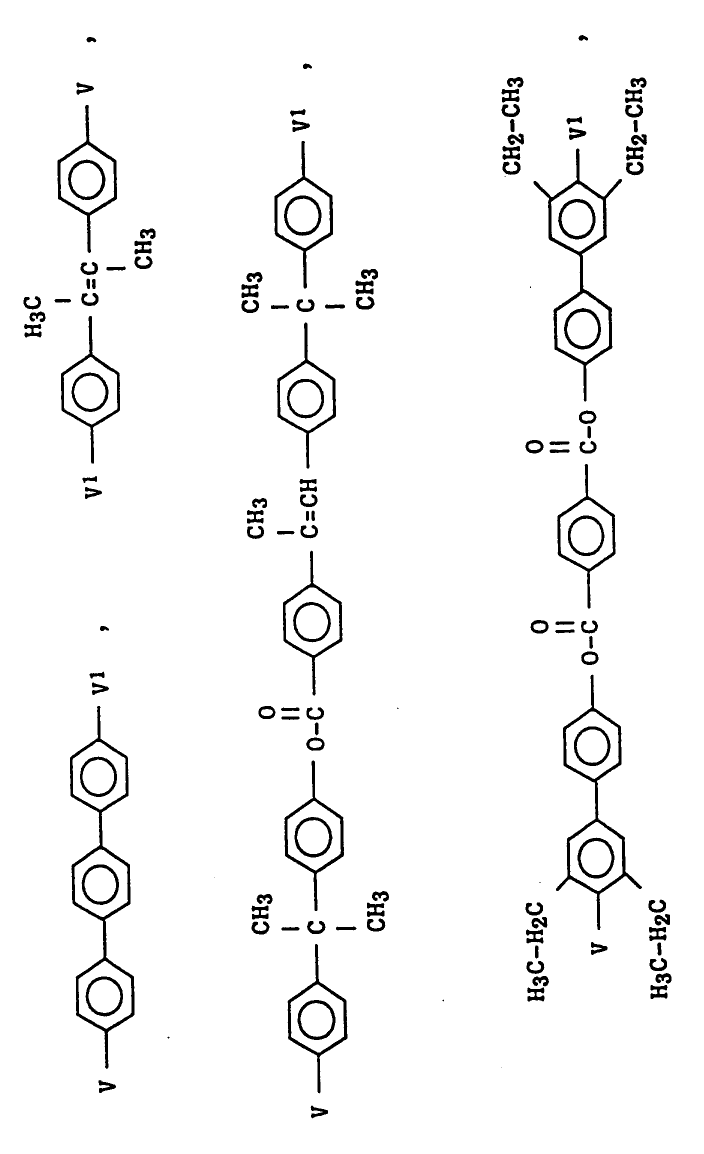 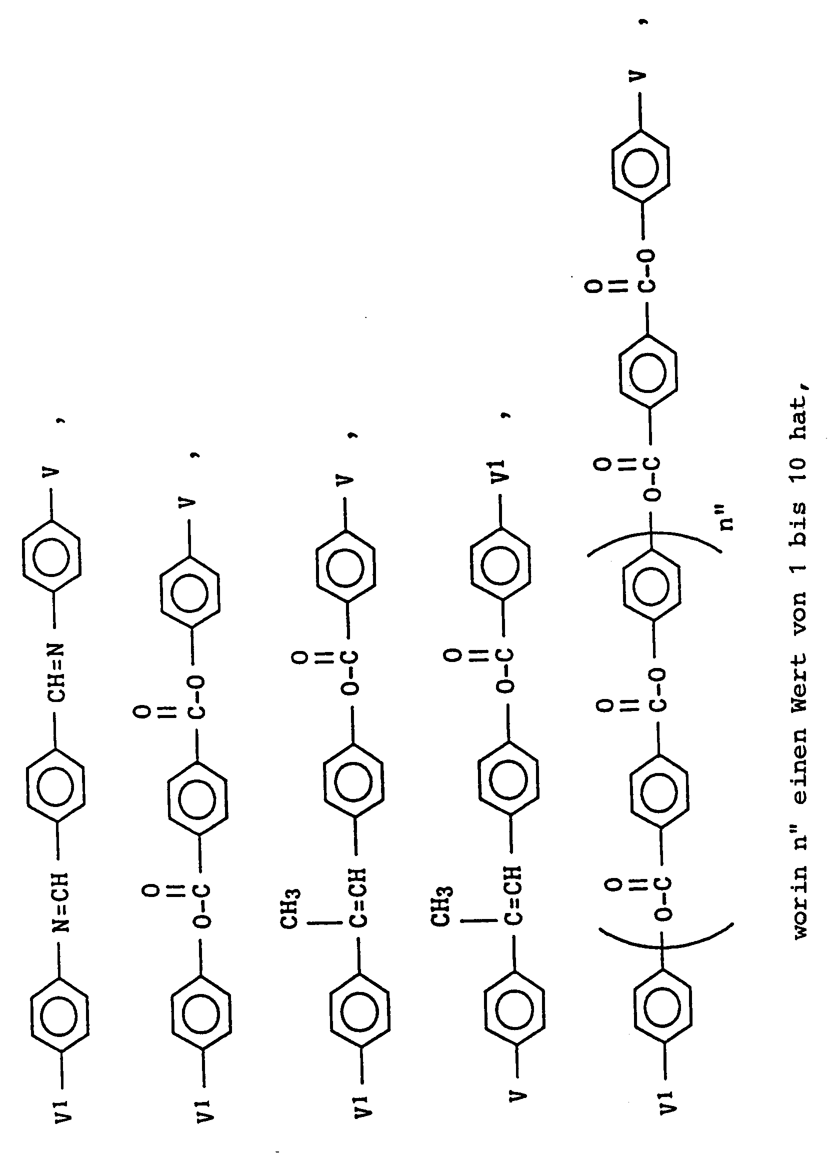 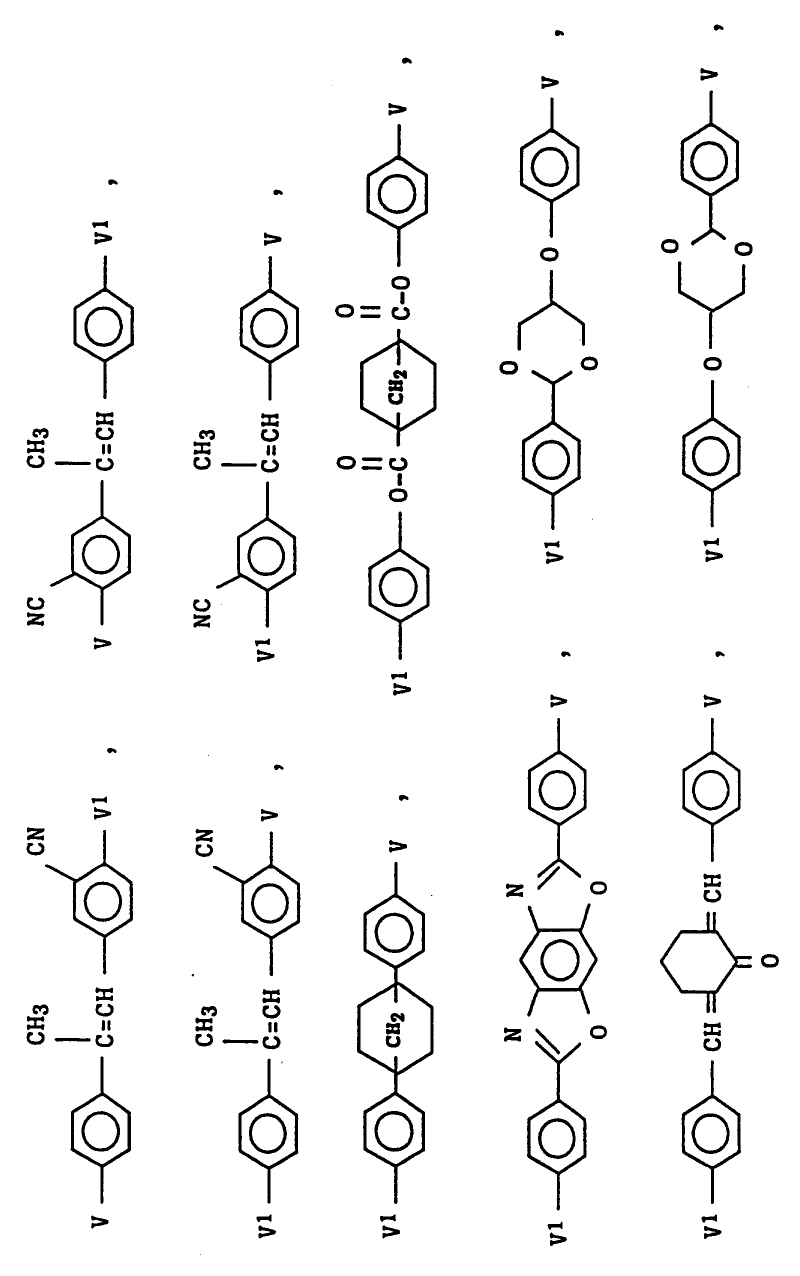 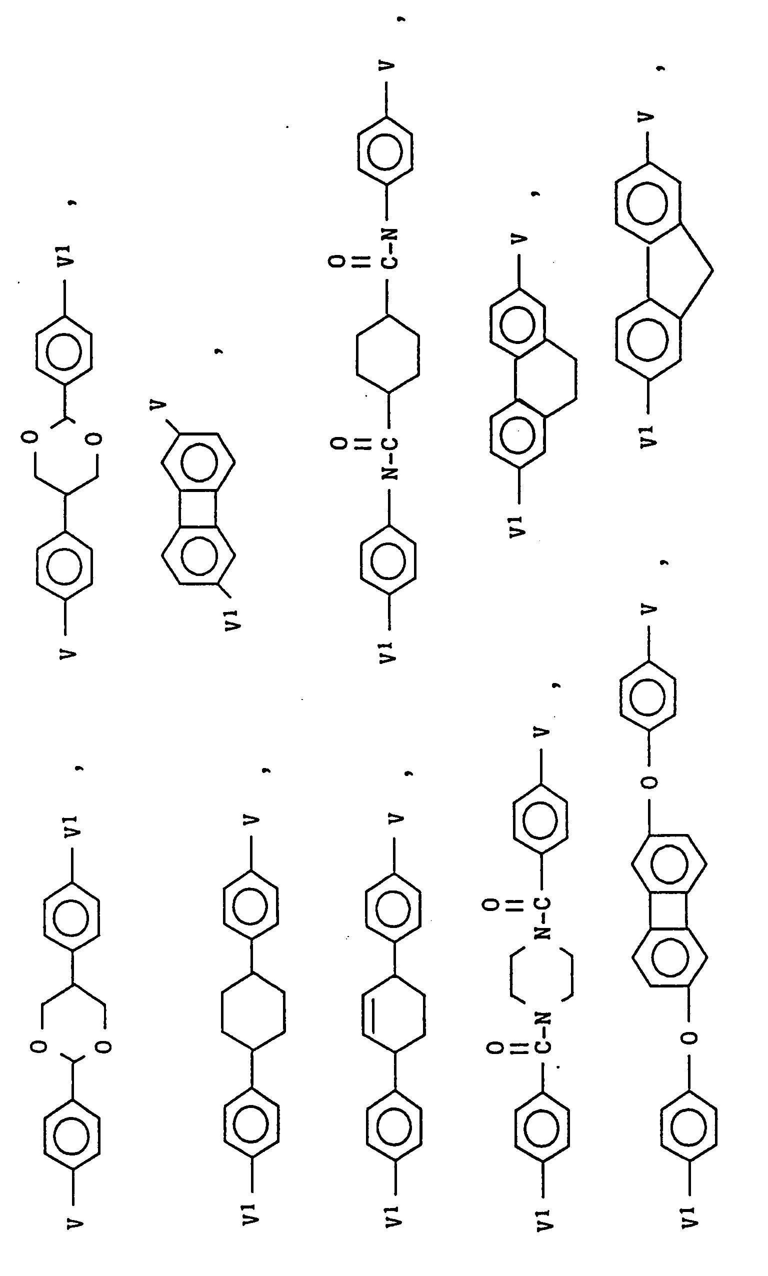 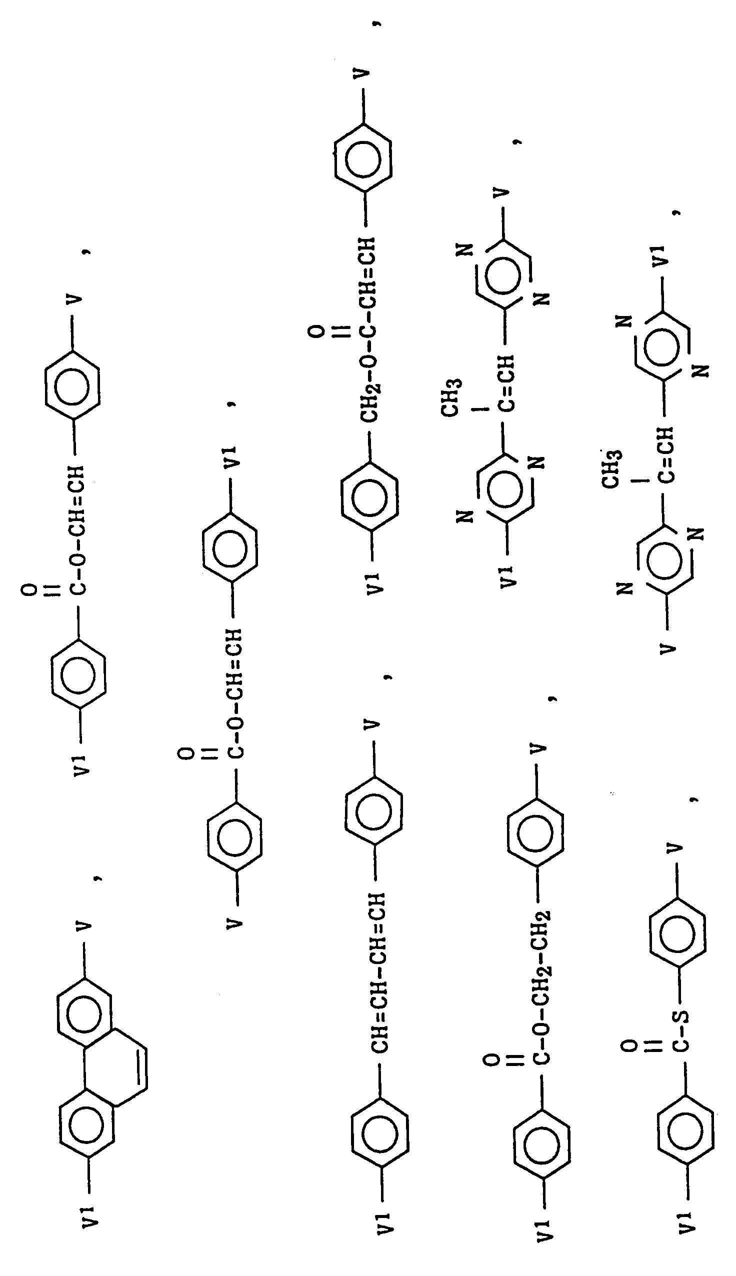 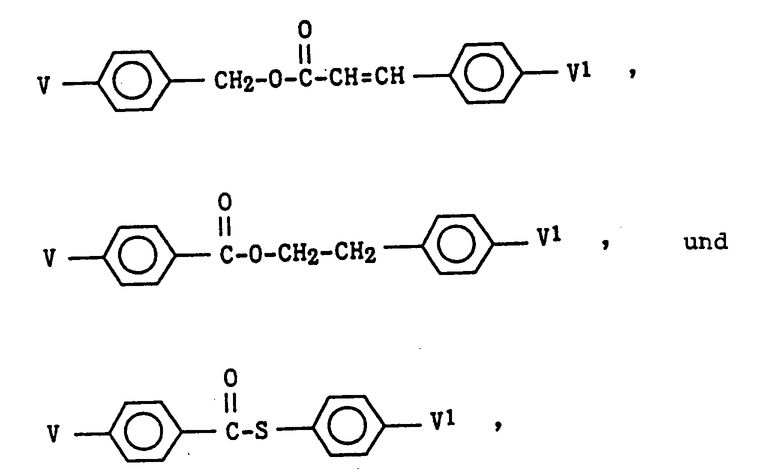 worin V¹ eine Maleinimidgruppe ist, welche durch die Formel wiedergegeben wird: 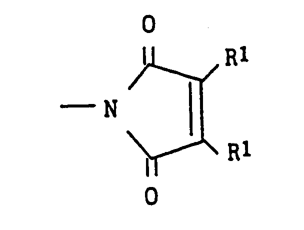 und V eine Cyanatgruppe, -O-C≡N, ist, und worin R¹ unabhängig Wasserstoff oder eine Hydrocarbylgruppe mit 1 bis 3 Kohlenstoffatomen ist.

10. Produkt, resultierend aus dem Aushärten der aushärtbaren Zusammensetzung von Anspruch 5, 6, 7, 8 oder 9.

11. Produkt von Anspruch 4 oder 9, worin die mesogenen oder stabförmigen Einheiten vor oder während des Aushärten orientiert wurden.

12. Produkt nach Anspruch 11, worin diese Orientierung mittels eines elektrischen oder magnetischen Feldes oder durch Scherkräfte herbeigeführt wurde.

## Revendications

1. Composé comportant au moins un groupe cyanate, au moins un groupe maléimide et au moins un segment mésogène ou en forme de bâtonnet, et représenté par l'une des formules suivantes I, II, III et IV : 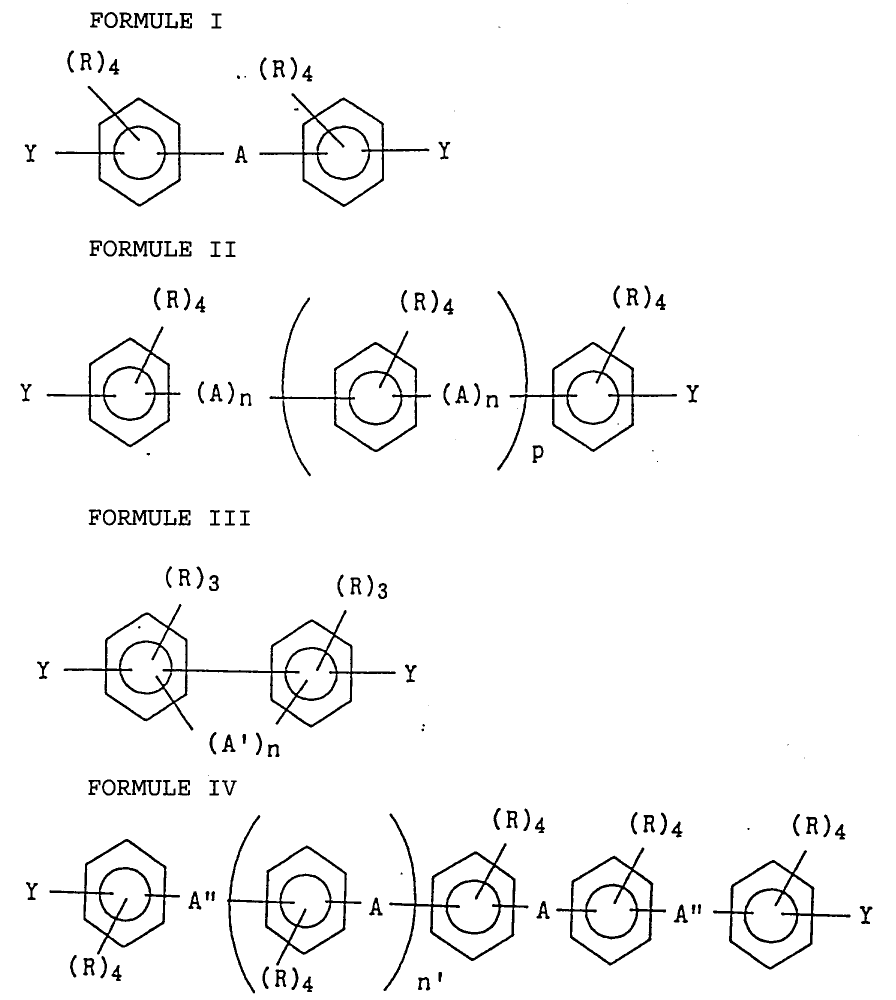 formules dans lesquelles les chaînons de raccordement -A- figurant dans les formules I, II et IV, ainsi que la liaison directe figurant dans la formule III, et les groupes Y se trouvent, pour au moins environ 80 % d'entre eux, en position para les uns des autres ; l'un des groupes Y est un groupe cyanate, c'est-à-dire -O-C≡N, et l'autre groupe Y est un groupe maléimide représenté par la formule : 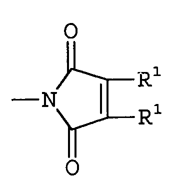 chaque A représente indépendamment -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 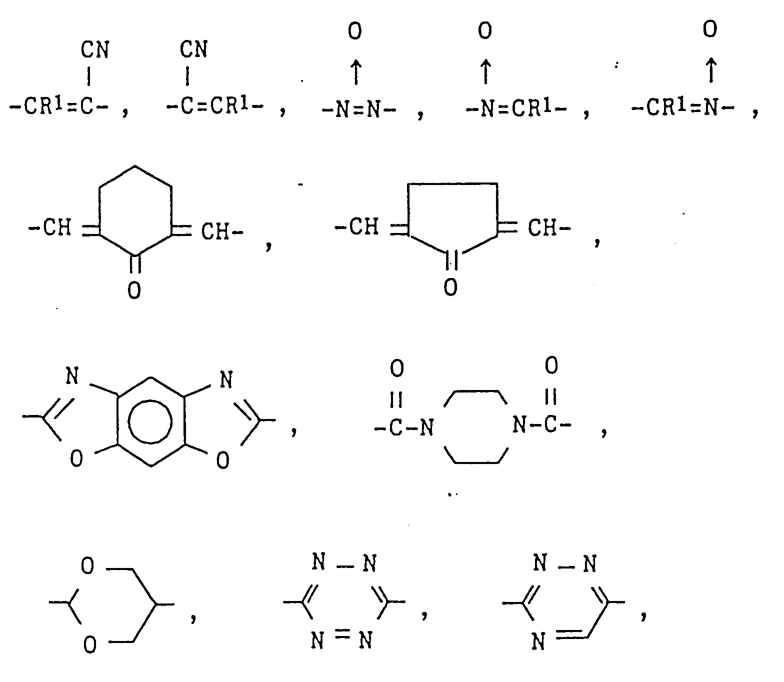 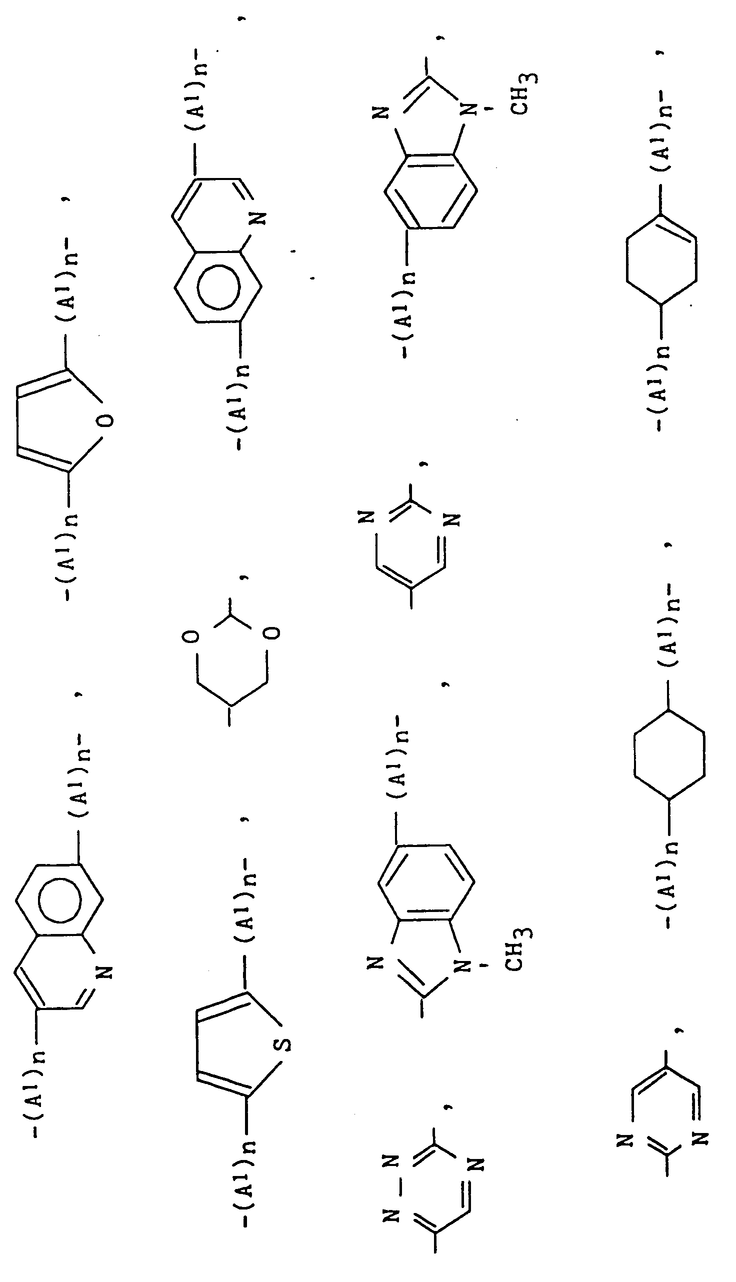 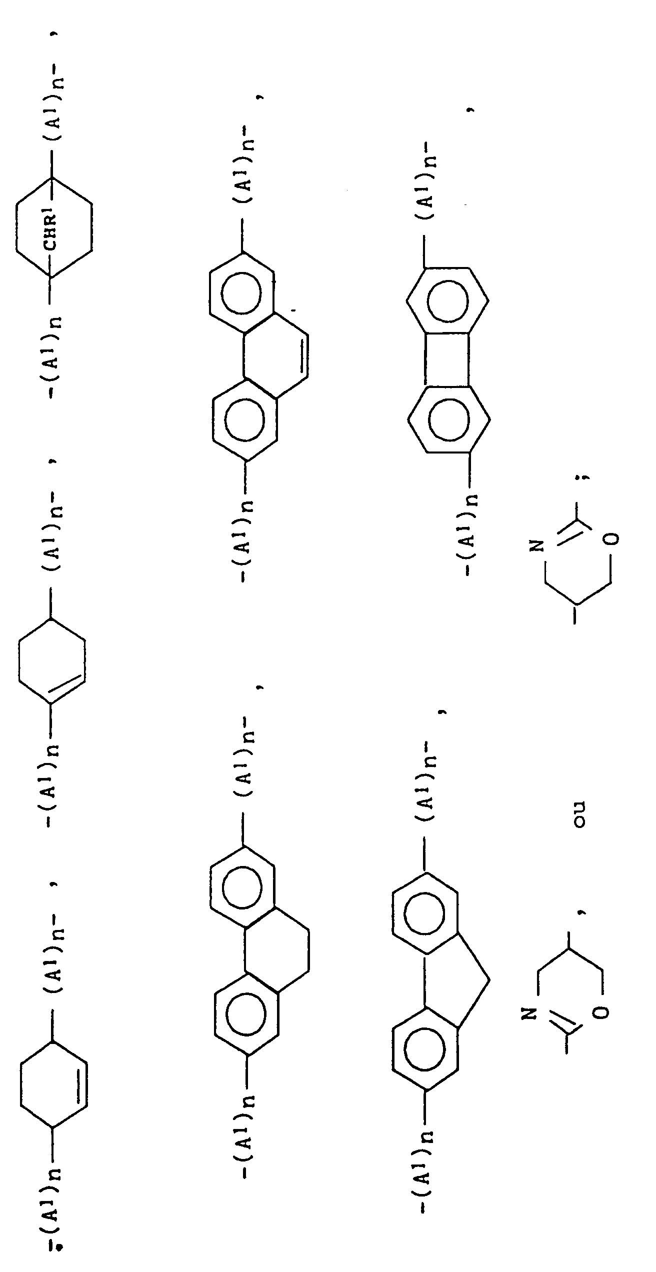 chaque A' représente indépendamment un groupe hydrocarbyle divalent, comportant de 1 à 10 atomes de carbone ; chaque A'' représente indépendamment un groupe alkylène comportant de 1 à 10 atomes de carbone, une liaison directe, ou un chaînon -O-, -CO-, -S-, -S-S-, -SO-, -SO₂- ou -O-CO-O ; chaque A¹ représente indépendamment un chaînon -CO-, -O-CO-, -CO-O-, -CO-NR¹- ou -NR¹-CO- ; chaque R représente indépendamment un atome d'hydrogène, un groupe hydrocarbyle ou hydrocarbyl-oxy comportant de 1 à 10 atomes de carbone, un atome d'halogène, un groupe nitro, un groupe cyano, un groupe phényle ou un groupe -CO-R¹ ; chaque R¹ représente indépendamment un atome d'hydrogène ou un groupe hydrocarbyle comportant de 1 à 3 atomes de carbone ; n vaut 0 ou 1 ; n' vaut en moyenne de 0 à 6 ; et p vaut en moyenne de 1 à 30 ; sous réserve que n'importe lequel des cycles aromatiques puisse, si on le souhaite, comporter un hétéroatome d'azote, d'oxygène ou de soufre.

2. Composé conforme à la revendication 1, dans lequel chaque A' comporte indépendamment de 1 à 4 atomes de carbone ; si A'' représente un groupe alkylène, il comporte de 1 à 4 atomes de carbone ; chaque R représente indépendamment un atome d'hydrogène ou un groupe hydrocarbyle comportant de 1 à 4 atomes de carbone, ou encore un atome de chlore ou de brome : n' vaut en moyenne de 0 à 3 ; et p vaut en moyenne de 1 à 3.

3. Composé conforme à la revendication 1, choisi dans l'ensemble constitué par : 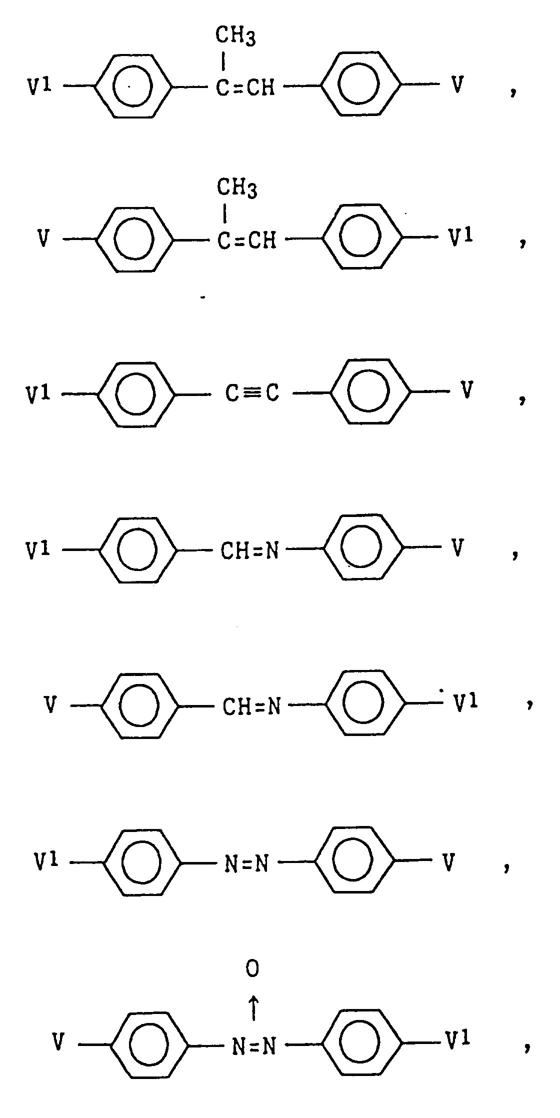 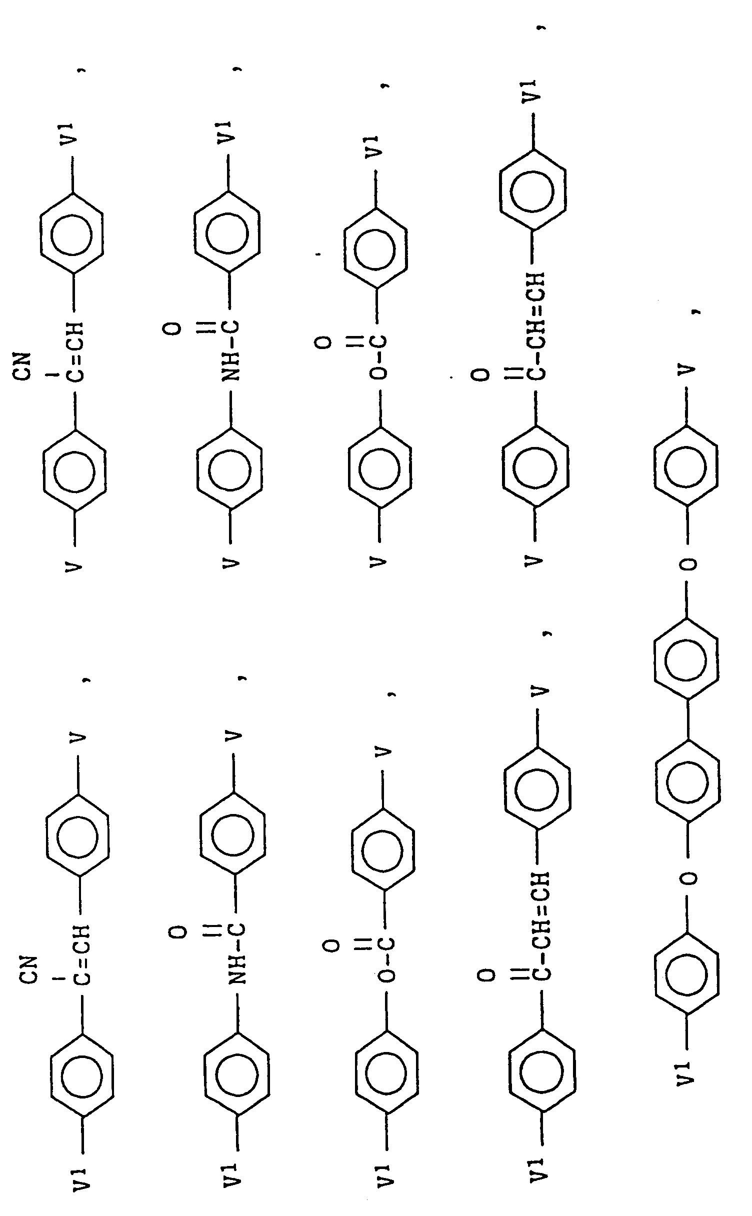 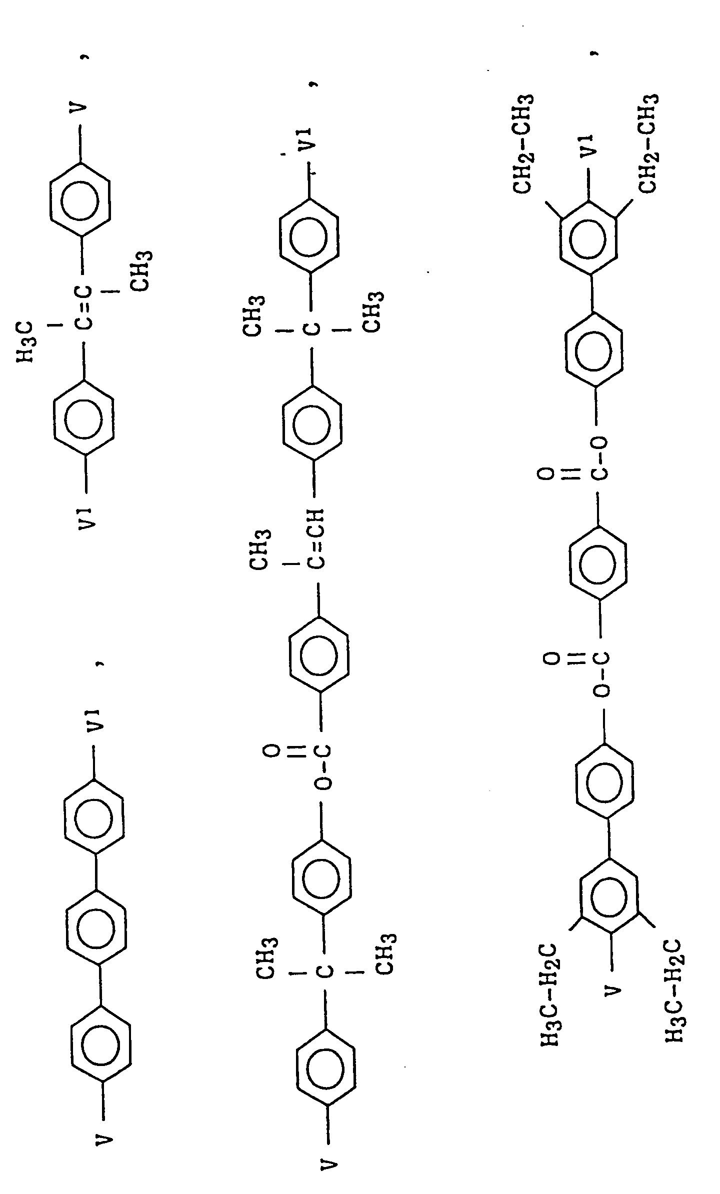 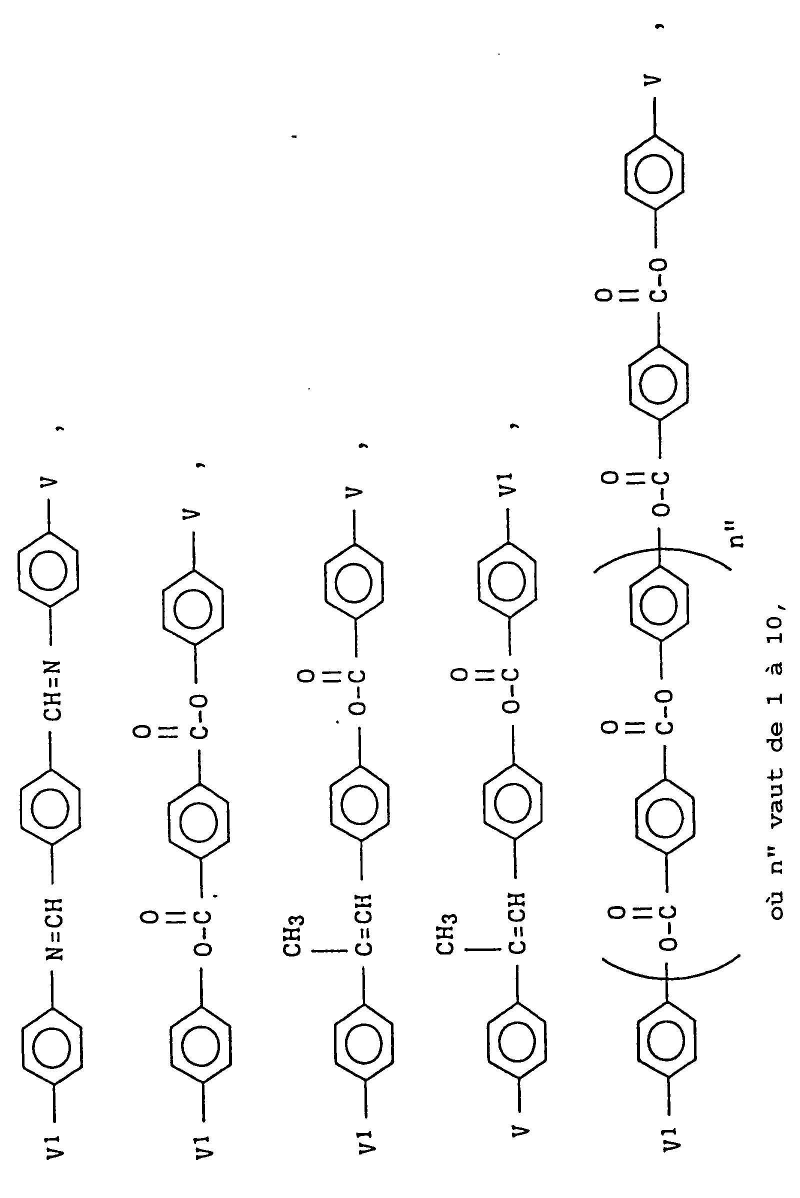 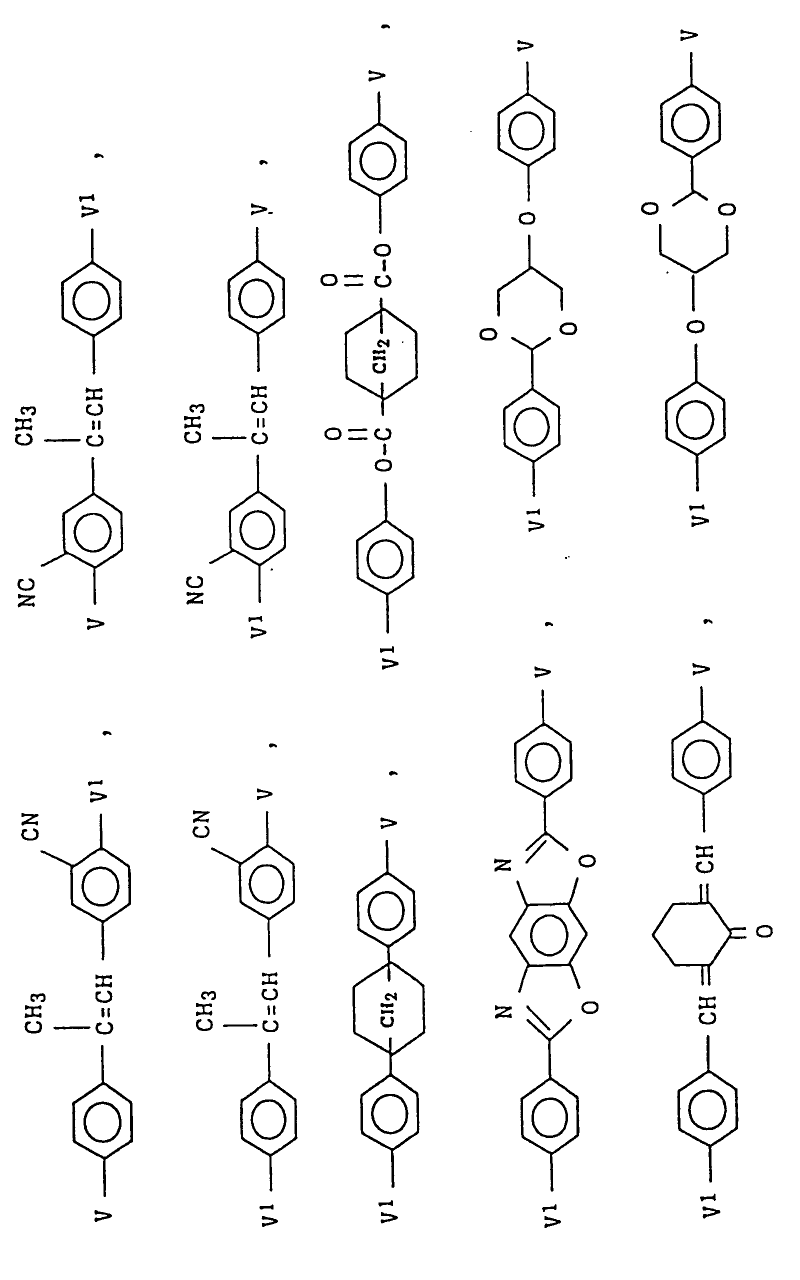 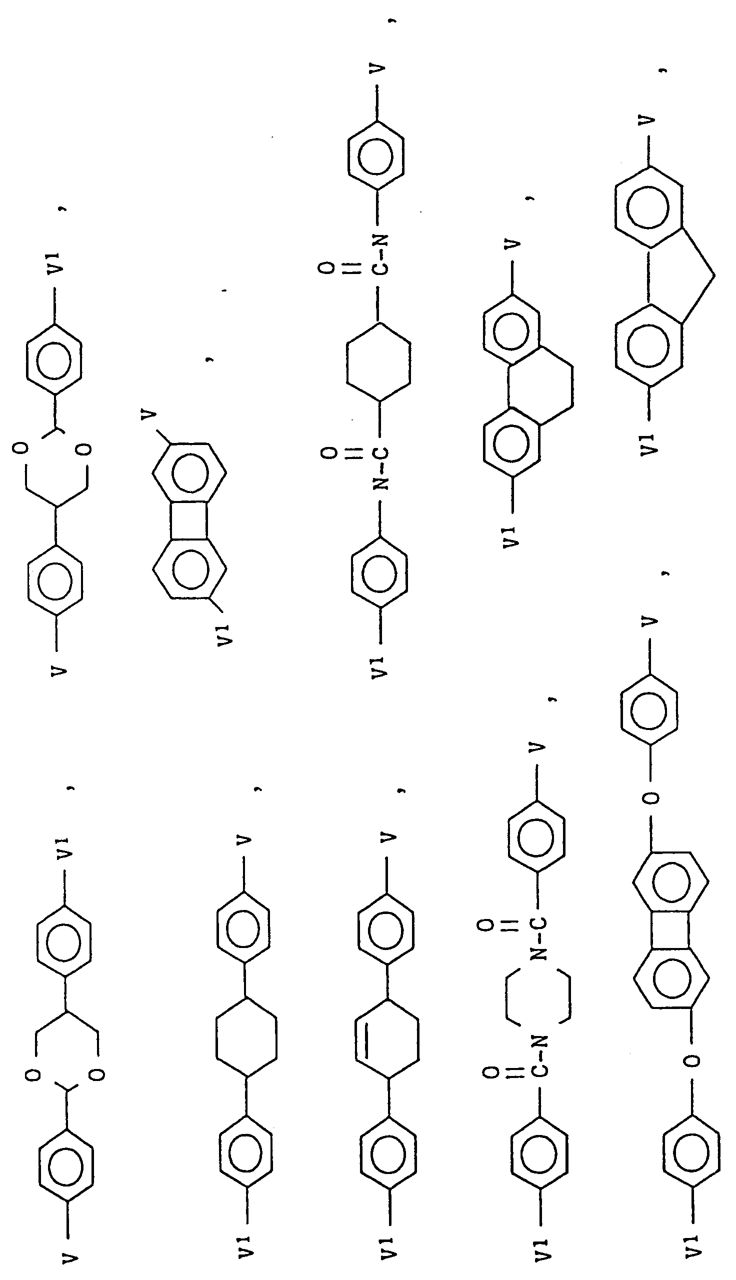 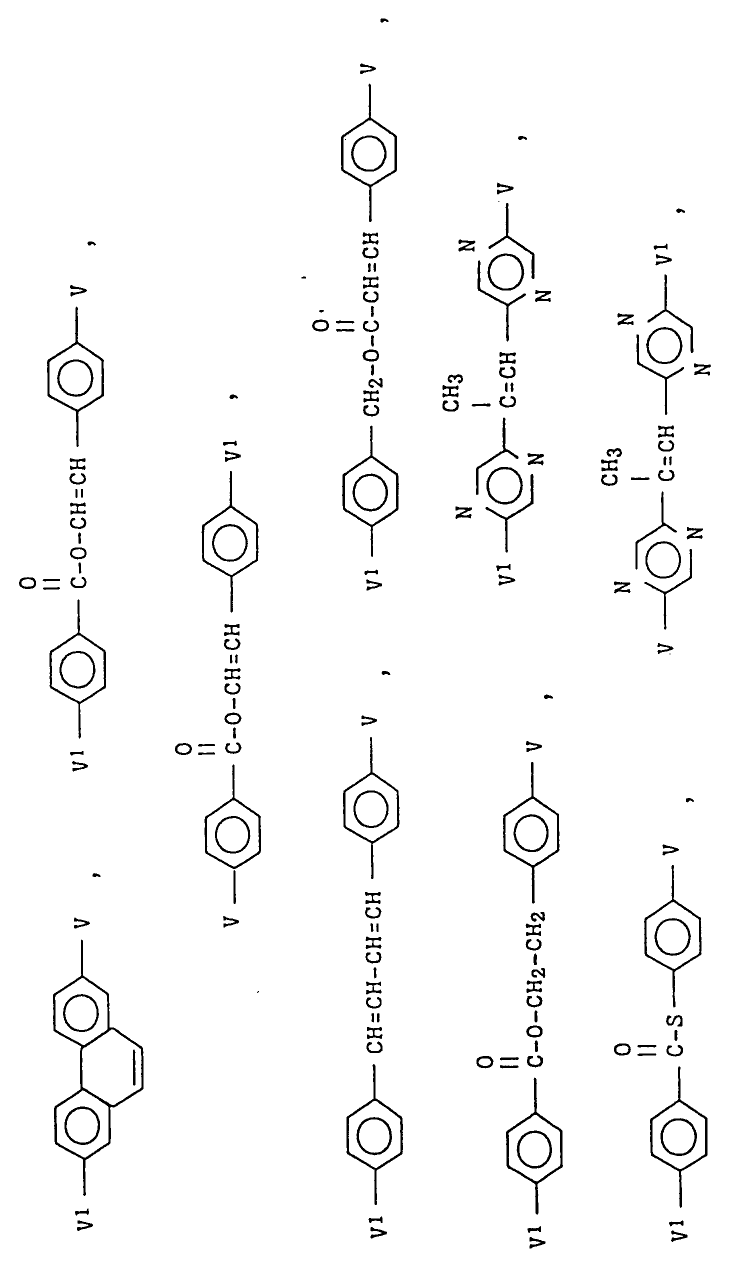 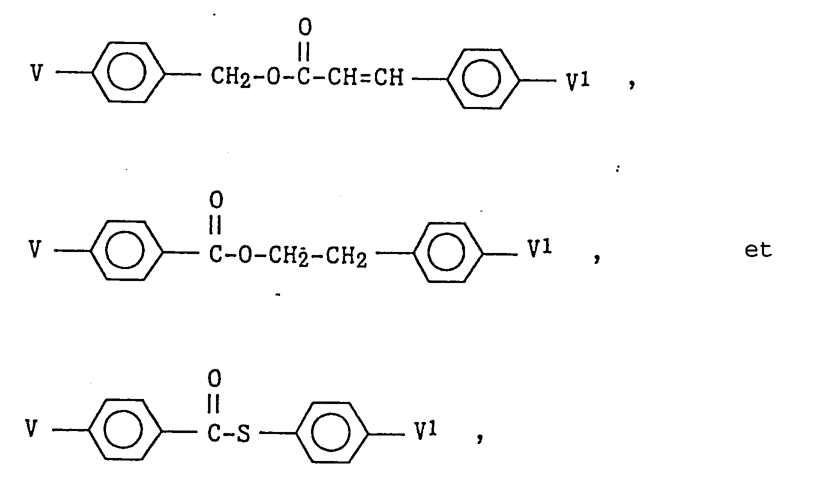 où V¹ représente un groupe maléimide de formule : 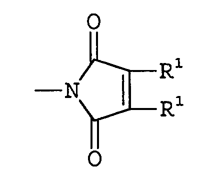 et V représente un groupe cyanate de formule -O-C≡N, et où R¹ représente indépendamment un atome d'hydrogène ou un groupe hydrocarbyle comportant de 1 à 3 atomes de carbone.

4. Produit résultant du durcissement d'une composition contenant un ou plusieurs composés conformes à la revendication 1, 2 ou 3, éventuellement en présence d'un ou de plusieurs catalyseurs.

5. Composition durcissable comportant un mélange contenant :
(A) au moins un composé comportant au moins un groupe cyanate, au moins un groupe maléimide et au moins un segment mésogène ou en forme de bâtonnet, défini dans la revendication 1 ; et
(B) au moins l'un des composés suivants :
(1) au moins un polycyanate ou un polycyanamide ne comportant pas de structures mésogènes ou en bâtonnets ;
(2) au moins une résine époxyde ;
(3) au moins un polymaléimide ;
(4) au moins une polyamine ;
(5) au moins un polyphénol ;
(6) au moins un composé comportant un ou plusieurs groupes polymérisables à insaturation éthylénique ;
(7) au moins un composé comportant, dans la même molécule, un groupe cyanate ou cyanamide et un groupe polymérisable à insaturation éthylénique ;
(8) au moins un composé comportant, dans la même molécule, un groupe 1,2-époxyde et un groupe polymérisable à insaturation éthylénique ;
(9) au moins un composé comportant, dans la même molécule, un groupe maléimide et un groupe cyanate, mais ne comportant pas de structures mésogènes ou en forme de bâtonnets ;
(10) au moins un composé comportant un ou plusieurs segments mésogènes ou en forme de bâtonnets, mais ne comportant qu'un groupe cyanate ou cyanamide par molécule ;
(11) au moins un prépolymère de n'importe lequel des composants (1) à (10) mentionnés ci-dessus ou de toute combinaison de deux de ces composants ou plus ; ou
(12) un mélange de deux ou plus des composants (1) à (11), en n'importe quelles proportions et en n'importe quelle combinaison.

6. Composition conforme à la revendication 5, dans laquelle le composant (A) se trouve en une quantité représentant de 1 à 99 % du poids total des composants (A) et (B), et le composant (B) se trouve en une quantité représentant de 99 à 1 % du poids total des composants (A) et (B).

7. Composition conforme à la revendication 5 ou 6, dans laquelle le composant (A) est constitué d'un ou de plusieurs composés représentés par les formules générales suivantes I, II, III et IV : 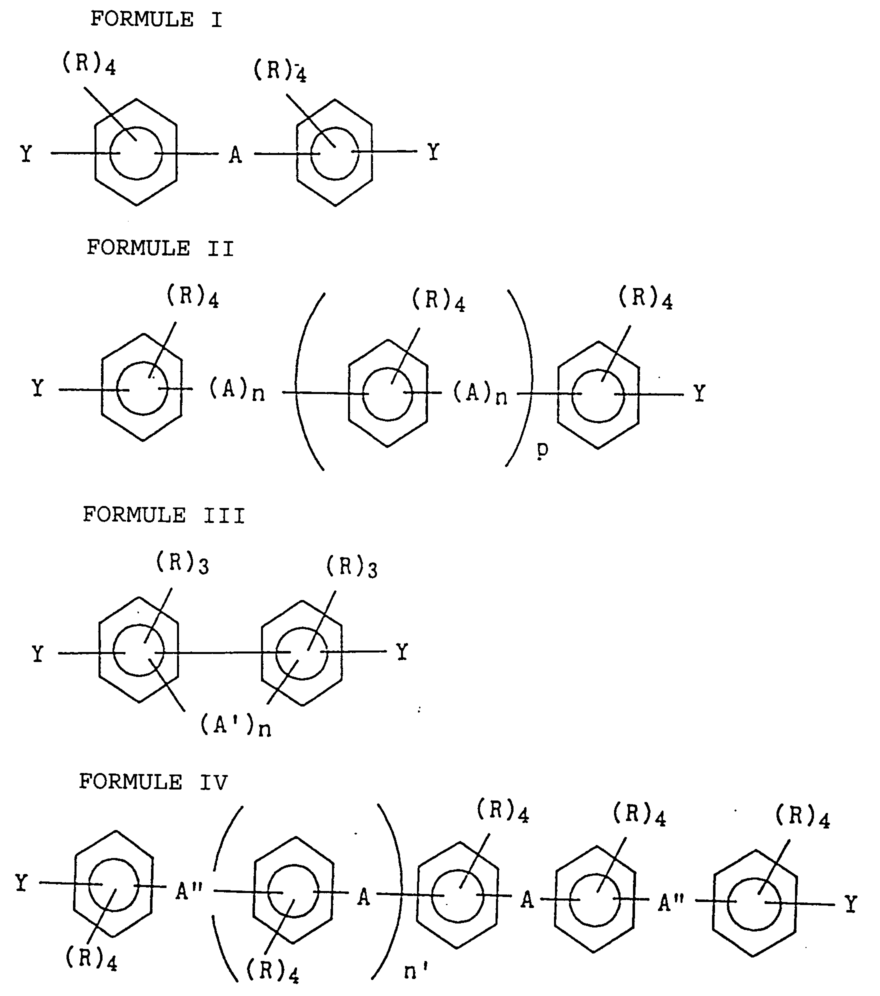 formules dans lesquelles les chaînons de raccordement -A- figurant dans les formules I, II et IV, ainsi que la liaison directe figurant dans la formule III, et les groupes Y se trouvent, pour au moins environ 80 % d'entre eux, en position para les uns des autres ; l'un des groupes Y est un groupe cyanate, c'est-à-dire -O-C≡N, et l'autre groupe Y est un groupe maléimide représenté par la formule : 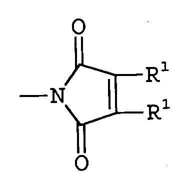 chaque A représente indépendamment -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 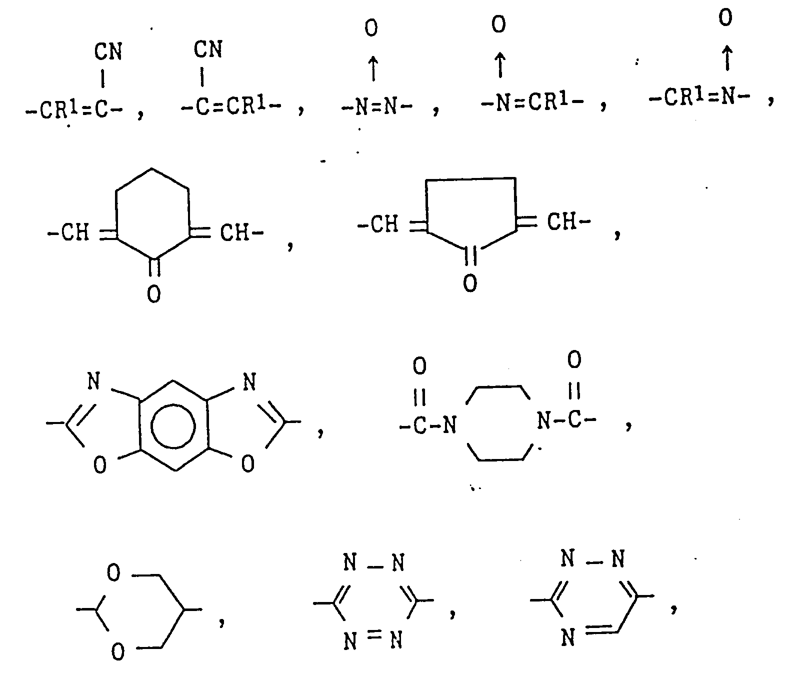 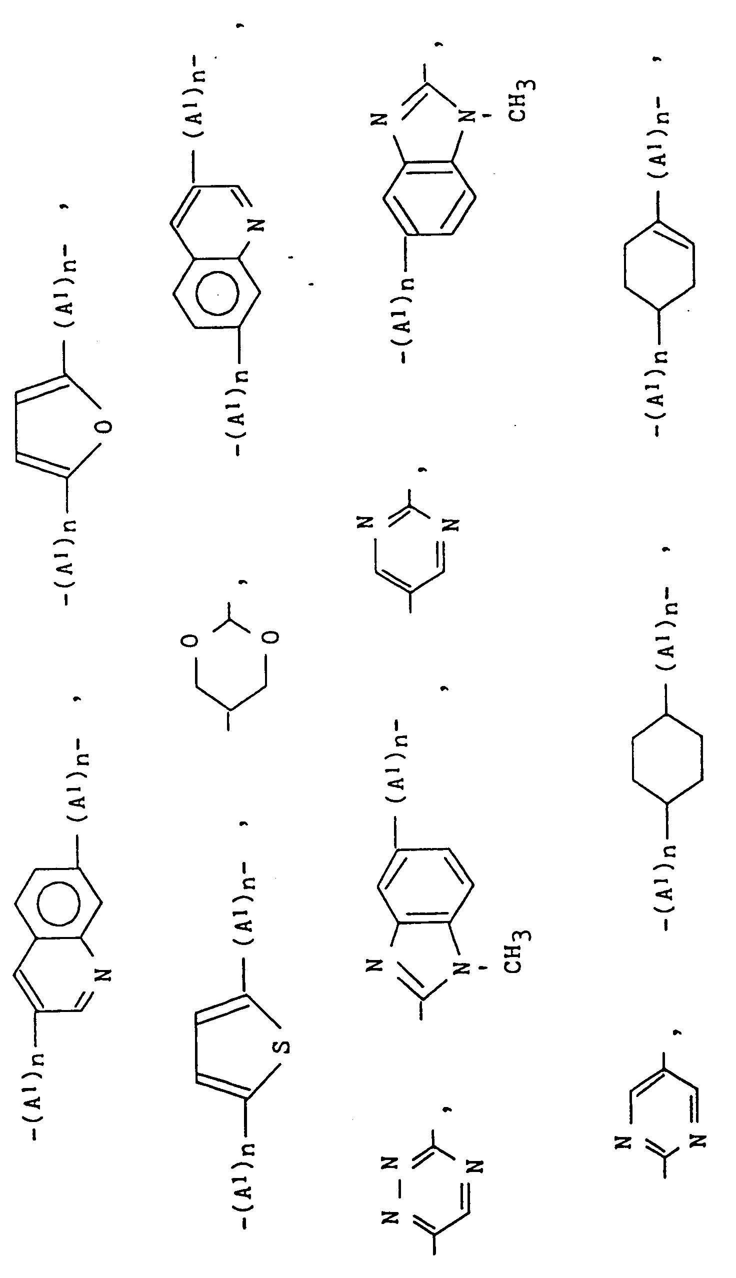 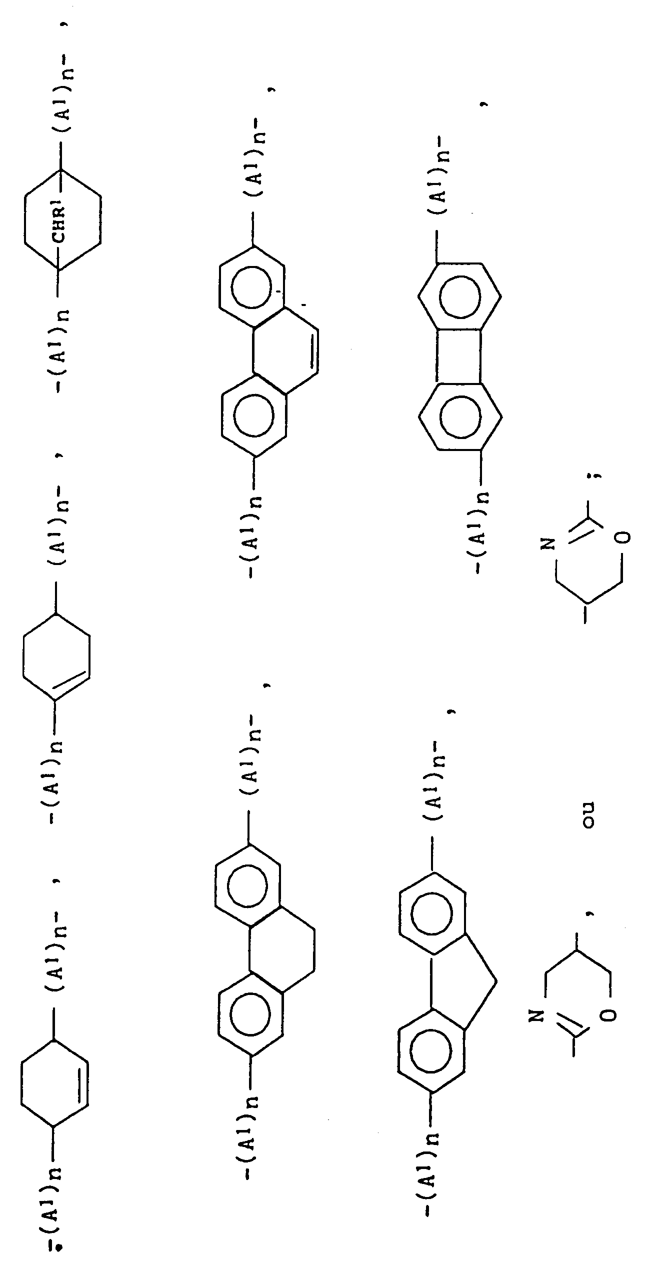 chaque A' représente indépendamment un groupe hydrocarbyle divalent, comportant de 1 à 10 atomes de carbone ; chaque A'' représente indépendamment un groupe alkylène comportant de 1 à 10 atomes de carbone, une liaison directe, ou un chaînon -O-, -CO-, -S-, -S-S-, -SO-, -SO₂- ou -O-CO-O ; chaque A¹ représente indépendamment un chaînon -CO-, -O-CO-, -CO-O-, -CO-NR¹- ou -NR¹-CO- ; chaque R représente indépendamment un atome d'hydrogène, un groupe hydrocarbyle ou hydrocarbyl-oxy comportant de 1 à 10 atomes de carbone, un atome d'halogène, un groupe nitro, un groupe cyano, un groupe phényle ou un groupe -CO-R¹ ; chaque R¹ représente indépendamment un atome d'hydrogène ou un groupe hydrocarbyle comportant de 1 à 3 atomes de carbone ; n vaut 0 ou 1 ; n' vaut en moyenne de 0 à 6 ; et p vaut en moyenne de 1 à 30 ; sous réserve que n'importe lequel des cycles aromatiques puisse, si on le souhaite, comporter un hétéroatome d'azote, d'oxygène ou de soufre.

8. Composition conforme à la revendication 7, dans laquelle, dans le composant (A), chaque A' comporte indépendamment de 1 à 4 atomes de carbone ; si A'' représente un groupe alkylène, il comporte de 1 à 4 atomes de carbone ; chaque R représente indépendamment un atome d'hydrogène ou un groupe hydrocarbyle comportant de 1 à 4 atomes de carbone, ou encore un atome de chlore ou de brome ; n' vaut en moyenne de 0 à 3 ; et p vaut en moyenne de 1 à 3.

9. Composition conforme à la revendication 5 ou 6, dans laquelle le composant (A) est constitué d'un ou de plusieurs composés choisis dans l'ensemble constitué par 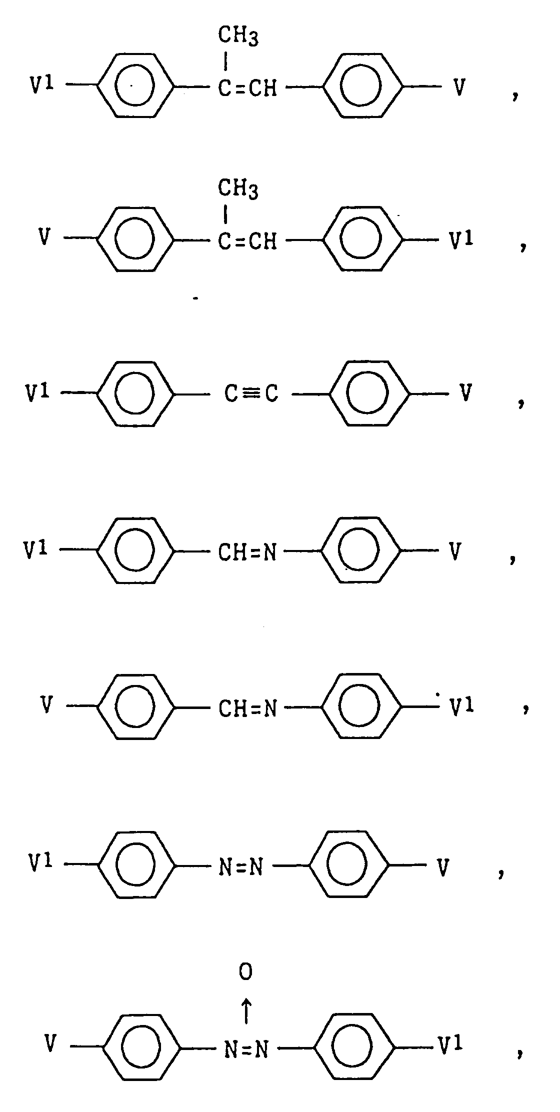 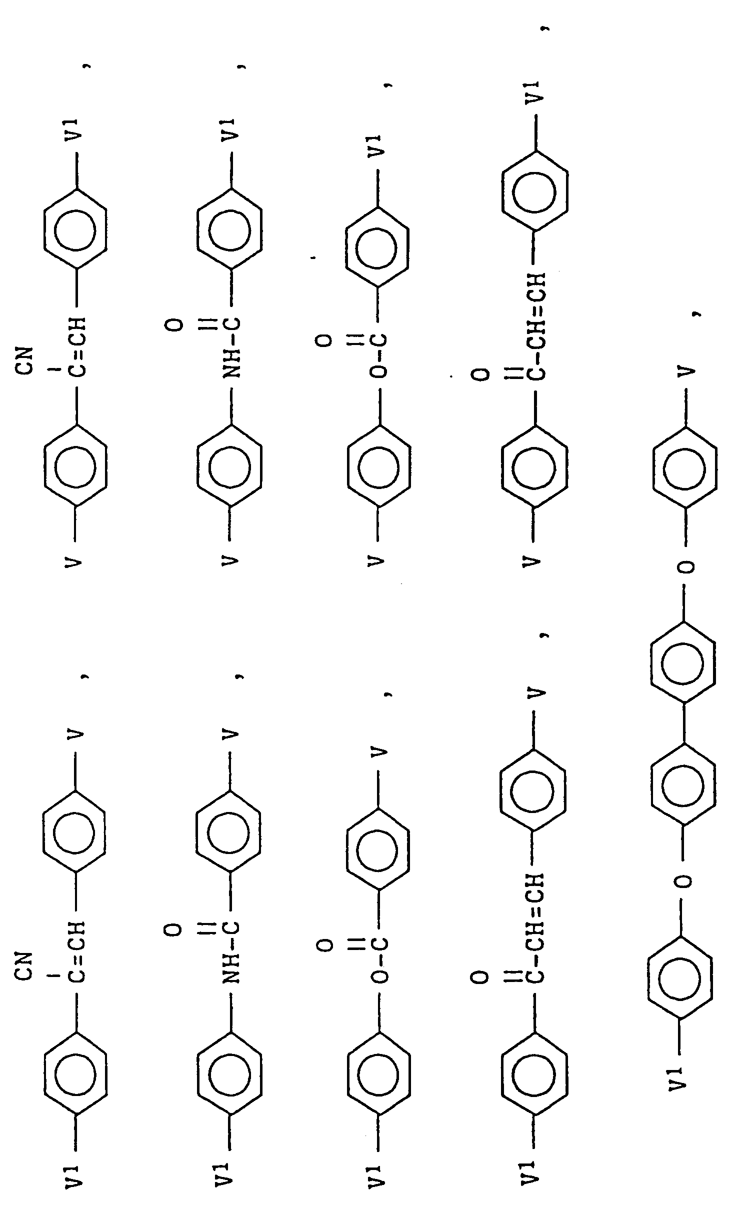 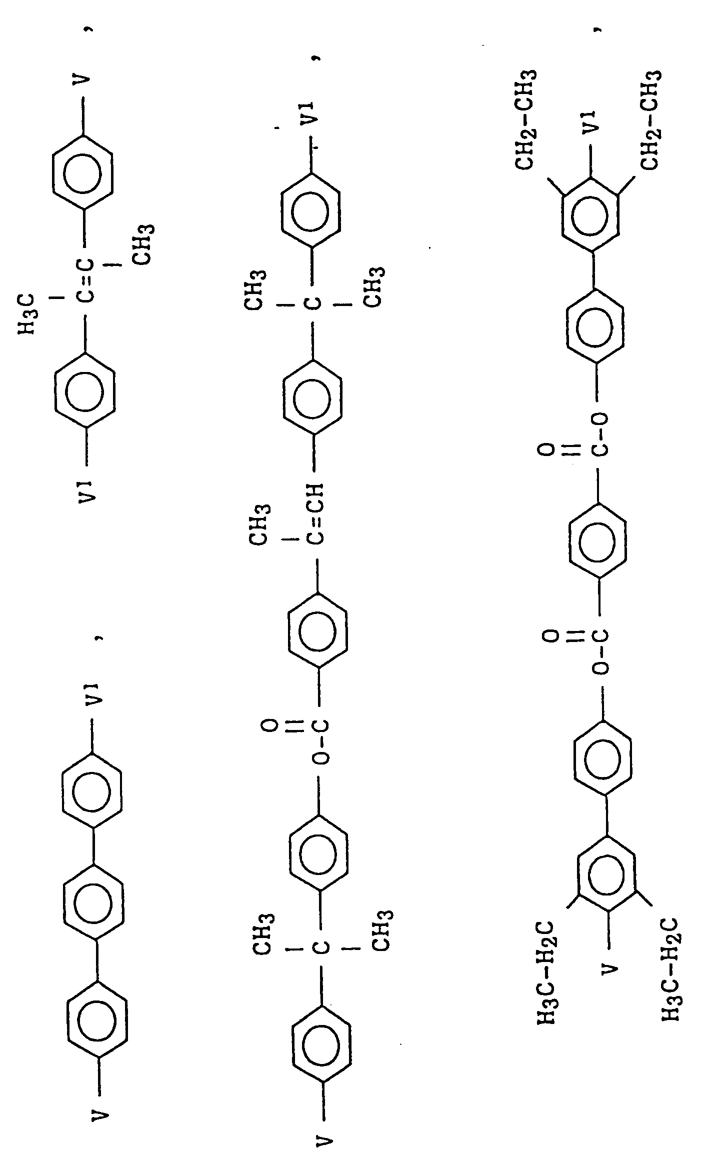 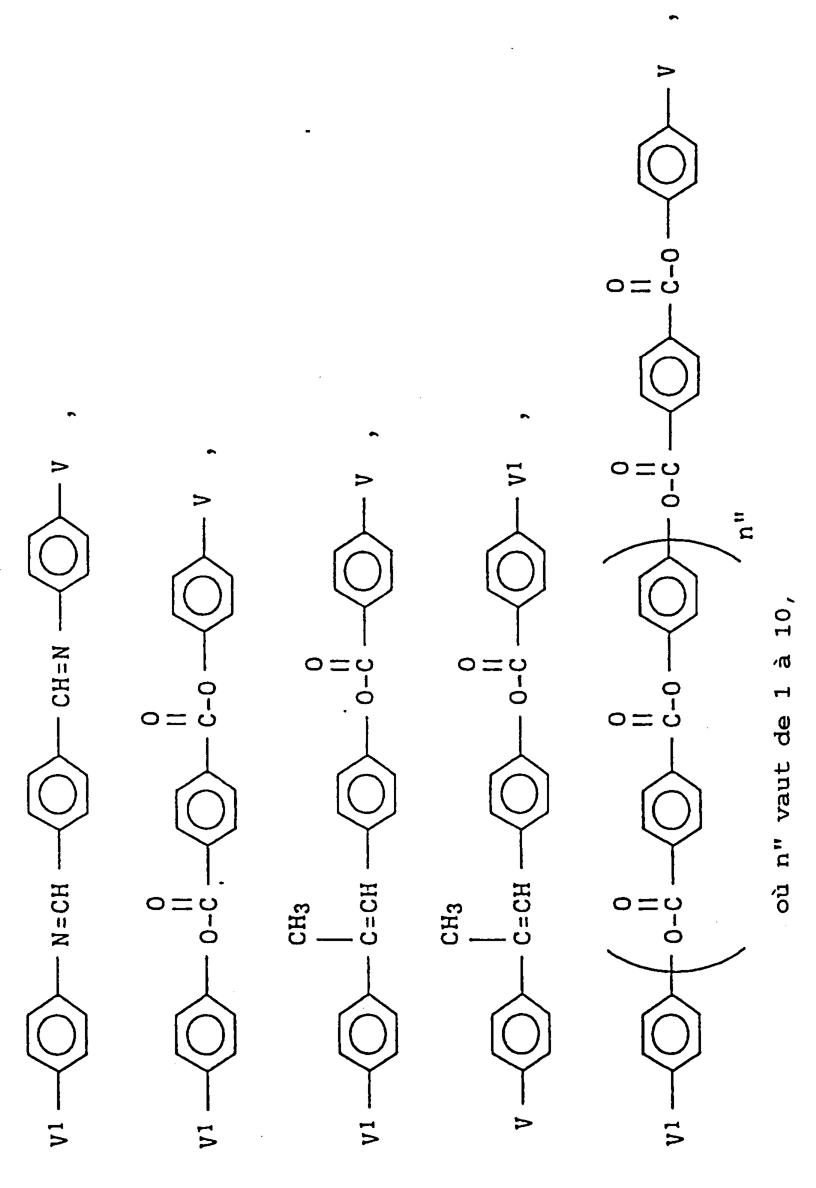 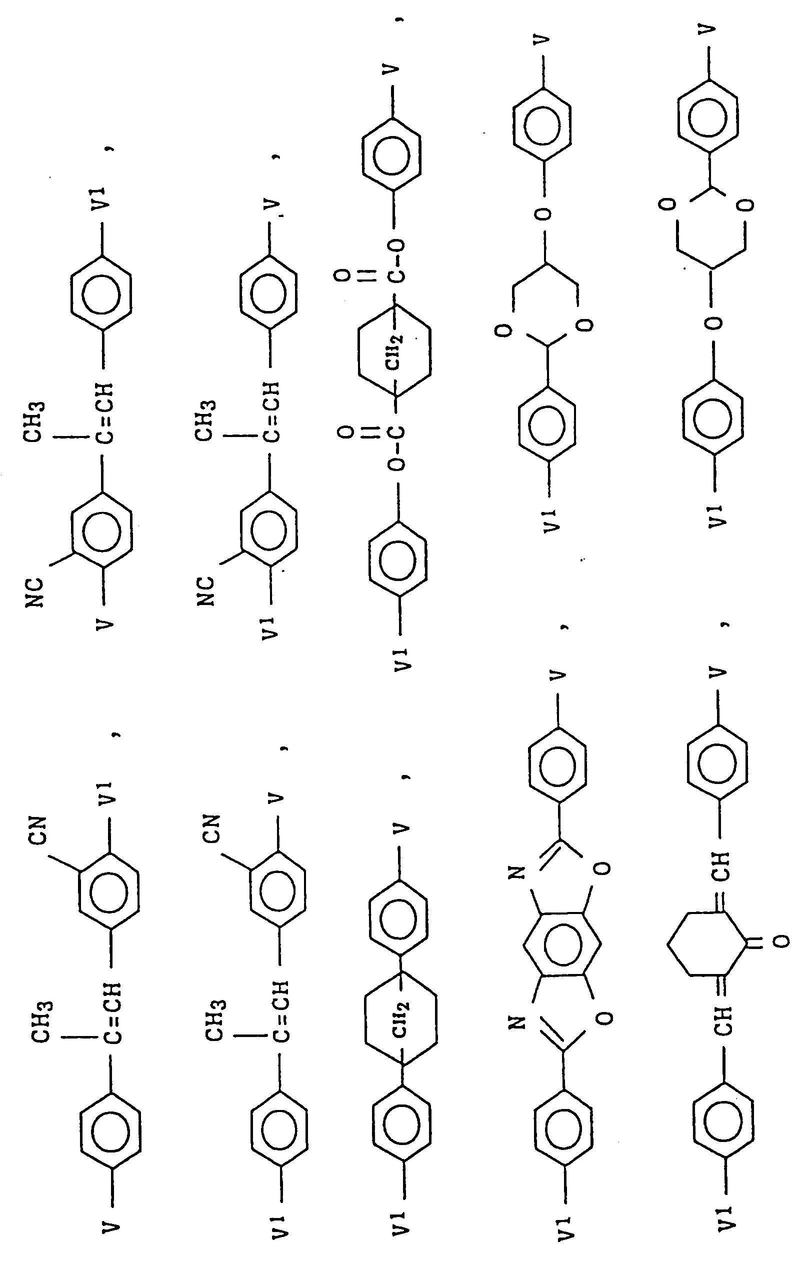 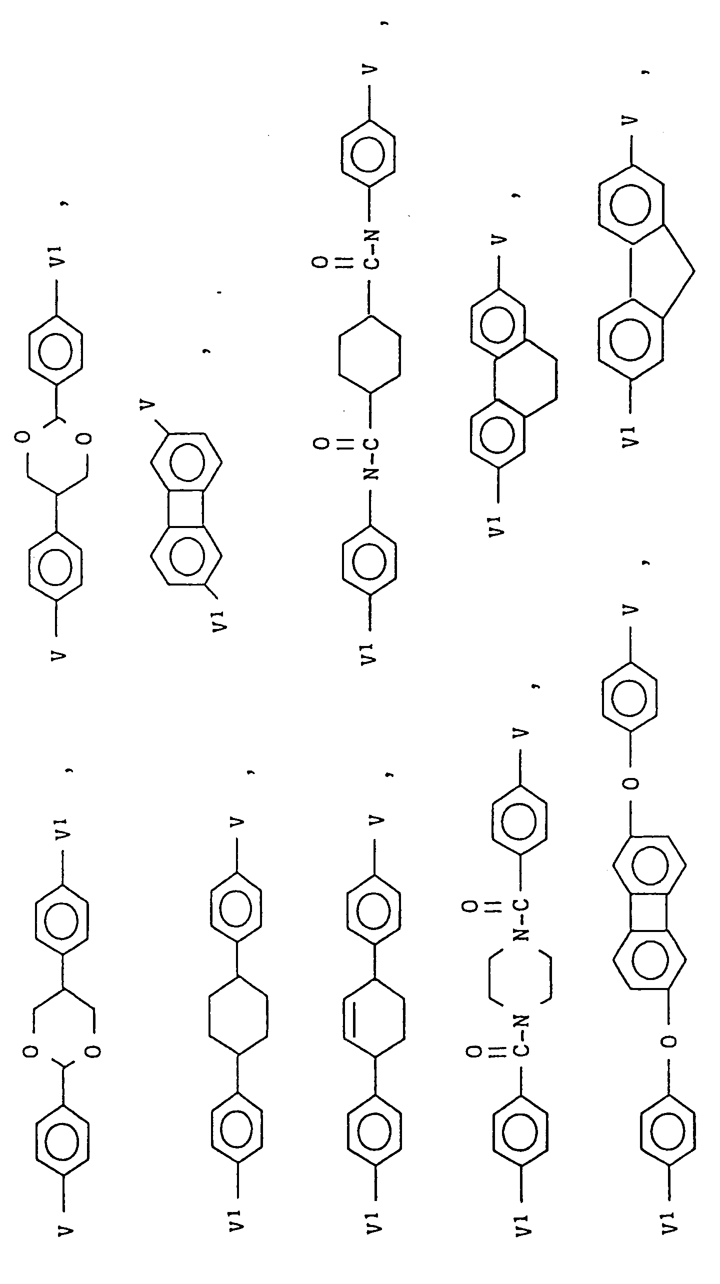 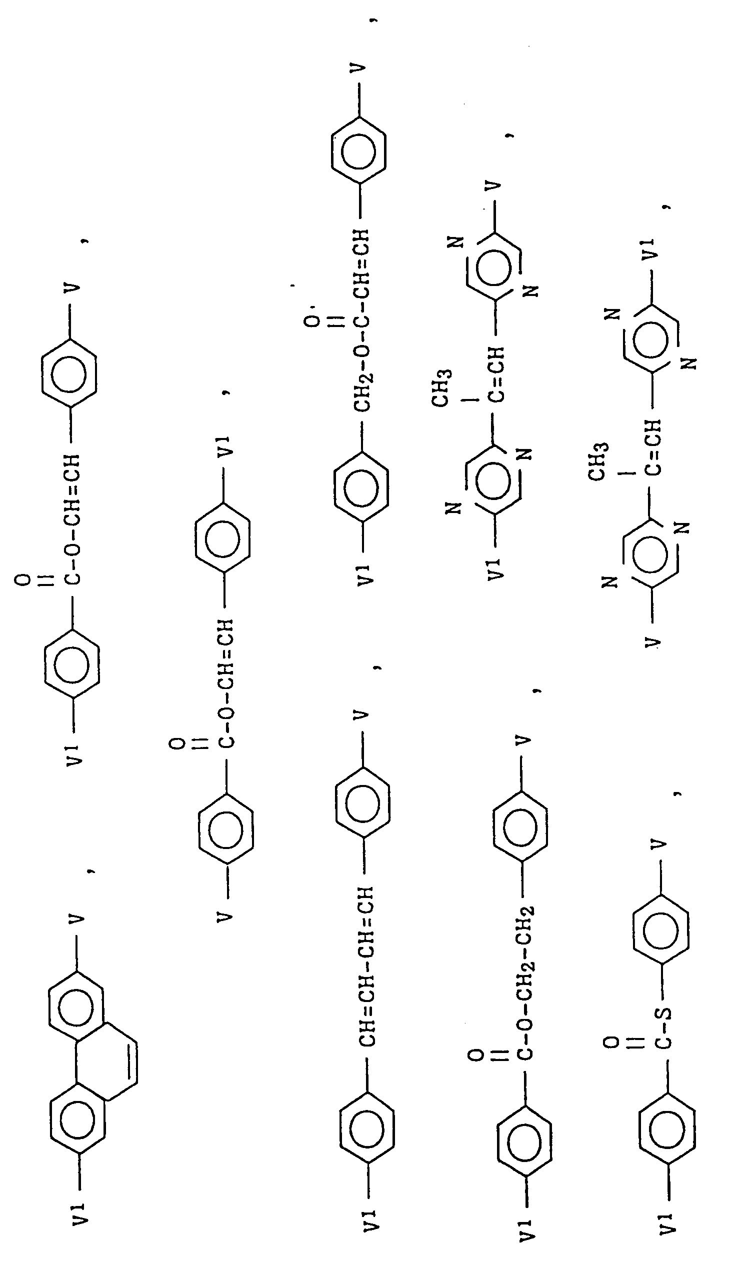 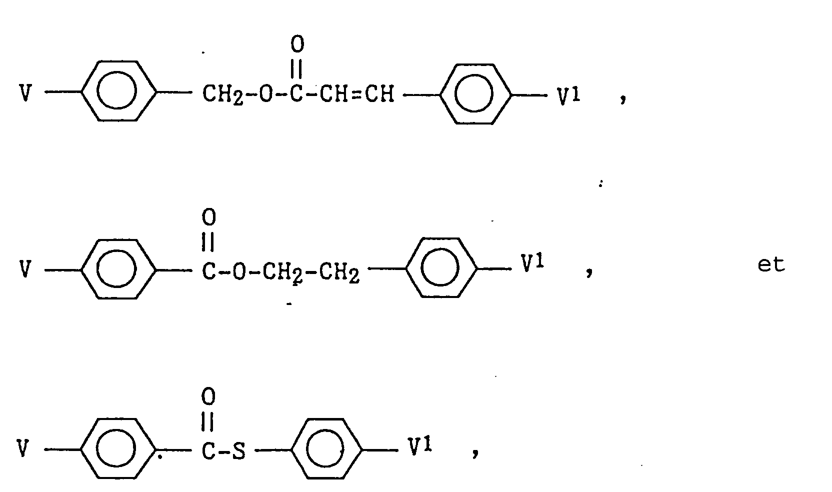 où V¹ représente un groupe maléimide de formule : 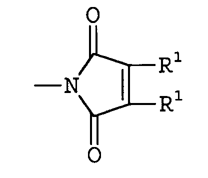 et V représente un groupe cyanate de formule -O-C≡N, et où R¹ représente indépendamment un atome d'hydrogène ou un groupe hydrocarbyle comportant de 1 à 3 atomes de carbone.

10. Produit résultant du durcissement d'une composition durcissable conforme à la revendication 5, 6, 7, 8 ou 9.

11. Produit conforme à la revendication 4 ou 9, dans lequel les segments mésogènes ou en forme de bâtonnets sont orientés avant ou pendant le durcissement.

12. Produit conforme à la revendication 11, dans lequel ladite orientation est réalisée au moyen d'un champ électrique ou magnétique ou à l'aide de forces de cisaillement.
